(19) **Europäisches Patentamt / European Patent Office / Office européen des brevets**

(11) **EP 3 659 585 B1**

(12) # EUROPEAN PATENT SPECIFICATION

(45) Date of publication and mention
of the grant of the patent:
**25.06.2025 Bulletin 2025/26**

(21) Application number: **18838827.6**

(22) Date of filing: **25.07.2018**

(51) International Patent Classification (IPC):
*A61K 9/14* (2006.01)      *A61K 9/51* (2006.01)
*A61K 48/00* (2006.01)     *A61K 31/704* (2006.01)
*A61K 49/00* (2006.01)     *A61K 45/06* (2006.01)
*A61L 24/00* (2006.01)     *A61L 24/02* (2006.01)

(52) Cooperative Patent Classification (CPC):
A61K 31/44; A61K 9/0019; A61K 9/143;
A61K 9/5115; A61K 31/203; A61K 31/4745;
A61K 31/704; A61K 38/1709; A61K 38/385;
A61K 38/465; A61K 45/06; A61K 48/00;
A61L 24/0015; A61L 24/0036; A61L 24/02;
(Cont.)

(86) International application number:
**PCT/KR2018/008445**

(87) International publication number:
**WO 2019/022521 (31.01.2019 Gazette 2019/05)**

(54) **COMPOSITION FOR DELIVERING PHYSIOLOGICALLY ACTIVE INGREDIENTS INTO BLOOD VESSEL**

ZUSAMMENSETZUNG ZUR ABGABE VON PHYSIOLOGISCH AKTIVEN INHALTSSTOFFEN IN EIN BLUTGEFÄSS

COMPOSITION POUR ADMINISTRER DES SUBSTANCES PHYSIOLOGIQUEMENT ACTIVES DANS UN VAISSEAU SANGUIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.07.2017 US 201762536548 P**

(43) Date of publication of application:
**03.06.2020 Bulletin 2020/23**

(73) Proprietor: **Lemonex Inc.**
**Seoul 08826 (KR)**

(72) Inventor: **WON, Cheol Hee**
**Seoul 08741 (KR)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
EP-A1- 3 173 074      WO-A1-2005/097677
CN-A- 103 751 857      KR-A- 20120 025 224
KR-A- 20140 010 285      KR-A- 20160 137 109
US-A1- 2009 181 096      US-A1- 2010 104 650
US-A1- 2012 283 379

- GARCIA FAKHOURY, J. R.: "Porous silicon microparticles as an embolic agent for the treatment of hepatocellular carcinoma", THESIS-THE UNIVERSITY OF TEXAS AT AUSTIN, 31 December 2011 (2011-12-31), The University of Texas at Austin, pages 1 - 166, XP055623579

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- **KAASALAINEN, M. ET AL.: "Size, Stability, and Porosity of Mesoporous Nanoparticles Characterized with Light Scattering", NANOSCALE RES. LETT., vol. 12, no. 74, 25 January 2017 (2017-01-25), pages 1 - 10, XP021237161**
- **JADHAV, S. A. ET AL.: "Porous Silica Particles: Synthesis, Physicochemical Characterization and Evaluation of Suspension Stability", PHYSICAL CHEMISTRY : AN INDIAN JOURNAL, vol. S1, no. 102, 201705400 - 9 May 2017 (2017-05-09), pages 1 - 11, XP055623607**

Remarks:
  •The complete document including Reference Table(s) and the Sequence Listing(s) can be downloaded from the EPO website

•The file contains technical information submitted after the application was filed and not included in this specification

(52)  Cooperative Patent Classification (CPC): (Cont.)
**C12N 15/111; C12N 15/1136; C12Y 301/27005;**
A61K 47/44; A61L 2400/12; A61L 2400/18;
C12N 2310/14; C12N 2320/32

**Description**

[Technical Field]

The present invention relates to a composition for delivering physiologically active ingredients in blood vessels.

[Background Art]

A drug delivery system refers to a medical technology that can efficiently deliver desired amount of drugs such as proteins, nucleic acids or other small molecules by minimizing side effects while maximizing efficacy and effects of existing drugs. This technology, which allows to save costs and time required to develop new drugs, has recently combined with nano-technology thus to become one of advanced technologies that create new added value in the pharmaceutical industry. In the late 1980s, technically developed countries such as United States, Japan, etc., have concentrated upon development of the drug delivery system as well as development of new drugs around businesses such as pharmaceutical companies, etc.

To date, viral genes, recombinant proteins, liposomes, cationic polymers, and diverse types of nanoparticles and nanomaterials have been used for drug delivery into animal cells. However, it has been found that many cationic liposomes and cationic polymers are unsuitable for clinical applications due to their high toxicity to cells. In addition, a method of chemically modifying a main chain of the nucleic acid has been attempted for stable penetration of the nucleic acid into a cell membrane. However, this method is not suitable for clinical applications because it is expensive, time consuming, and requires labor intensive processes. As a significant attempt, a drug delivery system (DDS) utilizing various types of nanoparticles, including quantum dots, magnetic particles, or gold nanoparticles, has been developed. However, there was a disadvantage that these particles are toxic to cells, and have a structure not easy for introduction of biopolymers such as nucleic acids, as well as have low efficiency of introduction into the cells.

An efficient delivery system is needed for studying functions of physiological active substances (or bioactive materials) in cells or for intracellular delivery. However, a universal delivery system capable of delivering a wide range of bioactive materials, a system capable of accommodating and delivering a large amount of drugs and a system for releasing drugs in a sustained manner have yet to be developed.

KR 2014 0010285 relates to a composition for drug delivery which includes RNA absorbed to a porous silica nanoparticle with expanded pores.

[Disclosure]

[Technical Problem]

It is an object of the present invention to provide a composition for delivering physiologically active substances (hereinafter, "bioactive materials") into blood vessels, which includes porous silica particles having stability in blood.

Another object of the present invention is to provide a composition for embolization (often referred to as an "embolic composition"), which includes porous silica particles having biodegradability and sustained release.

[Technical Solution]

1. A composition for delivering a bioactive material in blood vessels, including a porous silica particle, wherein the bioactive material is loaded on a surface of the particle or insides of pores thereof, and the porous silica particle has a zeta potential of +3 mV or more or -18 mV or less, and

the particle is chemically modified on the surfaces of the particle or the insides of the pores, wherein the particle is prepared by a small pore particle preparation, pore expansion process and calcination process, wherein the calcination is performed at 400 to 700 °C for 3 to 8 hours, wherein t when a ratio of absorbance in the following Equation 1 becomes 1/2 is 20 hours or more:

$$[Equation\ 1]$$

$$A_t\ /A_0\ \P$$

(wherein $A_0$ is absorbance of the porous silica particle measured by placing 5 ml of a suspension including 1 mg/ml of the porous silica particle into a cylindrical dialysis membrane having pores with a diameter of 50 kDa, 15 ml of the same solvent as the suspension is placed outside the dialysis membrane while being in contact with the dialysis membrane, followed by horizontal agitation at 60 rpm and 37 °C inside and outside the dialysis membrane,

pH of the suspension is 7.4, and

$A_t$ is absorbance of the porous silica particles measured after t hours elapses from the measurement of $A_0$, wherein the suspension is a buffer solution).

2. The composition according to the above 1,

wherein at least a part of a silanol group on the surface of the particle or the inside of the pore in the particle is substituted with at least one functional group selected from the group consisting of aldehyde, keto, carbamate, sulfate, sulfonate, amino, amine, aminoalkyl, silyl, carboxyl, sulfonic acid, thiol, ammonium, sulfhydryl, phosphate, ester, imide, thioimide, ether, indene, sulfonyl, methylphosphonate, polyethylene glycol, substituted or unsubstituted $C_1$ to $C_{30}$ alkyl, substituted or unsubstituted $C_3$ to $C_{30}$ cycloalkyl, substituted or unsubstituted $C_6$ to $C_{30}$ aryl and $C_1$ to $C_{30}$ ester groups.

3. The composition according to the above 1,

wherein at least a part of a silanol group on the surface of the particle or the inside of the pore in the particle is substituted with at least one functional group selected from the group consisting of amino, amine, PEG, propyl, octyl, carboxyl, thiol, sulfonic acid, methylphosphonate and aldehyde groups.

4. The composition according to the above 1,

wherein the particle has a diameter of 100 to 1000 nm.

5. The composition according to the above 1,

wherein the particle has a zeta potential of +3 mV to +100 mV or -100 mV to -18 mV.

6. The composition according to the above 1,

wherein the particle has a volume of 0.7 to 2.2 ml per gram (g).

7. The composition according to the above 1,

wherein a maximum release amount of the bioactive material loaded on the particle is 99% by weight or more.

8. The composition according to the above 1,

wherein the bioactive material is at least one selected from the group consisting of nucleic acids, nucleotides, proteins, peptides, amino acids, sugars, lipids, compounds, antibodies, antigens, cytokines, growth factors and elements constituting the same.

9. · The composition according to the above 1,

wherein the bioactive material is at least one selected from the group consisting of doxorubicin, irinotecan, sorafenib, adriamycin, daunomycin, mitomycin, cisplatin, epirubicin, methotrexate, 5-fluorouracil, aclacinomycin, nitrogen mustard, cyclophosphamide, bleomycin, daunorubicin, vincristine, vinblastine, vindesine, tamoxifen, valrubisin, pirarubicin, mitoxantrone, gemcitabine, idarubicin, temozolomide, paclitaxel, dexamethasone, aldesleukin, avelumab, bevacizumab, carboplatin, regorafenib, docetaxel, doxil, gefitinib, imatinib mesylate, herceptin, imatinib, aldesleukin, keytruda, opdivo, mitomycin C, nivolumab, olaparib, pembrolizumab, rituximab, sunitinib, atezolizumab, lapatinib and ipilimumab.

10. · The composition according to the above 1,

wherein the composition is released through a catheter into target tissues.

11. · An embolic composition including the composition according to any one of the above 1 to 10.

12. The composition according to the above 11,

further including at least one among contrast agents and embolic agents,

wherein the embolic agent is selected from the group consisting of lipiodol, dextran, polyvinyl alcohol, N-butyl cyanoacrylate, gel foam, gelatin, ethanol, dextran, silica, polysodium acrylate vinylalcohol copolymer, glass particles, poly-L-guluronic alginate, polyglycolic-polyactic acid, polydioxanone, polyglycolic acid-co-caprolactone, and polypropylene.

13. The composition according to the above 11, wherein the composition is released into a blood vessel directly connected to a tumor via a catheter.

[Advantageous effects]

The composition including porous silica particles according to the present invention may effectively deliver a bioactive material to target tissues or cells in the blood stream by modifying surfaces of the particles to inhibit aggregation and precipitation in the blood.

In addition to the above advantage, the embolic composition including porous silica particles according to the present invention has advantages of having specific physical properties such as biodegradability and sustained release thus to achieve excellent embolization effects and targetability toward target tumor tissues or cells, thereby reducing side effects.

[Description of drawings]

FIG. 1 is microphotographs of porous silica particles according to an embodiment of the present invention.

FIG. 2 is microphotographs of porous silica particles according to an embodiment of the present invention.

FIG. 3 is microphotographs of small pore particles in a process for manufacturing the porous silica particles according to an embodiment of the present invention.

FIG. 4 is microphotographs of small pore particles according to an embodiment of the present invention.

FIG. 5 is microphotographs of the porous silica particles by pore diameter according to an embodiment of the present invention.

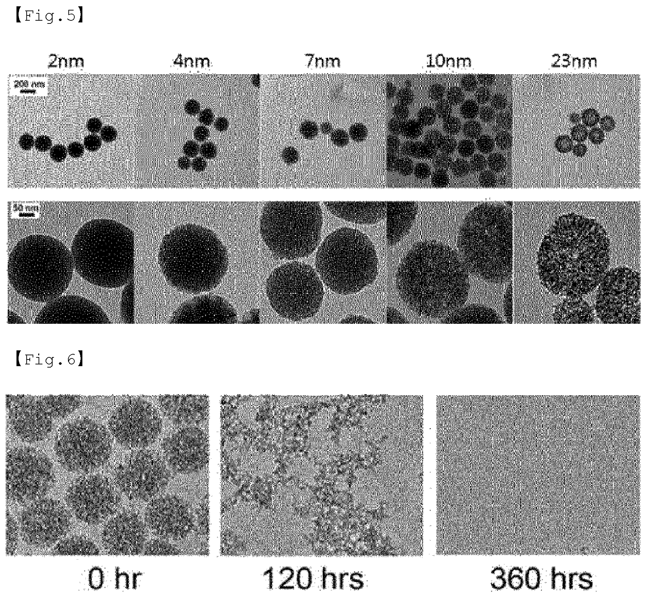

In FIG. 5, a degradable delivery vehicle (DDV) is the particle in the embodiment wherein the number in parenthesis denotes a diameter of the particle and the number of subscripts denotes a pore diameter. For example, DDV $200_{10}$ refers to a particle in the embodiment which has a particle diameter of 200 nm and a pore diameter of 10 nm.

FIG. 6 is microphotographs capable of confirming biodegradability of the porous silica particles according to an embodiment of the present invention.

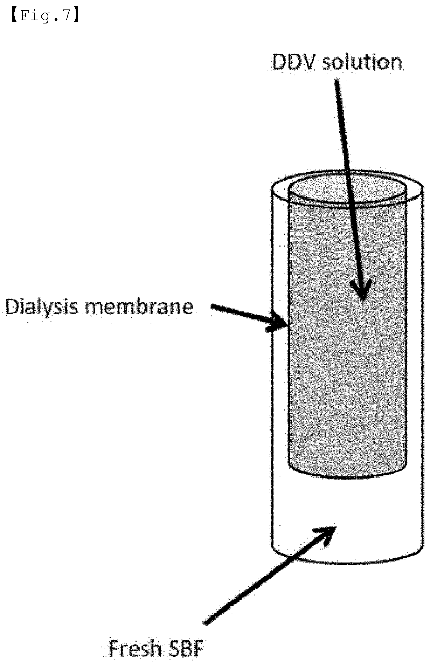

FIG. 7 is a view illustrating a tube provided with a cylindrical dialysis (or permeable) membrane according to one example of the present invention.

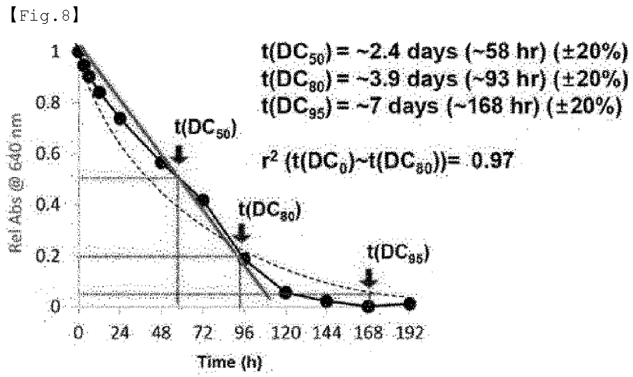

FIG. 8 is a graph illustrating results of decreased absorbance of the porous silica particles over time according to an embodiment of the present invention.

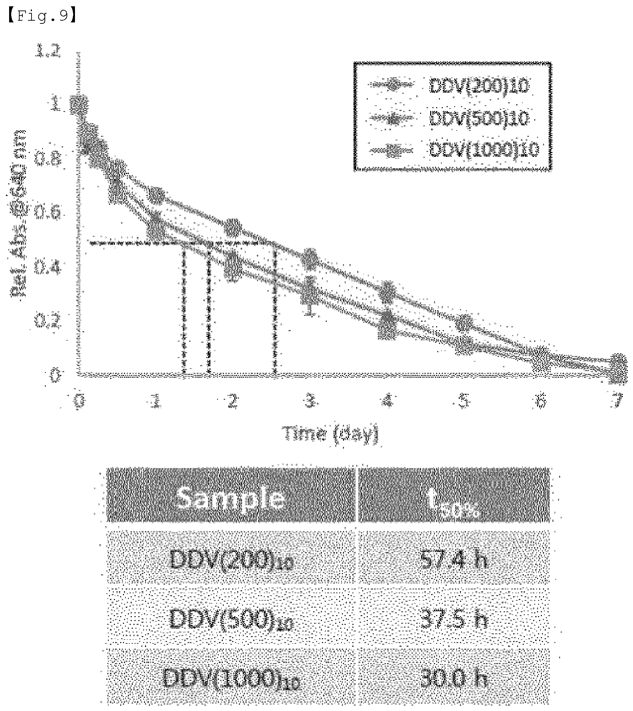

FIG. 9 is a graph and a table illustrating results of decreased absorbance of the porous silica particles by particle diameter over time according to an embodiment of the present invention.

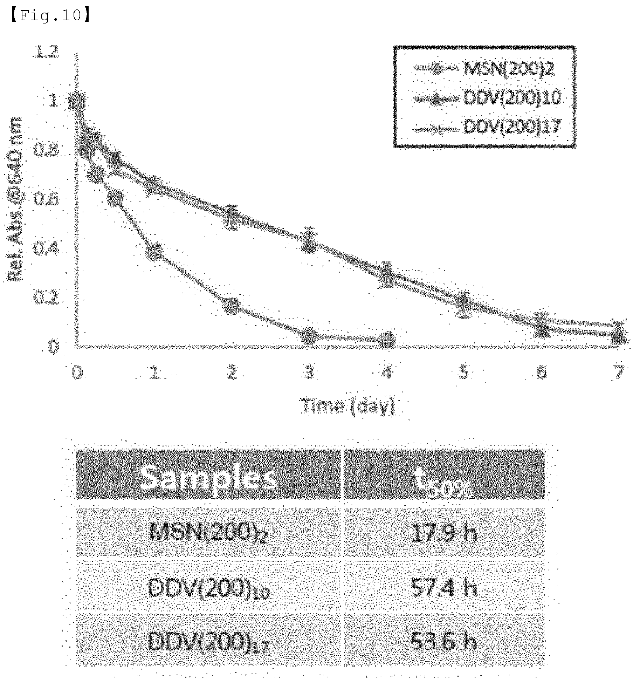

FIG. 10 is a graph and a table illustrating results of decreased absorbance of the porous silica particles by pore diameter over time according to an embodiment of the present invention.

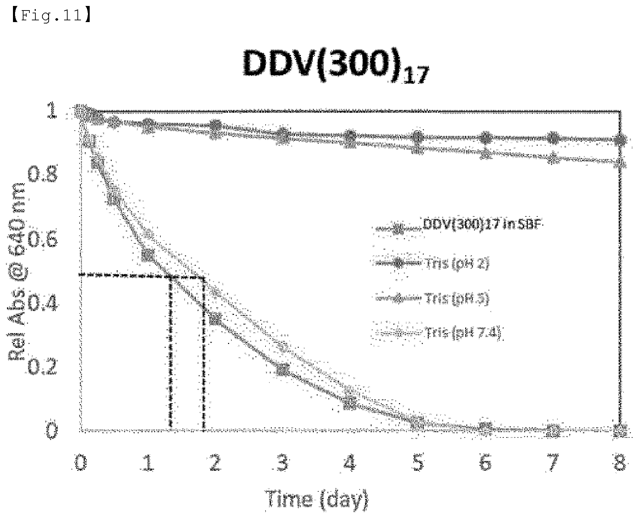

FIG. 11 is a graph illustrating results of decreased absorbance of the porous silica particles by pH of environment over time according an embodiment of the present invention.

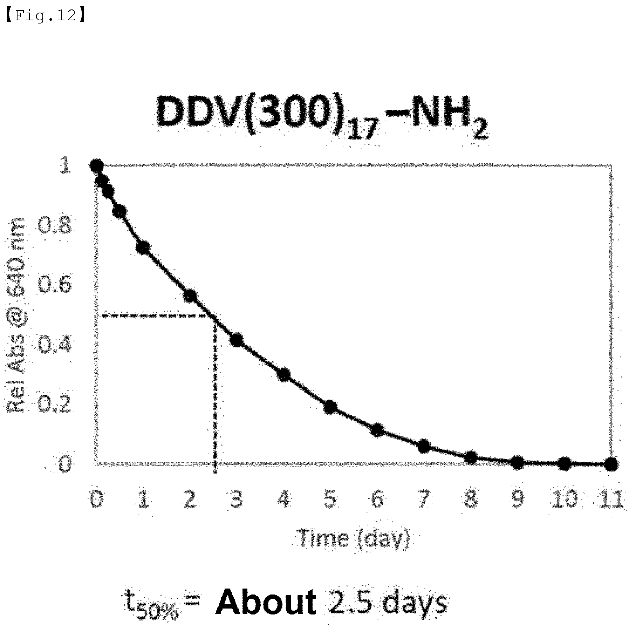

FIG. 12 is a graph illustrating results of decreased absorbance of the porous silica particles according to an embodiment of the preset invention.

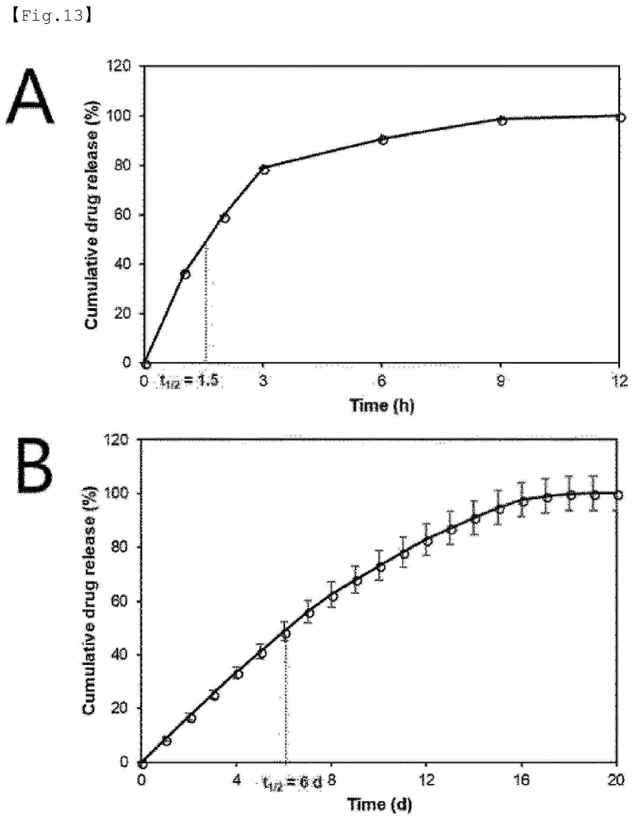

FIG. 13 is graphs illustrating the amount of doxorubicin release from the porous silica particles loaded with doxorubicin under two conditions.

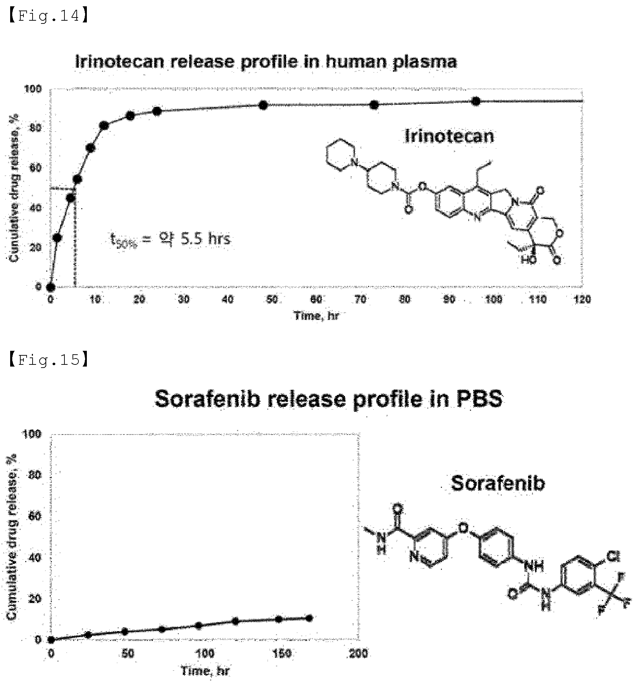

FIG. 14 is a graph illustrating the amount of irinotecan release from the porous silica particles loaded with irinotecan.

FIG. 15 is a graph illustrating the amount of sorafenib release from the porous silica particles loaded with sorafenib.

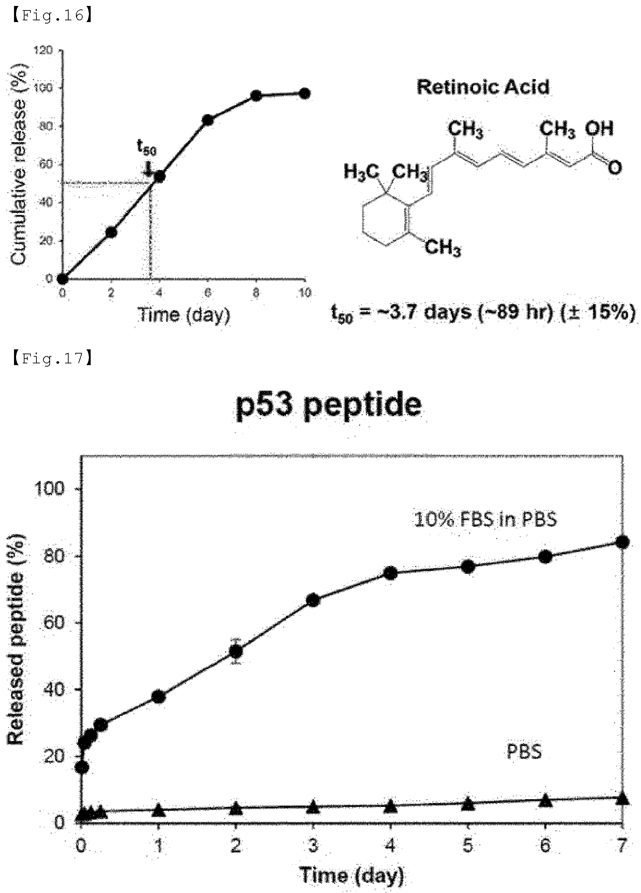

FIG. 16 is a graph illustrating the amount of retinoic acid release from the porous silica particles loaded with retinoic acid.

FIG. 17 is a graph illustrating the amount of p53 protein release from the porous silica particles loaded with p53 protein.

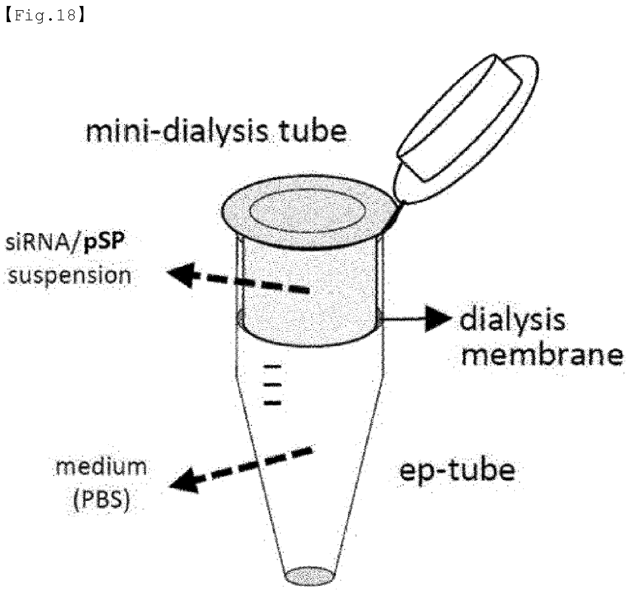

FIG. 18 is a view illustrating a tube for identifying the release of loaded bioactive material.

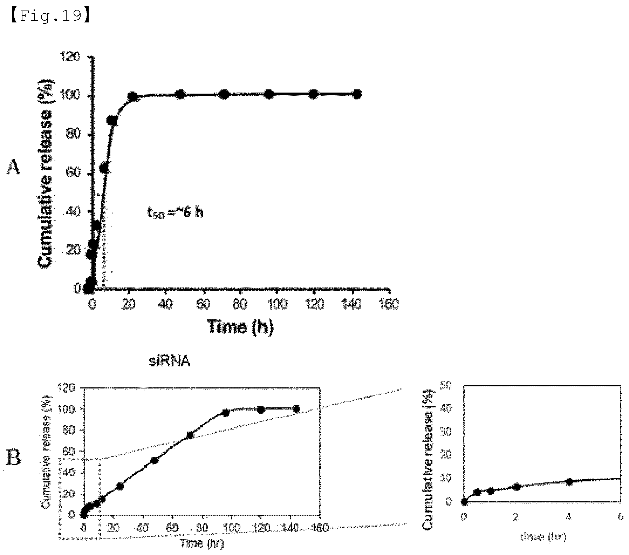

FIG. 19 is a graph illustrating the amounts of siRNA release from the porous silica particles loaded with siRNA.

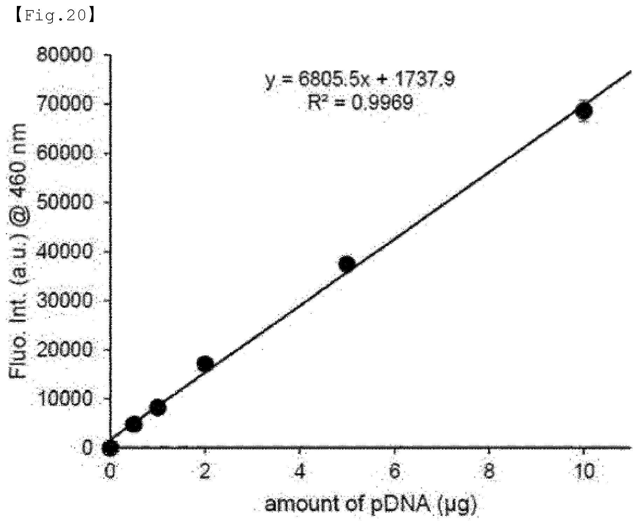

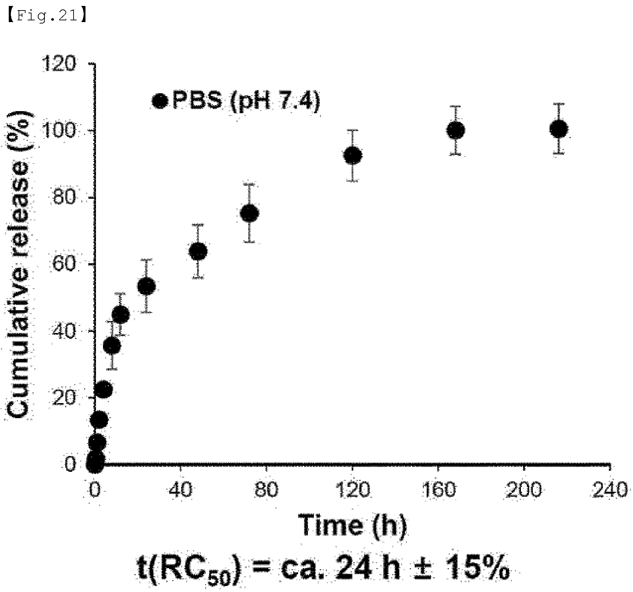

FIGS. 20 and 21 are graphs illustrating the amounts of pDNA release from the porous silica particles loaded with pDNA.

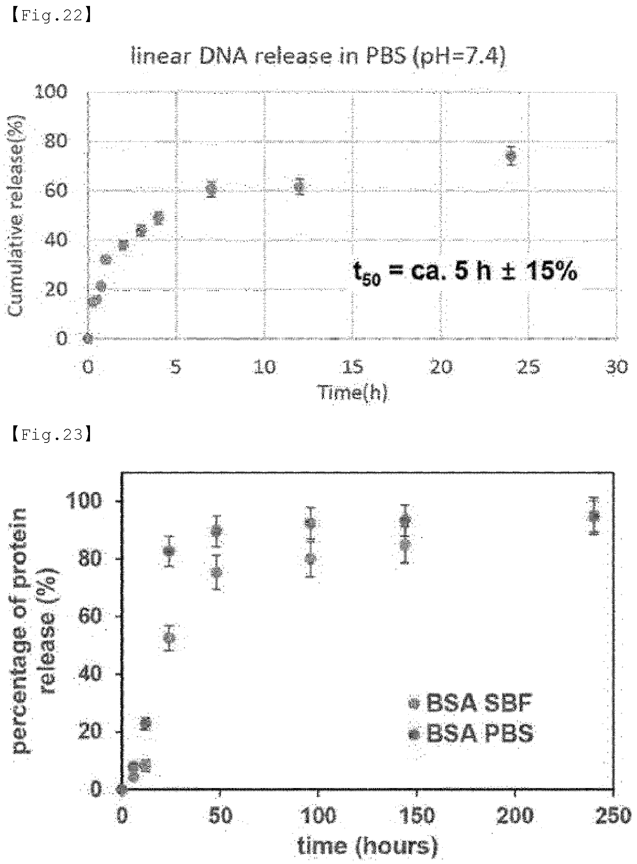

FIG. 22 is a graph illustrating the amount of linear DNA release from the porous silica particles loaded with linear DNA.

FIG. 23 is a graph illustrating the amount of BSA release from the porous silica particles loaded with BSA.

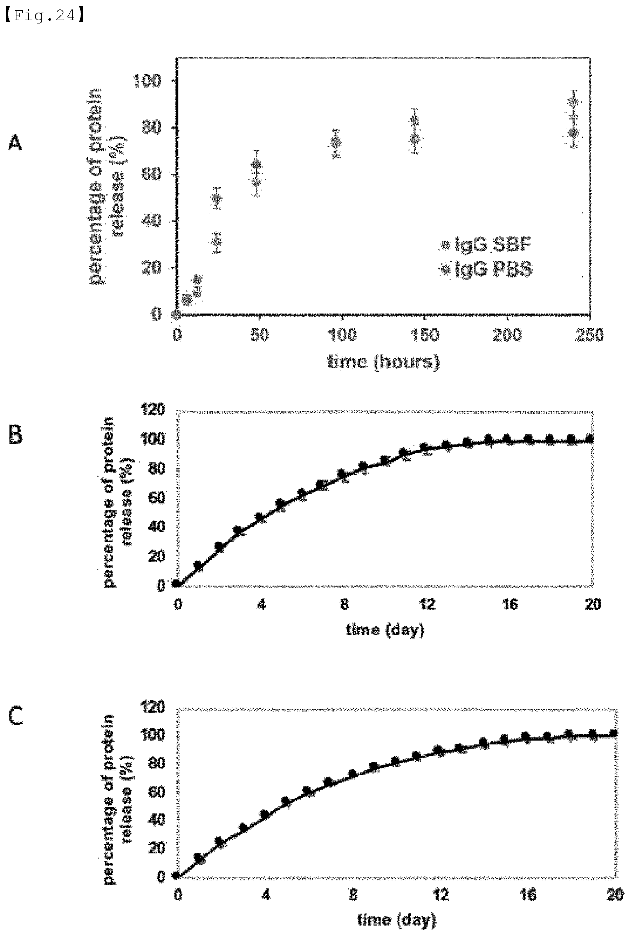

FIG. 24 is graphs illustrating the amounts of IgG, antibody 1 and antibody 2 releases from the porous silica particles loaded with IgG (A), antibody 1 (B) and antibody 2 (C), respectively.

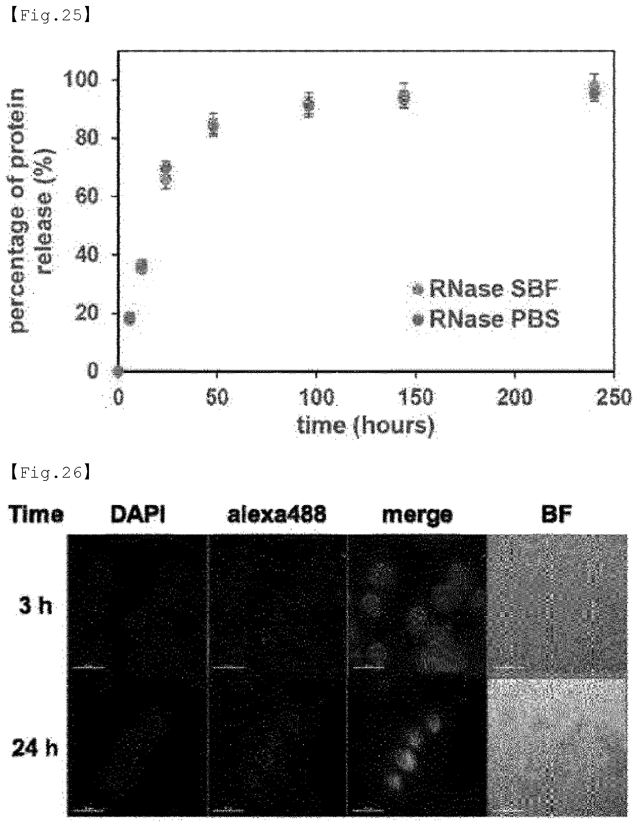

FIG. 25 is a graph illustrating the amount of RNase release from the porous silica particles loaded with RNase.

FIG. 26 is photographs illustrating Cas9 protein loaded on the porous silica particles and delivered into cells.

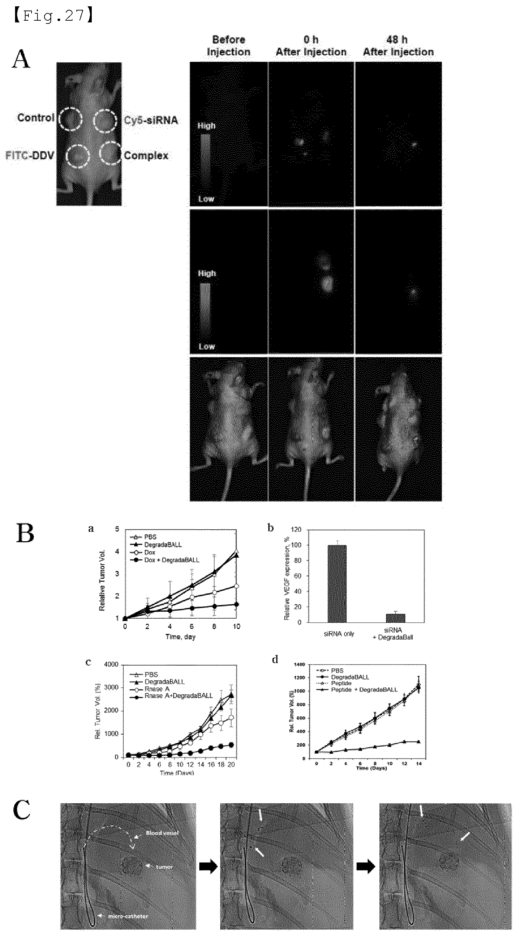

FIG. 27 is photographs and graphs illustrating siRNA loaded on the porous silica particles and released in mice (A); delivery and therapeutic effects of a composition including the porous silica particles loaded with doxorubicin, siRNA, RNase A and peptide in mice (B); and delivery of the composition of the present invention through a catheter (C).

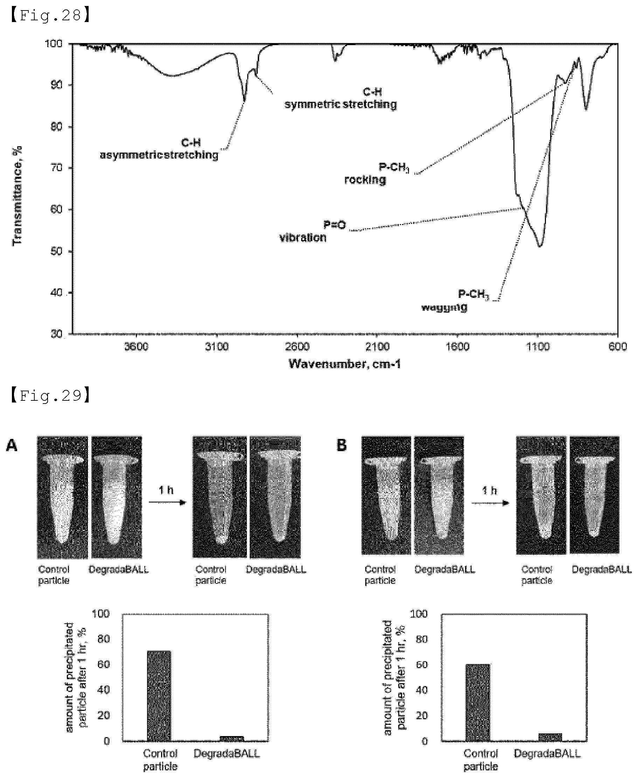

FIG. 28 is a graph illustrating FT-IR spectrum of the porous silica particles modified with anionic functional groups.

FIG. 29 is photographs and graphs illustrating the degree of precipitation of the porous silica particles in a blood-mimic solution.

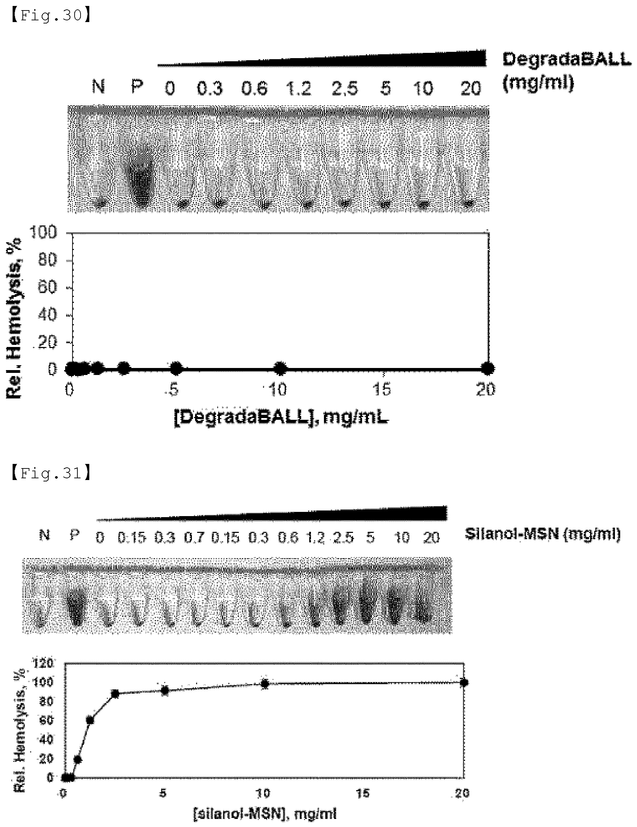

FIG. 30 is views illustrating the degree of erythrocytic hemolysis of the modified porous silica particles.

FIG. 31 is views illustrating the degree of erythrocytic hemolysis of the unmodified porous silica particles.

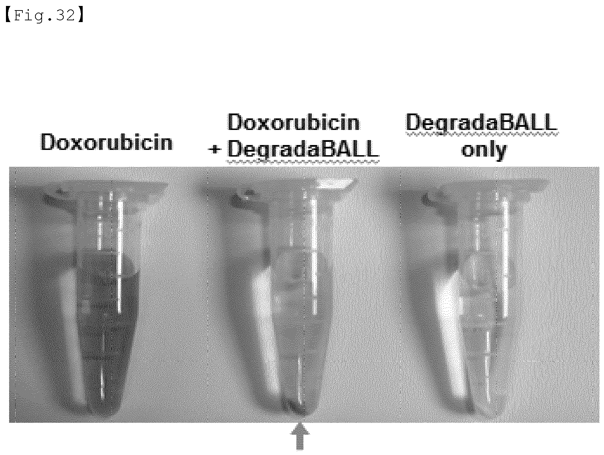

FIG. 32 is views illustrating a loading capacity of doxorubicin to the porous silica particles.

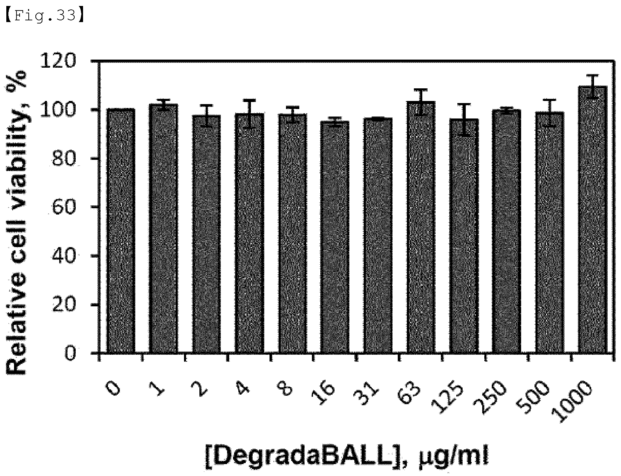

FIG. 33 is a graph illustrating test results of cytotoxicity of the porous silica particles.

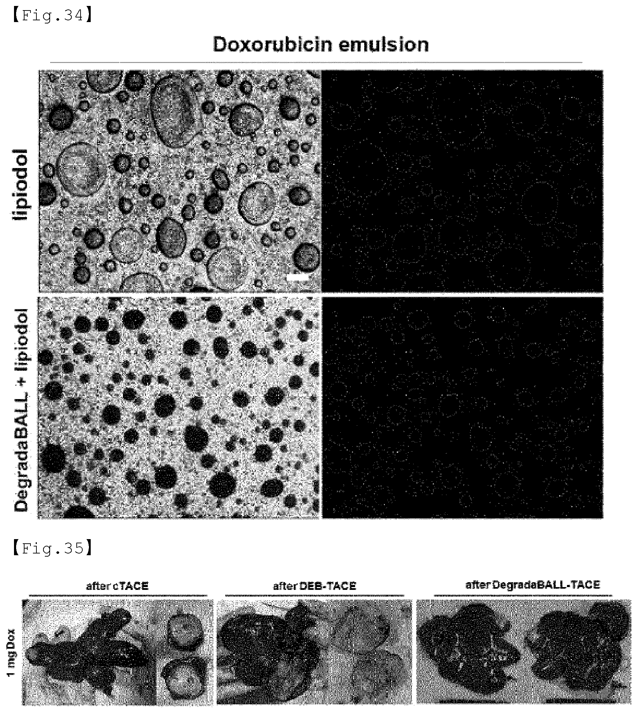

FIG. 34 is photographs illustrating particle stability when mixing the porous silica particles with lipiodol to emulsify.

FIG. 35 is photographs illustrating visually observed results of rabbit liver excised after the embolization using a composition for embolization, which includes the porous silica particles.

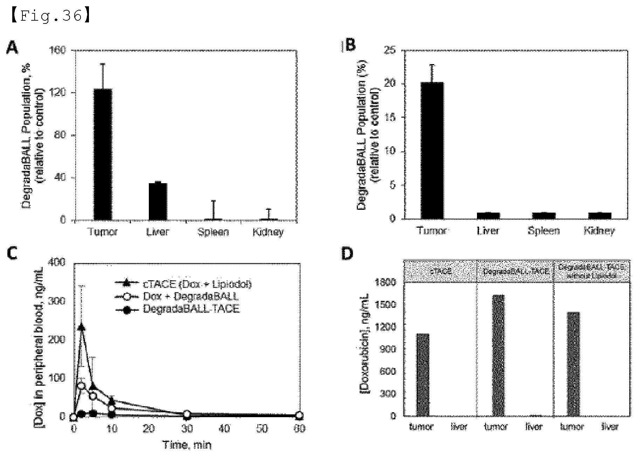

FIG. 36 is graphs illustrating targetabilities of the embolic composition including the porous silica particles to target tissues (A); and to target cells (B); insignificant toxicity of the above composition to surrounding normal cells (C); and targetability of the above composition to target tumors (D), respectively.

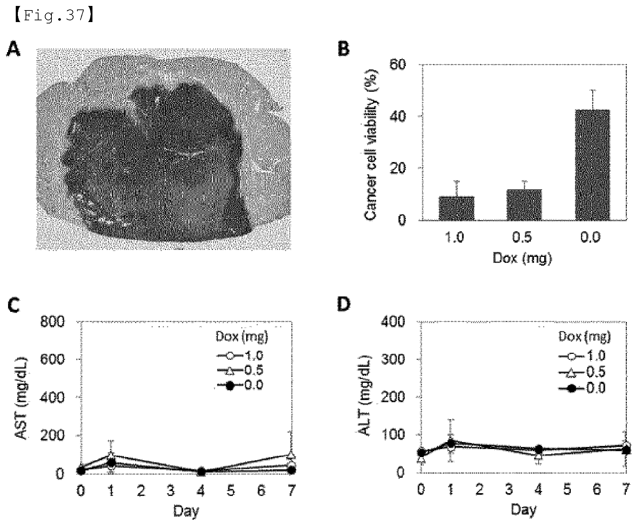

FIG. 37 is views and graphs illustrating low survival rates (A and B) of rabbit liver cancer cells when performing embolization with the embolic composition including the porous silica particles, as well as measured results of AST and ALT concentrations, demonstrating the absence of liver toxicity.

[Best mode]

As used herein, porous silica particles are a fine nanoporous silica microstructure including fine pores in a size ranging from several nanometers to several micrometers, have well defined regularity in pore alignment, and may be suitably controlled in aspects of material properties (pore size, specific surface area, surface properties, etc.) to accommodate to the environment of use. The porous silica particles are also referred to mesoporous silica particles.

Hereinafter, the present invention will be described in detail.

The present invention provides a composition for drug delivery in blood vessels, which includes porous silica particles to load a physiological active ("bioactive") substance on surfaces of the particles or insides of pores thereof while having a zeta potential of +3 mV or more 18 mV or less, wherein the particles are chemically modified on the surfaces of the particles or the insides of the pores, as defined in claim 1.

In the composition of the present invention, the bioactive material is a physiologically active substance/biological function modulator loaded on the porous silica particles and delivered to individuals to exhibit activity, which may include, for example, at least one selected from the group consisting of low molecular weight drugs, genetic drugs, protein drugs, extracts, nucleic acids, nucleotides, proteins, peptides, antibodies, antigens, RNAs, DNAs, PNAs, aptamers, chemicals, enzymes, amino acids, sugars, lipids, compounds (natural and/or synthetic compounds) and components constituting the same, for example, may be at least one selected from the group consisting of doxorubicin, irinotecan, sorafenib, adriamycin, daunomycin, mitomycin, cisplatin, epirubicin, methotrexate, 5-fluorouracil, aclacinomycin, nitrogen mustard, cyclophosphamide, bleomycin, daunorubicin, vincristine, vinblastine, vindesine, tamoxifen, valrubisin, pirarubicin, mitoxantrone, gemcitabine, idarubicin, temozolomide, paclitaxel, dexamethasone, aldesleukin, avelumab, bevacizumab, carboplatin, regorafenib, docetaxel, doxil, gefitinib, imatinib mesylate, herceptin, imatinib, aldesleukin, keytruda, opdivo, mitomycin C, nivolumab, olaparib, pembrolizumab, rituximab, sunitinib, atezolizumab, lapatinib and ipilimumab, but it is not limited thereto. These substances may include specific examples described below.

In the composition of the present invention, the bioactive material may be a therapeutically active agent capable of ensuring direct or indirect, therapeutic, physiological and/or pharmacological effects on a human or animal organism.

The therapeutically active agent may be, for example, typical medicines, drugs, prodrugs or target groups, or drugs or prodrugs including the target groups.

The therapeutically active agent may include, for example: cardiovascular drugs, in particular, antihypertensive agents (e.g., calcium channel blockers, or calcium antagonists) and antiarrhythmic agents; congestive heart failure drugs; muscle contractors; vasodilators; ACE inhibitors; diuretics; deoxidation dehydratase inhibitors; cardiac glycosides; phosphodiesterase inhibitors; blockers; β-blockers; sodium channel blockers; potassium channel blockers; β-adrenergic agonists; platelet inhibitors; angiotensin II antagonists; anticoagulants; thrombolytics; bleeding therapeutics; anemia therapeutics; thrombin inhibitors; antiparasitic agents; antibacterial agents; anti-inflammatory agents, in particular, non-steroidal anti-inflammatory agents (NSAIDs), more particularly, COX-2 inhibitors; steroidal anti-inflammatory agents; prophylactic anti-inflammatory agents; anti-glaucoma; mast cell stabilizer; mydriatic drugs; drugs affecting the respiratory system; allergic rhinitis drugs; alpha-adrenergic antagonists; corticosteroids; chronic obstructive pulmonary disease drugs; xanthine-oxidase inhibitors; anti-arthritis agents; gout therapeutics; potent drugs and potent drug antagonists; anti-TB drugs; antifungal agents; antiprotozoal agents; helminthics; antiviral agents, in particular, respiratory antiviral agents, antiviral agents against herpes, cytomegalovirus, human immunodeficiency virus and hepatitis infections; therapeutics for leukemia and Kaposi's sarcoma; pain controllers, in particular, opioids including anesthetics and analgesics, opioid receptor agonists, opioid receptor partial agonists, opioid antagonists, opioid receptor mixed agonists-antagonists; neuroleptics; sympathomimethic agents; adrenergic antagonists; drugs affecting neurotransmitter absorption and release; anti-cholinergic agents; anti-hemorrhagic agents; prophylactic or therapeutic agents for radiation or chemotherapy effects; adipogenic agents; fat reducing agents; anti-obesity agents such as lipase inhibitors; sympathetic stimulants; gastric ulcer and inflammation therapeutics such as proton pump inhibitors; prostaglandins; VEGF inhibitors; anti-hyperlipidemic agents, in particular, statin; drugs affecting central nervous system (CNS) such as antipsychotic, antiepileptic and antiseizure agents (anticonvulsants), psychoactive agents, stimulants, anti-anxiety and hypnotic agents; antidepressants; anti-Parkinson's agents; hormones such as sexual hormones and fragments thereof; growth hormone antagonists; gonadotropin release hormone and analogs thereof; steroidal hormones and antagonists thereof; selective estrogen modulators; growth factors; antidiabetic agents such as insulin, insulin fragments, insulin analogs, glucagon-like peptides and hypoglycemic agents; H1, H2, H3 and H4 antihistamines; peptides, proteins, polypeptides, nucleic acids and oligonucleotide drugs; analogs, fragments and variants of natural proteins, polypeptides, oligonucleotides and nucleic acids; drugs used for treatment of migraine headaches; asthma medicines; cholinergic antagonists; glucocorticoids; androgen; anti-androgens; inhibitors of adrenocorticoid biosynthesis; osteoporosis therapeutics such as biphosphonates; antithyroid agents; UV blocking agents, UV protectors and filters; cytokine antagonists; antitumor agents; anti-Alzheimer's agents; HMGCoA reductase inhibitors; fibrate; cholesterol absorption inhibitors; HDL cholesterol enhancers; triglyceride reducing agents; anti-aging or anti-wrinkling agents; precursor molecules for development of hormones; proteins such as collagen and elastin, antibacterial agents; anti-acne medications; antioxidants; hair treatments and skin whitening agents; UV blocking agents, UV protectors and filters; variants of human apolipoproteins; precursor molecules for development of hormones; proteins and peptides thereof; amino acids; plant extracts such as grape seed extract; DHEA; isoflavones; nutrients including vitamins, phytosterols and iridoid glycosides, sesquiterpene lactones, terpenes, phenol glycosides, triterpenes, hydroquinone derivatives, phenylalkanones; antioxidants such as retinol and other retinic acids, retinoids including coenzyme Q10; omega-3-fatty acid; glucosamine; nucleic acids, oligonucleotides, antisense drugs; enzymes; coenzymes; cytokine analogs; cytokine agonists; cytokine antagonists; immunoglobulins; antibodies; antibody medications; gene therapy agents; lipoproteins; erythropoietin; vaccine; and low molecular weight therapeutics for treatment or prevention of human or animal diseases such as allergy/asthma, arthritis, cancer, diabetes, growth disorders, cardiovascular disease, inflammation, immune disorders, baldness, pain, ocular disease, epilepsy, gynecological disorders, CNS disease, viral infections, bacterial infections, parasitic infections, GI disease, obesity and blood diseases, but it is not limited thereto.

The therapeutically active agent may be an additional active agent including, for example, erythropoietine (EPO), thrombopoietine, cytokine such as interleukin (including IL-1 to IL-17), insulin, insulin-like growth factors (including IGF-1 and IGF-2), epidermal growth factor (EGF), transforming growth factor (including TGF-alpha and TGF-beta), human growth hormone, transferrin, low density lipoprotein, high density lipoprotein, leptin, VEGF, PDGF, ciliary neurotrophic factor, prolactine, adrenocorticotropic hormone (ACTH), calcitonine, human chorionic gonadotropin, cortisol, estradiol, follicle stimulating hormone (FSH), thyroid-stimulating hormone (TSH), luteinizing hormone (LH), progesterone, testosterone, toxins including ricin and the like.

The therapeutically active agent may be selected from the group of drugs for treatment of oncological diseases or cellular or tissue modifications. Suitable therapeutic agents may be anti-neoplastic agents including, for example: alkylating agents, in particular, alkyl sulfonates such as busulfan, improsulfan, piposulfane, benzodepa, carboquone, metredepa, arizidine such as uredepa, etc.; ethyleneimine and methylmelamine such as altretamine, triethylene melamine, triethylene phosphoramide, triethylene thiophosphoramide, trimethylolmelamine, etc.; chlorambucil, chlornaphazine, cyclophosphamide, estramustine, ifosfamide, mechlorethamine, mechlorethamine oxide hydrochloride, melphalan, nomobichin, phenesterine, prednimustine, trofosfamide, so-called nitrogen mustard such as uracil mustard, etc.; nitroso urea compounds such as carmustine, chlorozotocin, fotenmustine, lomustine, nimustine, ranimustine, etc.; dacarbazine, mannomustine, mitobranitol, mitolactol, etc.; pipobroman; sorafenib; doxorubicin and cis-platinum and derivatives thereof, etc., as well as any combination and/or derivatives of the foregoing compounds.

The therapeutically active agent may be selected from the group including antiviral agents and antibacterial agents, for example, aclacinomycin, actinomycin, anthramycin, azaserin, bleomycin, cuctinomycin, carubicin, carzinophilin, chromomycin, ductinomycin, daunorbicin, 6-diazo-5-oxn-1-norleucin, doxorubicin, epirubicin, mitomycin, mycophenolsaure, mogalumycin, olivomycin, peplomycin, plicamycin, porfiromycin, puromycin, streptonigrin, streptozocin, tubercidine, ubenimex, zinostatin, zorubicin, aminoglycoside or polyene, macrolid-antibiotics, and any combination and/or derivatives thereof.

The therapeutically active agent may be selected from endostatin, angiostatin, interferon, platelet factor 4 (PF4), thrombospondin, transforming growth factor beta, tissue inhibitor of metalloproteinase-1, -2 and -3 (TIMP-1, -2 and -3), TNP-470, marimastat, neovastat, BMS-275291, COL-3, AG3340, thalidomide, squalamine, combrestastatin, SU5416, SU6668, IFN-[alpha], EMD121974, CAI, IL-12, radio-sensitizer drugs such as IM-862, steroidal or non-steroidal anti-inflammatory drugs, or formulations relating to angiogenesis, and any combination and/or derivatives thereof.

The therapeutically active agent may be selected from the group including nucleic acids, wherein the term of "nucleic acid" includes oligonucleotides wherein at least two nucleotides are covalently linked to each other, so as to acquire gene therapeutic or antisense effects. The nucleic acid preferably has a phosphodiester bond and includes analogs having different backbones. The analog may have a backbone including, for example, phosphoramide phosphorothioate, phosphorodithioate, O-methylphosphoroamidit-compound, and peptide-nucleic acid backbones and compounds thereof, etc. Other analogs are those having an ionic backbone, a nonionic backbone or a non-ribose-backbone, respectively. The nucleic acids containing one or more carbocycic sugars may be suitable as nucleic acids used in the present invention. Other than selection of nucleic acids and nucleic acid analogs known in the art, any combination of naturally occurring nucleic acids and analogs thereof or mixtures of nucleic acids and analogs thereof may be used.

The therapeutically active agent may include anti-migratory, anti-proliferative or immune-suppresive, anti-inflammatory or re-endotheliating agents, such as everolimus, tacrolimus, sirolimus, mycophenolate-mofetil, rapamycin, paclitaxel, actinomycine D, angiopeptin, batimastate, estradiol, VEGF, statins, and derivatives and analogs thereof.

The therapeutically active agent may include opioid receptor agonists and antagonists, compounds exhibiting agonistic/antagonistic combined activity, and compounds exhibiting partially agonistic activity, for example: morphine, depomorphine, etropin, diacetyl morphine, hydromorphine, oxymorphone, levorpanol, methadone, levomethadyl, meperidine, fentanyl, serpentanyl, alpentanyl, codeine, hydrocodone, oxycodone, thebaine, desormorphine, nicomorphine, dipropanoylmorphine, benzylmorphine, ethylmorphine, petidine, methadone, tramadol, dextropropoxyphene; naloxone and naltrexone; and buprenorphine, nalbuphine, butorpanol, pentazoxin and ethyl ketocyclazoxin.

The therapeutically active agents and combinations thereof may be selected from: heparin, synthetic heparin analogs (e.g., fondaparinux), hirudin, antithrombin III, drotrecogin alpha; fibrinolytics such as alteplase, plasmin, lysokinase, factor VIIa, prourokinase, urokinase, anistreplase, streptokinase, etc.; platelet aggregation inhibitors such as acetylsalicylic acid (aspirine), ticlopidine, clopidogrel, abciximab, dextran, etc.; corticosteroids such as alclometasone, amcinonide, augmented betamethasone, beclomethasone, betamethasone, budesonide, cortisone, clobetasol, clocortolone, desonide, desoximetasone, dexamethasone, fluocinolone, fluocinonide, fluandrenolide, flunisolide, fluticasone, halcinonide, halobetasol, hydrocortisone, methylprednisolone, momethasone, prednicarbate, prednisone, prednisolone, triamcinolone, etc.; non-steroidal anti-inflammatory drugs (NSAIDs) such as diclofenac, diflunisal, etodolac, fenoprofen, flurbiprofen, ibuprofen, indomethacin, ketoprofen, ketorolac, meclofenamate, mefenamic acid, meloxicam, nabumetone, naproxen, oxaprozin, piroxicam, salsalate, sulindac, tolmetin, celecoxib, rofecoxib, etc.; cytostatics such as alkaloide, for example, vinblastine, vincristine, etc. and podophyllum toxin, etc.; cytotoxic antibiotics such as daunorbicin, doxorubicin, other anthracycline and related substances, bleomycin, mitomycin, etc.; antimetabolites such as folic acid analogs, purine analogs or pyrimidine analogs, etc.; paclitaxel, docetaxel, sirolimus, etc.; platinum compounds such as carboplatin, cisplatin, oxaliplatin, etc.; amsacrin, irinotecan, imatinib, topotecan, interferon-alpha 2a, interferon-alpha 2b, hydroxycarbide, miltefosine, pentostatin, porfimer, aldesleukin, bexaroten, tretinoin; antiandrogens and antiestrogens; antiarrhythmics such as quinarine type antiarrhythmic, specifically, type I antiarrhythmics such as quinidine type antiarrhythmic, quinidine, disopyramid, azmaline, prajmalium bitartrate, detajimium bitartrate, etc.; for example, lidocaine type antiarrhythmics such as lidocaine, mexiletin, phenytoin, tocainid, etc.; for example, type Ic antiarrhythmics such as propafenone and flecainide (acetate), etc.; type II antiarrhythmic beta-receptor blockers such as metoprolol, esmolol, propranolol, atenolol, oxprenolol, etc.; type III antiarrhythmics such as amiodarone and sotalol; type IV antiarrhythmics such as diltiazem, verapamil, gallopamil, etc.; other antiarrhythmics such as adenosine, orciprenaline, ipratropium bromide, etc.; formulations that stimulate angiogenesis in myocardium, such as vascular endothelial growth factor (VEGF), basic fibroblast growth factor (bFGF), non-viral DNA, viral DNA, endothelial growth factor, etc.; FGF-1, FGF-2, VEGF, and TGF; antibiotics, monoclonal antibodies, anticalin; stem cells, endothelial progenitor cells (EPCs); digitalis glycosides such as acetyl digoxin/methyldigoxin, digitoxin, digoxin, etc.; cardiac glycosides such as ouabain, proscillaridin, etc.; antihypertensive agents such as methyldopa, CNS active antiadrenergic substances as an imidazoline receptor agonist, etc.; calcium channel blockers such as nifedipine, nitrendipine, etc.; ACE inhibitors; quinaprilate, cilazapril, moexipril, trandolapril, spirapril, imidapril; angiotensin II antagonists; candesartancilexetil, valsartan, telmisartan, olmesartanmedoxomil, eprosartan; peripherally active alpha-receptor blockers such as prazosin, urapidil, doxazosin, bunazosin, terazosin, indoramin, etc.; vasodilatators such as dihydralazine, diisopropylamine dichloraetate, minoxidil, nitroprusside sodium, etc.; other antihypertensive agents such as indapamide, co-dergocrine mesylate, dihydroergotoxin methanesulfonate, cicletanin, bosetan, fludrocortisones, etc.; antihypertensive agents such as phosphodiesterase inhibitors, for example, milrinon and enoximon, specifically, adrenergic and dopaminergic substances such as dobutamine, ephinephrine, etilefrine, norfenefrine, norepinephrine, oxilofrine, dopamine, midodrine, pholedrine, methyl ameziniumm, etc.; partial adrenoceptor agonists such as dihydroergotamine; inflammatory cytokines such as fibronectin, polylysine, ethylene vinyl acetate, TGF$\beta$, PDGF, VEGF, bFGF, TNF$\alpha$, NGF, GM-CSF, IGF-a, IL-1, IL-8, IL- 6, growth hormone, etc.; in addition, adhesive substances such as cyanoacrylate, beryllium, silica, etc.; in addition, growth factors such as erythropoetin, hormones such as corticotropin, gonadotropin, somatotropin, thyrotrophin, desmopressin, terlipressin, pxytocin, cetrorelix, corticorelin, leuprorelin, triptorelin, gonadorelin, ganirelix, buserelin, naarelin, goserelin, etc., and regulatory peptides such as somatostatin, octreotid, etc.; bone and cartilage stimulating peptides, recombinant human BMP-2 (rhBMP-2), recombinant BMPs such as bisphophonates (e.g. risedronate, pamidronate, ibandronate, zoledronic acid, clodronic acid, etidronic acid, alendronic acid, tiludronic acid, etc., bone morphogenetic proteins (BMPs) which are fluorides such as disodium fluorophosphate, sodium fluoride, etc.; calcitonin, dihydrotachystyrol; epidermal growth factor (EGF), platelet-derived growth factor (PDGF), fibroblast growth factor (FGFs), transforming growth factor-b (TGFs-b), transforming growth factors-a (TGFs-a), erythropoietin (EPO), insulin-like growth factor-I (IGF-I), insulin-like growth factor-II (IGF-II), interleukin-1 (IL-1), interleukin-2 (IL 2)), interleukin-6 (IL-6), interleukin-8 (IL-8), tumor necrosis factor-a (TNF-a) tumor necrosis factor-b (TNF-b), interferon-g (INF-g), colony stimulating factors (CSFs); monocyte chemotactic protein, fibroblast stimulating factor 1, histamine, fibrin or fibrinogen, endothelin-1, angiotensin II, collagen, bromocriptine, metysergide, methotrexate, carbon tetrachloride, thioacetamide and ethanol; in addition, silver (ion), titanium dioxide, specifically, for example, β-lactamase-sensitive penicillin such as benzyl penicillin (penicillin G), phenoxymethyl penicillin (penicillin V), etc.; for example, β-lactamase-resistant penicillin such as amoxicillin, ampicillin, bacampicillin, etc.; acylaminopenicillins such as mezlocillin and piperacillin; carboxypenicilins such as cefazoline, cefuroxim, cefoxitin, cefotiam, cefaclor, cefadroxil, cefalexin, loracarbef, cefixim, cefuroximaxetil, ceftibuten, cefpodoximproxetil, etc.; aztreonam, ertapenem, meropenem; β-lactamase inhibitors such as sulbactam and sulfamicillintosylate; tetratracyclines such as doxycycline, minocycline, tetracycline, chlorotetracycline, oxytetracycline, etc.; aminoglycosides such as gentamicin, neomycin, streptomycin, tobramycin, amikacin, netilmicin, paromomycin, framyceetin, spectinomycin, etc.; macrolide antibiotics such as azithromycin, clarithromycin, erythromycin, roxithromycin, spiramycin, josamycin, etc.; lincosamides such as clindamycin and lincomycin; for example, gyrase inhibitors such as ciprofloxacin, ofloxacin, moxifloxacin, norfloxacin, gatifloxacin, enoxacin, fleroxacin, levofloxacin which is fluoroquinolone, etc.; quinolones such as pipemidic acid; sulfonamide, trimethoprim, sulfadiazine, sulfflalene; glycopeptide antibiotics such as vancomycin and teicoplanin; polypeptide antibiotics such as polymyxin, for example, colistin and polymyxin-b, nitroimidazole derivatives, for example, metronidazole and tinidazole; aminoquinolones such as chloroquine, mefloquine, hydroxychloroquine, etc.; biguanides such as proguanil; qunine alkaloid such as pyrimethamine, and diaminopyrimidine; amphenicol such as chloramphenicol; rifabutin, dapson, fusidic acid, fosfomycin, nifuratel, telithromycin, fusafungin, pentamidine diisethionate, rifampicin, taurolidine, atovaquone, linezolid; virus statics such as aciclovir, ganciclovir, famciclovir, foscarnet, inosine-(dimefranol-4-acetamidobenzoate), valganciclovir, valaciclovir, cidofovir, brivudin, etc.; antiretroviral active ingredients (nucleosideanalog reverse-transcriptase inhibitors and derivatives) such as lamivudine, zalcitabine, didanosine, zidovudin, tenofovir, stavudin, avacavir, etc.; non-nucleoside analog reverse-transcriptase inhibitors; amprenavir, indinavir, saquinavir, lopinavir, ritonavir, nelfinavir; and amantadine, ribavirine, zanamivir, oseltamivir or lamivudine, and any combinations and mixtures thereof.

The therapeutically active agent may be antidepressants, antipsychotics or anti-anxiety agents, including, for example: alprazolam, amoxapine, bentazepam, bromazepam, clolazepine, clobazam, clotiazepam, diazepam, lorazepam, flunitrazepam, flulazepam, lormetazepam, medazepam, nitrazepam, oxazepam, temazepam, maprotiline, myanserine, nortriptyline, risperidone, sertraline, trazodone, valoperidol, trimipramine maleate fluoxetine, ondansetron, midazolam, chlorpromazine, haloperidol, triazolam, clozapine, fluoropromazine, fluphenazine decanoate, fluanisone, perfenazine, pimozide, prochlorperazine, sulpiride, thioridazine, paroxetine, citalopram, bupropion, phenelzine, olanzapine, divalproex sodium and venlafaxine.

The therapeutically active agent may include opioid receptor agonists and antagonists, compounds exhibiting agonistic/antagonistic combined activity, and compounds exhibiting partially agonistic activity, for example: morphine, depomorphine, etropin, diacetyl morphine, hydromorphine, oxymorphone, levorpanol, methadone, levomethadyl, meperidine, fentanyl, serpentanyl, alpentanyl, codeine, hydrocodone, oxycodone, thebaine, desormorphine, nicomorphine, dipropanoylmorphine, benzylmorphine, ethylmorphine, petidine, methadone, tramadol, dextropropoxyphene; naloxone and naltrexone; and buprenorphine, nalbuphine, butorpanol, pentazoxin and ethyl ketocyclazoxin.

The therapeutically active agent may be tricyclic compounds including, for example, azothiophene, amitriptyline, famotidine, promethazine, paroxetine, oxcarbazepine and mirtazapine.

The therapeutically active agent may be antidiabetic agents including, for example, acetohexamide, chlorpropamide, glibenclamide, gliclazide, glipizide, metformin, tolazamide, glimepiride and tolbutamide.

The therapeutically active agent may be antiepileptic agents including, for example, beclamide, carbamazepine, gabapentin, tiagabine, vigabatrin, topiramate, clonazepam, ethotoin, metodine, methsuximide, methyl phenobarbitone, oxycarbazepine, paramethadione, phenacemide, phenobarbitone, phenyltoin, phensuximide, primidone, sulthiamine, phenytoin sodium, nitrofurantoin monohydrate, gabapentin, lamotrigine, zonisamide, ethosuximide and valproic acid.

The therapeutically active agent may be hypnotics/sedatives and/or muscle relaxants including, for example, zolpidem tartrate, amylobarbitone, barbitone, butobarbitone, pentobarbitone, brotizolam, carbromal, chlordiazepoxide, chlormethiazole, ethinamate, meprobamate, methaqualone, cyclobenzaprene, cyclobenzaprine, tizanidine, baclofen, butalbital, zopiclone, atracurium, tubocurarine and phenobarbital.

The therapeutically active agent may be antifungal, antiprotozoal or antiparasitic agents including, for example: amphotericin, butoconazole nitrate, clotrimazole, econazole nitrate, fluconazole, flucytosine, griseofulvin, itraconazole, ketoconazole, miconazole, natamycin, nystatin, sulconazole nitrate, terconazole, thioconazole and undecenoic acid; benznidazole, clioquinol, decoquinate, diiodohydroxyquinoline, diloxanide furoate, dinitolmide, furazolidone, metronidazole, nimorazole, nitrofurazone, ornidazole, terbinafine, clotrimazole, chloroquine, mefloquine, itraconazole, pyrimethamine, praziquantel, quinacrine, mebendazole and tinidazole.

The therapeutically active agent may be antihypertensive or heart therapeutic agents including, for example, candesartan, hydralazine, clonidine, triamterene, felodipine, gemfibrozil, fenofibrate, nifedical, prazosin, mecamylamine, doxazosin, dobutamine and cilexetil.

The therapeutically active agent may be antimigraine agents including, for example, dihydroergotamine mesylate, ergotamine tartrate, methisergide maleate, pizotifen maleate and sumatriptan succinate.

The therapeutically active agent may be anti-muscarine agents including, for example, atropine, benzhexol, biferdene, ethopropazine, hyoscyamine, mepenzolate bromide, oxybutynin, oxyphencyclimine and tropicamide.

The therapeutically active agent may be anti-neoplastic agents (or immunosuppressive agents) including, for example, aminoglutethimide, amsacrine, azathioprine, busulfan, chlorambucil, cyclosporin, dacarbazine, estramustine, etoposide, lomustine, melphalan, mercaptopurine, methotrexate, mitomycin, mitotane, mitoxanthrone, procarbazine, tamoxifen citrate, testolactone, tacrolimus and sirolimus.

The therapeutically active agent may be anti-Parkinson's agents including, for example, bromocriptine mesylate, levodopa, tolcapone, ropinitrol, bromocriptine, hypoglycemic agents such as sulfonylurea biguanide, alpha-glucosidase inhibitor, thiazolidinedione, cabergoline, carbidopa and lisuride maleate.

The therapeutically active agent may be antithyroidal agents including, for example, carbimazole and propylthiouracil.

The therapeutically active agent may be cardiac muscle contractors including, for example, amrinone, milrinone, digitoxin, enoximone, lanatoside C and medigoxin.

The therapeutically active agent may be hypolipidemia or hyperlipidemia therapeutic agents including, for example, fenofibrate, clofibrate, probucol, ezetimibe and torcetrapib.

The therapeutically active agent may be anti-inflammatory agents including, for example, meloxycam, triamcinolone, cromolyn, nedocromil, hydroxychloroquine, montelukast, zileuton, zafirlukast and meloxicamp.

The therapeutically active agent may be antihistamine agents including, for example, fexofenadine, chloral hydrate, hydroxyzine, promethazine, cetirizine, cimetidine, cyclizine, meclizine, dimenhydrinate, loratadine, nizatadine and promethazine.

The therapeutically active agent may be anti-ulcer agents including, for example, omeprazole, lansoprazole, pantoprazole and ranitidine.

The therapeutically active agent may be diuretics including, for example, hydrochlorothiazide, amyloride, acetazolamide, furosemide and torsemide.

The therapeutically active agent may be retinoids including, for example: first occurring retinoids such as retinol, retinal, tretinoin (retinoic acid, retin-A), isotretinoin and alitretinoin; second occurring retinoids such as etretinate and its metabolite, that is, acitretin; third occurring retinoids such as tazarotene, bexarotene and adapalene.

The therapeutically active agent may be statins and/or derivatives thereof including, for example, atorvastatin, fluvastatin, lovastatin, nystatin, rosuvastatin, pravastatin, orlistat and simvastatin.

The therapeutically active agent may be stimulants including, for example, amfetamine, pentamine, tyramine, ephedrine metaraminol, phenylephrine, dexamfetamine, dexfenfluramine, fenfluramine, nicotine, caffeine and marginol.

The therapeutically active agent may be vasodilators including, for example, carvedilol, terazosin, phentolamine and menthol.

The therapeutically active agent may be anti-Alzheimer's agents including, for example, levetiracetam, levitiracetam and donepezil.

The therapeutically active agent may be ACE inhibitors including, for example, benzapril, enalapril, ramipril, fosinopril sodium, lisinopril, minoxidil, isosorbide, ramipril and quinapril.

The therapeutically active agent may be beta-adrenergic receptor antagonists including, for example, atenolol, timolol, pindolol, pronanolol hydrochloride, bisoprolol, esmolol, metoprolol succinate, metoprolol and metoprolol tartrate.

The therapeutically active agent may be angiotensin II antagonists including losartan.

The therapeutically active agent may be platelet inhibitors including, for example, abciximab, clopidogrel, tirofiban and aspirin.

The therapeutically active agent may be alcohols or phenols including, for example, tramadol, tramadol hydrochloride, allopurinol, calcitriol, cilostazol, sotalol, ursodiol, bromperidol, droperidol, flupenthixol decanoate, albuterol, albuterol sulfate, carisoprodol, clobetasol, ropinirol, labetalol and methocarbamol.

The therapeutically active agent may be ketones or esters including, for example, amiodarone, fluticasone, spironolactone, prednisone, trazodone, desoxymethasone, methyl prednisolone, benzonatate nabumetone and buspirone.

The therapeutically active agent may be antiemetic agents including, for example, metoclopramide.

The therapeutically active agent may be ocular therapeutic agents including, for example, dorzolamide, brimonidine, olopatadine, cyclopentolate, pilocarpine and ecothiopate.

The therapeutically active agent may be anticoagulant or antithrombotic agents including, for example, warfarin, enoxaparin and lepirudin.

The therapeutically active agent may be gout therapeutic agents including, for example, probenesin and sulfinpyrazone.

The therapeutically active agent may be COPD or asthma therapeutic agents including, for example, ipratropium.

The therapeutically active agent may be osteoporosis therapeutic agents including, for example, raloxifene, pamidronate and risedronate.

The therapeutically active agent may be peptides for cosmetics including, for example, acetyl hexapeptide-3, acetyl hexapeptide-8, acetyl octapeptide and l-carnosine.

The therapeutically active agents may include, for example: vaccines including toxoids (inactivated toxic compounds); proteins, protein subunits and polypeptides; polynucleotides such as DNAs and RNAs; conjugates; vaccines including saponins, virosomes, inorganic and organic adjuvants such as zostavax.

The therapeutically active agent may be nutritional or cosmetic active substances including, for example: coenzyme Q10 (or ubiquinone), ubiquinol or resveratrol; carotenoids such as $\alpha$, $\beta$ or $\gamma$-carotene, lycopene, lutein, zeaxanthin and astaxanthin; Phytonutrients such as lycopene, lutein and thioxanthin; omega-3 fatty acids, including linoleic acid, conjugated linoleic acid, docosahexaenoic acid (DHA) and ericosapentaenoic acid (EPA) and their glycerol-esters; fat-soluble vitamins, including vitamin D (D2, D3 and derivatives thereof), vitamin E ($\alpha$, $\beta$, $\gamma$, $\delta$-tocopherol, or $\alpha$, $\beta$, $\gamma$, $\delta$-tocotrienol), vitamin A (retinol, retinal, retinoic acid and derivatives thereof), vitamin K (K1, K2, K3 and derivatives thereof), capric/caprylic triglycerides, folic acid, iron, niacin, glyceryl linoleate, omega-6 fatty acids, vitamin F, selenium, cyanocobalamin, aloe vera, beta glucan, bisabolol, camellia thea (green tea) extract, gotu kola extract, cetearyl olivate, chlorophyll, orange oil, cocoyl proline, dicapryl ether, disodium lauriminodipropionate tocopheryl phosphate (vitamin E phosphate), glycerin, glyceryl oleate, licorice extract, witch hazel extract, lactic acid, lecithin, lutein, macadamia seed oil, chamomile extract, evening primrose oil, olive leaf extract, rice bran oil, avocado oil, milkweed extract, pomegranate sterol, resveratrol, rose oil, sandalwood oil, titanium dioxide, folic acid, glycerin, glyceryl linoleate (omega-6 (fatty acid vitamin F)), vitamin A palmitate, grape seed oil, halobetasole, adenosine, adenosine triphosphate, alpha hydroxy acid, allantoin, hyaluronic acid and derivatives thereof, isoleutrol, tranexamic acid, glycolic acid, arginine, ascorbyl glucosamine, ascorbyl palmitate, salicylic acid, carnosic acid, alpha lipoic acid, gamma linolenic acid (GLA), panthenol, retinyl propionate, retinyl palmitate, furfuryl adenine, retinaldehyde, grypeptide, idebenone, dimethylaminoethanol (DMAE), niacin amide, beta-glucan, palmitoyl pentapeptide-4, palmitoyl oligopeptide/tetrapeptide-7, etoshine, ceramide, phenylalanine, glucuronolactone, L-carnitine, hydroxyapatite, palmitoyl tripeptide-3, phoscholine, zinc oxide, $\alpha$-bisabolol, eugenol, silybin, soy isoflavone, catalpol, pseudoguaianolides derived from arnica chamissonis, rosemarinic acid, rosmanol, silasilates, for example, salicine, saligenine and salicylic acid, taraxasterol, $\alpha$-lactucerol, isolactucerol, taraxacoside, ceramide, albutin, gingerol, shogaol, hypericin, elastin, collagen and peptides thereof.

In the composition of the present invention, the surface of the porous silica particle (Mesoporous Silica Particle, MSP) and/or the inside of the pore is chemically modified.

The modification may refer to substitution of -OH functional group of silanol group (Si-OH) in the silica particles with other functional groups. More particularly, the modification may serve reduce side effects such as hemolysis due to interaction between the silanol group and a quaternary ammonium group on the surface of red blood cell through intravascular injection of the composition according to the present invention. Further, depending on types of functional groups to be modified and the degree of modification, the above-described types of bioactive materials suitable for loading may be different. In addition, since zeta potential may vary and an intensity of the zeta potential may also cause a difference in a size, inter-particle precipitation or aggregation in blood stream may be prevented through charge repulsion between particles, thus to ensure flow smoothness in the blood stream. Further, interaction between the porous silica particles with respect to the environment for releasing the bioactive material is controlled so that a degradation rate of the particles may be regulated to control a release rate of the bioactive material. In addition, a binding force of the bioactive material to nanoparticles may be adjusted to control release of the bioactive material by diffusion from the particles.

Chemical or biological modification may be selected for the modification described above, but it is not limited thereto. In fact, the modification may be performed by well known methods in the art. However, in consideration of substitution of a functional group through covalent bond with silica particles, chemical modification is preferably adopted. Further, the surface of the particle and the inside of the pore may be modified in the same manner or may be differently modified.

The modification may be implemented by reacting a compound having a hydrophilic, hydrophobic, cationic or anionic substituent to be introduced with the particles, but it is not limited thereto. In fact, the modification may be implemented by reacting any compound having a substituent, which loads the bioactive material, transfers the bioactive material to a target cell, loads a material used for other purposes or binds other additional substituents, with the particles, wherein the substituent may further include an antibody, a ligand, a cell permeable peptide or an aptamer, etc.

The compound may be, for example, an alkoxysilane having a C1 to C10 alkoxy group, but it is not limited thereto. The alkoxysilane has one or more alkoxy groups, for example, 1 to 3 alkoxy groups, and may include a substituent to be introduced into a site in which the alkoxy group is not bonded or another substituent substituted by the above substituent.

When the alkoxysilane reacts with the porous silica particles, a covalent bond is formed between a silicon atom and an oxygen atom, such that the alkoxysilane may be bonded to the surface of the porous silicon particle and/or inside the pore. Further, since the alkoxysilane has a substituent to be introduced, the corresponding substituent may be introduced into the surface of the porous silicon particle and/or inside the pore.

The reaction may be performed by reacting porous silica particles dispersed in a solvent with alkoxysilane. Water and/or an organic solvent may be used as the solvent, and the organic solvent may be, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, γ-butyrolactone, 1,3-dimethyl-imidazolidinone, methylethylketone, cyclohexanone, cyclopentanone, 4-hydroxy-4-methyl-2-pentanone, etc.; carbon-based aromatics such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolves) such as ethyleneglycol monoethylether, ethyleneglycol monomethylether, ethyleneglycol monobutylether, diethyleneglycol monoethylether, diethyleneglycol monomethylether, diethyleneglycol monobutylether, propyleneglycol monomethylether, propyleneglycol monoethylether, dipropyleneglycol diethylether, triethyleneglycol monoethylether, etc.; and other dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethyl phosphoamide, tetramethylurea, N-methylcarrolactam, tetrahydrofuran, m-dioxane, P-dioxane, 1,2-dimethoxyethane and the like. Specifically, toluene may be used, but it is not limited thereto.

The reaction of the particles with the alkoxysilane may be implemented, for example, under heating, wherein the heating may be performed at 80 to 180 °C, for example, in a range of 80 to 160 °C, 80 to 150 °C, 100 to 160 °C, 100 to 150 °C, 110 to 150 °C, etc., but it is not limited thereto.

In addition, the reaction of the particles with alkoxysilane may be implemented for 4 to 20 hours, for example, in a range of 4 to 18 hours, 4 to 16 hours, 6 to 18 hours, 6 to 16 hours, 8 to 18 hours, 8 to 16 hours, 8 to 14 hours, 10 to 14 hours, etc., but it is not limited thereto.

In the above modification, the modification to the cationic substituent may be performed in order to positively charge the particles or load a negatively charged bioactive material, and may be performed by reacting the particles with, for example, alkoxysilane having a basic group, that is, a nitrogen-containing group such as an amino group, an aminoalkyl group and the like. Specifically, N-[3-(trimethoxysilyl)propyl]ethylenediamine, N1-(3-trimethoxysilylpropyl)diethylenetriamine, (3-aminopropyl)trimethoxysilane, N-[3-(trimethoxysilyl)propyl]aniline, trimethoxy[3-(methylamino)propyl]silane, 3-(2-aminoethylamino)propyldimethoxymethylsilane, etc. may be used, but it is not limited thereto.

In the above modification, the modification with an anionic substituent may be performed in order to negatively charge the particles or load a positively charged bioactive material, and may be performed by reacting the particles with, for example, alkoxysilane having an acidic group such as a carboxyl group, a sulfonic acid group, a thiol group and the like. Specifically, (3-mercaptopropyl)trimethoxysilane may be used, but it is not limited thereto.

In the above modification, the modification to the hydrophilic substituent has advantages in terms of easiness in use and formulation of the composition according to the present invention. In fact, such advantages may be achieved by reacting the particles with, for example, alkoxysilane having a carboxyl group, an amino group, a carbonyl group, a sulfhydryl group, a phosphate group, a thiol group, an ammonium group, an ester group, an imide group, a thoimide group, a keto group, an ether group, an indene group, a sulfonyl group, a polyethyleneglycol group and the like. Specifically, N-[3-(trimethoxysilyl)propyl]ethylenediamine, N1-(3-trimethoxysilylpropyl)diethylenetriamine, (3-aminopropyl)trimethoxysilane, (3-mercaptopropyl)trimethoxysilane, trimethoxy[3-(methylamino)propyl]silane, 3-(2-aminoethylamino) propyldimethoxymethylsilane may be used, but it is not limited thereto.

In the above modification, the modification to the hydrophobic substituent has an advantage that the binding force with a poorly water-soluble (hydrophobic) bioactive material is enhanced. In fact, the modification may be performed by reacting the particles with, for example, alkoxysilane having substituted or unsubstituted $C_1$ to $C_{30}$ alkyl, substituted or unsubstituted $C_3$ to $C_{30}$ cycloalkyl, substituted or unsubstituted $C_6$ to $C_{30}$ aryl, substituted or unsubstituted $C_2$ to $C_{30}$ heteroaryl, halogen, $C_1$ to $C_{30}$ ester, halogen containing group or the like. Specifically, trimethoxy(octadecyl)silane, trimethoxy-n-octylsilane, trimethoxy(propyl)silane, isobutyl(trimethoxy)silane, trimethoxy(7-octen-1-yl)silane, trimethoxy(3,3,3-trifluoropropyl)silane, trimethoxy(2-phenylethyl)silane, vinyltrimethoxysilane, cyanomethyl, 3-[(trimethoxysilyl)propyl]trithiocarbonate, (3-bromopropyl) trimethoxysilane, etc. may be used, but it is not limited thereto.

The modification may be performed in combination, for example, two or more surface modifications may be performed on an outer surface or inside the pore. As a more specific example, the positively charged particles may be changed so as to have different surface properties by binding a compound having a carboxyl group to the silica particles, into which the amino group is introduced, through an amide bond, but it is not limited thereto.

In the modification, a reaction temperature, time, and an amount of the compound used for the modification may be selected depending on an extent of modification. Further, varying reaction conditions depending on hydrophilicity, hydrophobicity and a charge level of the bioactive material may regulate hydrophilicity, hydrophobicity and charge level of the silica particles, thereby controlling the release rate of the bioactive material. For example, if the bioactive material has strong negative charge at neutral pH, the reaction temperature may be increased, the reaction time may be extended, or an amount of the compound to be treated may also be increased so that the porous silica particles have strong positive charge, but it is not limited thereto.

In the composition of the present invention, the porous silica particles (Mesoporous Silica Particle, MSP) are biodegradable particles. When the biodegradable particles load the bioactive material and then are administered in the body, these particles are biodegradable in the body while releasing the bioactive material, whereby the particles are slowly degraded in the body while enabling the loaded bioactive material to have sustained release property. According to the invention, t when a ratio of absorbance in the following Equation 1 becomes 1/2 is 20 hours or more.

$$[\text{Equation } 1]$$

$$A_t/A_0$$

(wherein $A_0$ is absorbance of the porous silica particles measured by placing 5 ml of a suspension including 1 mg/ml of the porous silica particles into a cylindrical dialysis membrane having pores with a diameter of 50 kDa,

15 ml of the same solvent as the suspension is placed outside the dialysis membrane while being in contact with the dialysis membrane, followed by horizontal agitation at 60 rpm and 37 °C inside and outside the dialysis membrane, pH of the suspension is 7.4, and

At is absorbance of the porous silica particles measured after t hours elapses from the measurement of $A_0$).

Equation 1 indicates how fast the porous silica particles are degraded under environments similar to the body, wherein the absorbance $A_0$ and $A_t$ may be measured, for example, after placing the porous silica particles and the suspension in a cylindrical dialysis membrane, and further placing the same suspension on the outside of the dialysis membrane.

The suspension is a buffer solution and, for example, at least one selected from the group consisting of phosphate buffered saline (PBS) and simulated body fluid (SBF), and more specifically, PBS.

The particles are biodegradable and may be slowly degraded in the suspension, wherein the diameter of 50 kDa corresponds to about 5 nm, the biodegraded particles can pass through a 50 kDa dialysis membrane, this cylindrical dialysis membrane is under horizontal agitation at 60 rpm, such that the suspension is evenly admixed, and the degraded particles may come out of the dialysis membrane.

The absorbance in Equation 1 may be measured, for example, under an environment in which the suspension outside the dialysis membrane is replaced with a new suspension. The suspension may be one that is constantly replaced, one that is replaced at a constant period wherein the constant period may be periodic or irregular. For example, the replacement may be performed within a range of 1 hour to 1 week, in particular, at 1-, 2-, 3-, 6-, 12-, 24-hours intervals, or 2-, 3-, 4-, 7-days interval, etc., but it is not limited thereto.

A ratio of absorbance of 1/2 means that, after t hours, the absorbance becomes half of the initial absorbance, therefore, means that approximately half of the porous silica particles have been degraded.

t when the ratio of absorbance in Equation 1 becomes 1/2 is 20 or more or 24 or more, for example, t may be 20 to 120, specifically, 20 to 96, 20 to 72, 30 to 70, 40 to 70, 50 to 65, etc. within the above range, but it is not limited thereto.

The particles are characterized in that t when the ratio of absorbance in Equation 1 becomes 1/5 may be, for example, 70 to 140, specifically, 80 to 140, 80 to 120, 80 to 110, 70 to 140, 70 to 120, 70 to 110, etc. within the above range, but it is not limited thereto.

The particles are characterized in that t when the ratio of absorbance in Equation 1 becomes 1/20 may be, for example, 130 to 220, specifically, 130 to 200, 140 to 200, 140 to 180, 150 to 180, etc. within the above range, but it is not limited thereto.

The particles are characterized in that t when the measured absorbance becomes 0.01 or less may be, for example, 250 or more, specifically, 300 or more, 350 or more, 400 or more, 500 or more, 1000 or more, etc. within the above range while having an upper limit of 2000, but it is not limited thereto.

The particles are characterized in that the absorbance ratio in Equation 1 has high positive correlation with t, specifically, Pearson correlation coefficient may be 0.8 or more, for example, 0.9 or more, 0.95 or more, etc.

t in Equation 1 means how fast the porous silica particles are degraded under environments similar to the body, for example, may be controlled by adjusting the surface area, particle diameter, pore diameter, substituents on the surface of the porous silica particle and/or inside the pore, compactness of the surface, etc.

More particularly, t may be reduced by increasing the surface area of the particle or may be increased by reducing the surface area thereof. The surface area may be regulated by adjusting the diameter of the particles and/or the diameter of the pores. In addition, placing a substituent on the surface of the particle and/or the inside of the pore may reduce direct exposure of the porous silica particles to the environment (such as a solvent), thereby increasing t. Further, loading the bioactive material on the porous silica particles and increasing affinity between the bioactive material and the porous silica particles may reduce direct exposure of the porous silica particles to the environment, thereby increasing t. In addition, the surface may be made more densely in the preparation of the particles so as to increase t. In the above, various examples of adjusting t in Equation 1 have been described, but it is not limited thereto.

In the composition of the present invention, the porous silica particles (Mesoporous Silica Particle, MSP) are particles of silica ($SiO_2$) material, and have a diameter of several nanometers to several micrometers.

The average diameter of the particles may be, for example, 100 to 1000 nm, specifically, 100 to 800 nm, 100 to 500 nm, 100 to 400 nm, 100 to 300 nm, 100 to 200 nm, etc. within the above range, but it is not limited thereto.

In the composition of the present invention, the porous silica particles (Mesoporous Silica Particle, MSP) are porous particles including nano-sized pores wherein the above-mentioned bioactive material may be loaded in the pores or on the surfaces of particles.

The average pore diameter of the particles may be, for example, 1 to 100 nm, specifically, 5 to 100 nm, 7 to 100 nm, 7 to 50 nm, 10 to 50 nm, 10 to 30 nm, 7 to 30 nm, etc. within the above range, but it is not limited thereto. Further, in consideration of an amount and a size of the bioactive material to be loaded, the average pore diameter is preferably selected and adjusted.

In the composition of the present invention, a shape of the porous silica particle (Mesoporous Silica Particle, MSP) is not particularly limited to a specific form. However, in consideration of smoothness of flow in the blood stream, smoothness of interaction with blood cells in the blood stream and the antihemolytic aspect of erythrocytes, a spherical shape is preferably adopted.

In the composition of the present invention, the porous silica particles (Mesoporous Silica Particle, MSP) may have a BET surface area of, for example, 200 to 700 $m^2$/g, specifically, 200 to 700 $m^2$/g, 200 to 650 $m^2$/g, 250 to 650 $m^2$/g, 300 to 700 $m^2$/g, 300 to 650 $m^2$/g, 300 to 600 $m^2$/g, 300 to 550 $m^2$/g, 300 to 500 $m^2$/g, 300 to 450 $m^2$/g, etc. within the above range, but it is not limited thereto.

In the composition of the present invention, the porous silica particles (Mesoporous Silica Particle, MSP) may have a volume per gram (g) of, for example, 0.7 to 2.2 ml, specifically, 0.7 to 2.0 ml, 0.8 to 2.2 ml, 0.8 to 2.0 ml, 0.9 to 2.0 ml, 1.0 to 2.0 ml, etc. within the above range, but it is not limited thereto. If the volume per gram (g) is too small, the degradation rate may be too high. Further, it may be difficult to manufacture excessively large particles or the particles may not have a complete shape.

In the composition of the present invention, the porous silica particles (Mesoporous Silica Particle, MSP) have surface charge, that is, have zeta potential other than 0 mV. As described above, an electronic repulsive force between the particles modified in the same manner may inhibit a phenomenon in which the particles are aggregated or precipitated in the blood, thereby facilitating the flow in the blood and delivering the effectively loaded bioactive material to a target tissue or cells.

A value of the surface charge of the particles, that is, a value of the zeta potential may be, for example, +3 to +100 mV when positively charged, but it is not limited thereto. Further, when negatively charged, the value of the zeta potential may be, for example, -100 to -18 mV, but it is not limited thereto. The value of the zeta potential may be adjusted to meet purposes thereof in consideration of different aspects such as a type and amount of the bioactive material to be loaded, or control of the release rage. However, when the value of the zeta potential is greater than -18 mV or less than +3 mV, the repulsive force between the porous silica particles is lowered to aggregate the particles and it may be difficult to load the charged bioactive material. Further, if the value of the zeta potential is greater than +100 mV or less than -100 mV, the binding force with the charged bioactive material is excessively high so that effective release may be difficult.

In the composition of the present invention, the porous silica particles (Mesoporous Silica Particle, MSP) may load the above-described bioactive material on the surface of the particle and/or the inside of the pore.

Loading the particles with the bioactive material may be performed, for example, by mixing porous silica particles and the bioactive material in a solvent. In this regard, water and/or an organic solvent may be used as the solvent. The organic solvent used herein may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; carbon-based aromatics such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.

Further, a phosphate buffered saline solution (PBS), simulated body fluid (SBF), borate-buffered saline, tris-buffered saline may be used as the solvent.

A ratio of the porous silica particles and the bioactive material is not particularly limited and, for example, the weight ratio may be 1:0.05 to 0.8, specifically, 1:0.05 to 0.7, 1:0.05 to 0.6, 1:0.1 to 0.8, 1:0.1 to 0.6, 1:0.2 to 0.8, 1:0.2 to 0.6, etc. within the above range.

In the composition of the present invention, the porous silica particles (Mesoporous Silica Particle, MSP) may gradually release the loaded bioactive material over a long period of time.

The bioactive material loaded on the particles may be released as the particles are biodegraded, and the particles may be slowly degraded to allow sustained release of the loaded bioactive materials. This may be controlled by, for example, adjusting the surface area, particle diameter, pore diameter, substituents on the surface of the particle and/or the inside of the pore, compactness of the porous silica particles, and the like, but it is not limited thereto.

In addition, the bioactive material loaded on the particles may be released while being separated from the porous silica particles and diffused, which is affected by the relationship between the porous silica particles, the bioactive material and the bioactive material releasing environment. Therefore, adjusting these conditions may control the release of bioactive material. For example, the release of bioactive material may be controlled by strengthening or weakening the binding force of the porous silica particles with the bioactive material by surface modification.

More particularly, if the loaded bioactive material is poorly water-soluble (hydrophobic), the surface of the particle and/or the inside of the pore may have a hydrophobic substituent to increase the binding force between the particles and the bioactive material, whereby the bioactive material may be released in a sustained manner. This may be achieved by, for example, surface modification of the particles with alkoxysilane having a hydrophobic substituent.

As used herein, "poorly soluble" means being insoluble (practically insoluble) or only slightly soluble (with respect to water), which is a terminology defined in "pharmaceutical Science" 18th Edition (U.S.P., Remington, Mack Publishing Company).

The poorly water-soluble bioactive material may have, for example, water solubility of less than 10 g/L, specifically less than 5 g/L, more specifically less than 1 g/L at 1 atmosphere and 25 °C, but it is not limited thereto.

When the loaded bioactive material is water-soluble (hydrophilic), the surface of the particle and/or the inside of the pore may have a hydrophilic substituent to increase the binding force between the porous silica particles and the bioactive material, whereby the bioactive materials may be released in a sustained manner. This may be achieved by, for example, surface modification of the porous silica particles with alkoxysilane having a hydrophilic substituent.

The water-soluble bioactive material may have, for example, water solubility of 10 g/L or more at 1 atmosphere and 25 °C, but it is not limited thereto.

When the loaded bioactive material is charged, the surface of the particle and/or the inside of the pore may be charged with the opposite charge thus to increase the binding force between the porous silica particles and the bioactive material, whereby the bioactive material may be released in a sustained manner. This may be achieved by, for example, surface modification of the porous silica particles with alkoxysilane having an acidic group or a basic group.

Specifically, if the bioactive material is positively charged at neutral pH, the surface of the particle and/or the inside of the pore may be negatively charged at neutral pH thus to increase the binding force between the porous silica particles and the bioactive material, whereby the bioactive material may be released in a sustained manner. This may be achieved by, for example, surface modification of the porous silica particles with alkoxysilane having an acidic group such as a carboxyl group (-COOH), sulfonic acid group ($-SO_3H$), etc.

Further, if the bioactive material is negatively charged at neutral pH, the surface of the particle and/or the inside of the pore may be positively charged thus to increase the binding force between the porous silica particles and the bioactive material, whereby the bioactive material may be released in a sustained manner. This may be achieved by, for example, surface modification of the porous silica particles with alkoxysilane having a basic group such as an amino group, nitrogen-containing group, etc.

The loaded bioactive material may be released for a period of, for example, 7 days to 1 year or more depending on the type of treatment required, release environment, and porous silica particles to be used, etc.

In the composition of the present invention, since the porous silica particles (Mesoporous Silica Particle, MSP) are biodegradable and may be degraded by 100%, the bioactive material loaded thereon can be released by 100%.

Because of 100% biodegradability of the particles, an amount of the loaded bioactive material may be appropriately set according to corresponding purposes and used in drug delivery in the blood vessels, thereby having significant advantages of avoiding problems such as side effects due to overuse of the bioactive material, preventing severe situations such as clogging the blood vessels without complete degradation of the particles, and overcoming impossibility of embolization described below to the same path, which a significant problem of the conventional embolization.

In the composition of the present invention, the porous silica particles (Mesoporous Silica Particle, MSP) are prepared by a small pore particle preparation and pore expansion and calcination, and surface modification processes, etc. If the particles are subjected to both the calcination and the surface modification processes, the particles may be surface-modified after the calcinations.

The small pore particles may be, for example, particles having an average pore diameter of 1 to 5 nm, which can be obtained by adding a surfactant and a silica precursor to a solvent and then agitating and homogenizing the solution.

Water and/or organic solvents may be used as the solvent, and the organic solvent used herein may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, γ-butyrolactone, 1,3-dimethyl-imidazolidinone, methylethylketone, cyclohexanone, cyclopentanone, 4-hydroxy-4-methyl-2-pentanone, etc.; carbon-based aromatics such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolves) such as ethyleneglycol monoethylether, ethyleneglycol monomethylether, ethyleneglycol monobutylether, diethyleneglycol monoethylether, diethyleneglycol monomethylether, diethyleneglycol monobutylether, propyleneglycol monomethylether, propyleneglycol monoethylether, dipropyleneglycol diethylether, triethyleneglycol monoethylether, etc.; and other dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethyl phosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

When using a mixed solvent of water and an organic solvent as the solvent, a ratio of water and an organic solvent may be used in a volume ratio of, for example, 1:0.7 to 1.5, e.g., 1:0.8 to 1.3, but it is not limited thereto.

The surfactant may be, for example, cetyltrimethylammonium bromide (CTAB), hexadecyltrimethylammonium bromide (TMABr), hexadecyltrimethylpyridinium chloride (TMPrCl), tetramethylammonium chloride (TMACl), and the like, and specifically, CTAB may be used.

For example, the surfactant may be added in an amount of 1 to 10 g, specifically, 1 to 8 g, 2 to 8 g, 3 to 8 g, etc. per liter of solvent within the above range, but it is not limited thereto.

The silica precursor may be added after the agitation with addition of the surfactant to the solvent. The silica precursor may be, for example, tetramethyl orthosilicate (TMOS), but it is not limited thereto.

The agitation may be performed, for example, for 10 minutes to 30 minutes, but it is not limited thereto.

The silica precursor may be added thereto, for example, in an amount of 0.5 to 5 ml per liter of solvent, specifically, 0.5 to 4 ml, 0.5 to 3 ml, 0.5 to 2 ml, 1 to 2 ml, etc. within the above range, but it is not limited thereto. Rather, if necessary, sodium hydroxide as a catalyst may further be used, wherein the catalyst may be added while agitating after adding the surfactant to the solvent and before adding the silica precursor to the solvent.

The catalyst, that is, sodium hydroxide may be used in an amount of, for example, 0.5 to 8 ml per liter of solvent, specifically, 0.5 to 5 ml, 0.5 to 4 ml, 1 to 4 ml, 1 to 3 ml, 2 to 3 ml, etc. within the above range, based on 1 M aqueous sodium hydroxide solution, but it is not limited thereto.

After the addition of the silica precursor, the solution may be reacted with agitation. The agitation may be performed, for example, for 2 to 15 hours, specifically, 3 to 15 hours, 4 to 15 hours, 4 to 13 hours, 5 to 12 hours, 6 to 12 hours, 6 to 10 hours, etc. within the above range, but it is not limited thereto. If an agitation time (reaction time) is too short, nucleation may be insufficient.

After the agitation, the solution may be aged. Aging may be performed, for example, for 8 to 24 hours, specifically, 8 to 20 hours, 8 to 18 hours, 8 to 16 hours, 8 to 14 hours, 10 to 16 hours, 10 to 14 hours, etc. within the above range, but it is not limited thereto.

Thereafter, a reaction product may be washed and dried to obtain porous silica particles and, if necessary, an unreacted material may be isolated before washing, which may be performed, for example, by separating the supernatant through centrifugation.

The centrifugation may be performed, for example, at 6,000 to 10,000 rpm, for example, for 3 to 60 minutes, specifically, 3 to 30 minutes, 5 to 30 minutes, etc. within the above range, but it is not limited thereto.

The washing may be carried out with water and/or an organic solvent. In particular, since different substances are soluble in different solvents respectively, water and the organic solvent may be used once or several times by turns. Alternatively, water and/or the organic solvent may be used alone for washing once or several times. Such several times may include, for example, two or more, ten or less, specifically, three or more and ten or less, four or more and eight or less, four or more and six or less, etc.

The organic solvent used herein may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, $\gamma$-butyrolactone, 1,3-dimethyl-imidazolidinone, methylethylketone, cyclohexanone, cyclopentanone, 4-hydroxy-4-methyl-2-pentanone, etc.; carbon-based aromatics such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolves) such as ethyleneglycol monoethylether, ethyleneglycol monomethylether, ethyleneglycol monobutylether, diethyleneglycol monoethylether, diethyleneglycol monomethylether, diethyleneglycol monobutylether, propyleneglycol monomethylether, propyleneglycol monoethylether, dipropyleneglycol diethylether, triethyleneglycol monoethylether, etc.; and other dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethyl phosphoamide, tetramethylurea, N-methylcarrolactam, tetrahydrofuran, m-dioxane, P-dioxane, 1,2-dimethoxyethane, etc., and, specifically, alcohol and, more specifically, ethanol may be used, but it is not limited thereto.

The washing may be performed under centrifugation, for example, at 6,000 to 10,000 rpm, for example, for 3 to 60 minutes, specifically, 3 to 30 minutes, 5 to 30 minutes, etc. within the above range, but it is not limited thereto.

The washing may be performed by filtering particles with a filter without centrifugation. The filter may include pores with a diameter of less than or equal to the diameter of the porous silica particles. If the reaction solution is filtered through such a filter, only particles remain on the filter and may be washed by pouring water and/or an organic solvent over the filter.

For washing, water and the organic solvent may be used once or several times by turns. Alternatively, the washing may be performed once or several times even with water or the organic solvent alone. The several times may include, for example, two or more and ten or less, specifically, three or more and ten or less, four or more and eight or less, four or more and six or less and the like.

The drying may be performed, for example, at 20 to 100 °C, but it is not limited thereto. Alternatively, the drying may be performed in a vacuum state.

Thereafter, the pores of the obtained porous silica particles are expanded using, for example, a pore swelling agent.

The pore swelling agent used herein may include, for example, trimethylbenzene, triethylbenzene, tripropylbenzene, tributylbenzene, tripentylbenzene, trihexylbenzene, toluene, benzene, etc. and, specifically, trimethylbenzene may be used, but it is not limited thereto.

Alternatively, the pore swelling agent used herein may be, for example, N,N-dimethylhexadecylamine (DMHA), but it is not limited thereto.

Pore expansion described above may be performed, for example, by mixing porous silica particles in a solvent with a pore swelling agent, and heating and reacting the mixture. The solvent used herein may be, for example, water and/or an organic solvent. The organic solvent used herein may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; carbon-based aromatics such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; and, specifically, alcohol and, more specifically, ethanol may be used, but it is not limited thereto.

The porous silica particles may be added in a ratio of, for example, 10 to 200 g per liter of solvent, specifically, 10 to 150 g, 10 to 100 g, 30 to 100 g, 40 to 100 g, 50 to 100 g, 50 to 80 g, 60 to 80 g, etc. within the above range, but it is not limited thereto.

The porous silica particles may be evenly dispersed in a solvent, for example, the porous silica particles may be added to the solvent and ultrasonically dispersed therein. In the case of using a mixed solvent, the second solvent may be added after the porous silica particles are dispersed in the first solvent.

The pore swelling agent may be added in an amount of, for example, 10 to 200 parts by volume (vol. parts), specifically, 100 to 150 vol. parts, 10 to 100 vol. parts, 10 to 80 vol. parts, 30 to 80 vol. parts, 30 to 70 vol. parts based on 100 vol. parts of solvent within the above range, but it is not limited thereto.

The reaction may be performed, for example, at 120 to 180 °C, specifically, 120 to 170 °C, 120 to 160 °C, 120 to 150 °C, 130 to 180 °C, 130 to 170 °C, 130 to 160 °C, 130 to 150 °C , etc. within the above range, but it is not limited thereto.

The reaction may be performed, for example, for 24 to 96 hours, specifically, 30 to 96 hours, 30 to 80 hours, 30 to 72 hours, 24 to 80 hours, 24 to 72 hours, 36 to 96 hours, 36 to 80 hours, 36 to 72 hours, 36 to 66 hours, 36 to 60 hours, 48 to 96 hours, 48 to 88 hours, 48 to 80 hours, 48 to 72 hours, etc. within the above range, but it is not limited thereto.

By adjusting the time and the temperature within the above ranges, respectively, the reaction may be performed sufficiently without being too much. For example, when the reaction temperature is lower, the reaction time may be increased, otherwise, when the reaction temperature is lower, the reaction time may be shortened. If the reaction is not sufficient, pore expansion may not be sufficient. On the other hand, if the reaction proceeds excessively, the particles may collapse due to the expansion of the pores.

The reaction may be performed, for example, while gradually increasing the temperature. Specifically, the reaction may be performed while gradually increasing the temperature at a rate of 0.5 to 15 °C/min from the room temperature, specifically, 1 to 15 °C/min, 3 to 15 °C/min, 3 to 12 °C/min, 3 to 10 °C/min, etc. within the above range, but it is not limited thereto.

After the reaction, the reaction solution may be cooled slowly, for example, cooled by lowering the temperature step by step. Specifically, the reaction solution may be cooled by gradually decreasing the temperature at a rate of 0.5 to 20 °C/min to room temperature, specifically,, 1 to 20 °C/min, 3 to 20 °C/min, 3 to 12 °C/min, 3 to 10 °C/min, etc. within the above range, but it is not limited thereto.

After cooling, the reaction product may be washed and dried to obtain porous silica particles having expanded pores

If necessary, unreacted material may be isolated prior to washing, for example, by centrifugation to separate a supernatant.

The centrifugation may be performed, for example, at 6,000 to 10,000 rpm for 3 to 60 minutes, specifically, 3 to 30 minutes, 5 to 30 minutes, etc. within the above range, but it is not limited thereto.

The washing may be carried out with water and/or an organic solvent. In particular, since different substances are soluble in different solvents respectively, water and the organic solvent may be used once or several times by turns. Alternatively, water and/or the organic solvent may be used alone for washing once or several times. Such several times may include, for example, two or more, ten or less, specifically, three times, 4 times, 5 times, 6 times, 7 times, 8 times, etc.

The organic solvent used herein may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; carbon-based aromatics such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc., and, specifically, alcohol and, more specifically, ethanol may be used, but it is not limited thereto.

The washing may be carried out under centrifugation, for example at 6,000 to 10,000 rpm, for example, for 3 to 60 minutes, specifically, 3 to 30 minutes, 5 to 30 minutes, etc. within the above range, but it is not limited thereto.

The washing may be performed by filtering particles with a filter without centrifugation. The filter may have pores with a diameter of less than or equal to the diameter of the porous silica particles. If the reaction solution is filtered through such a filter, only particles remain on the filter and may be washed by pouring water and/or an organic solvent over the filter.

For washing, water and the organic solvent may be used once or several times by turns. Alternatively, the washing may be performed once or several times even with water or the organic solvent alone. The several times may include, for example, two or more and ten or less, specifically, three or more and ten or less, four or more and eight or less, four or more and six or less and the like.

The drying may be performed, for example, at 20 to 100 °C, but it is not limited thereto. Alternatively, the drying may be performed in a vacuum state.

Thereafter, the pores of the obtained porous silica particles are subjected to calcinations, which is a process of heating the particles to have a more dense structure on the surface thereof and the inside of the pore, and removing organic materials filling the pores. The calcinations are performed at 400 to 700 °C for 3 to 8 hours, specifically, at 500 to 600 °C for 4 to 5 hours.

Then, the obtained porous silica particles may be modified on the surface thereof and/or the inside of the pore as described above.

In the composition of the present invention, the porous silica particles (Mesoporous Silica Particle, MSP) may also be obtained by, for example, preparation of small pore particles, pore expansion, surface modification and/or modification of inside of the pore.

Small pore particle preparation and pore expansion may be performed according to the above-described processes, then the washing and drying processes may be performed.

If necessary, the unreacted material may be isolated prior to washing, for example, by centrifugation to separate the supernatant.

The centrifugation may be performed, for example, at 6,000 to 10,000 rpm, for example, for 3 to 60 minutes, specifically, 3 to 30 minutes, 5 to 30 minutes, etc. within the above range, but it is not limited thereto.

The washing after the preparation of the small pore particles may be performed by any method under conditions within the above-illustrated range, but it is not limited thereto.

The washing after the pore expansion may be performed under more relaxed conditions than the above illustrative embodiments. For example, washing may be carried out three times or less, but it is not limited thereto.

The surface of the particle and/or the inside of the pore may be modified by the above-described method, wherein the modification may be performed in an order of the surface of the particle and then the inside of the pore, and particle washing may be further performed between the above two processes.

When the washing is carried out in more relaxed conditions after the preparation of small pore particles and pore expansion, the pores are filled with a reaction solution such as a surfactant used in the particle preparation and the pore expansion, such that the inside of the pore is not modified during surface modification, instead, only the surface of the particle may be modified. After then, washing the particles may remove the reaction solution in the pores.

Particle washing between surface modification and modification of the inside of the pore may be performed with water and/or an organic solvent. In particular, since different substances are soluble in different solvents respectively, water and the organic solvent may be used once or several times by turns. Alternatively, water and/or the organic solvent may be used alone for washing once or several times. Such several times may include, for example, two or more, ten or less, specifically, three or more and ten or less, four or more and eight or less, four or more and six or less, etc.

The washing may be performed under centrifugation, for example, at 6,000 to 10,000 rpm, for example, for 3 to 60 minutes, specifically, 3 to 30 minutes, 5 to 30 minutes, etc. within the above range, but it is not limited thereto.

The washing may be performed by filtering particles with a filter without centrifugation. The filter may include pores with a diameter of less than or equal to the diameter of the porous silica particles. If the reaction solution is filtered through such a filter, only particles remain on the filter and may be washed by pouring water and/or an organic solvent over the filter.

For washing, water and the organic solvent may be used once or several times by turns. Alternatively, the washing may be performed once or several times even with water or the organic solvent alone. The several times may include, for example, two or more and ten or less, specifically, three or more and ten or less, four or more and eight or less, four or more and six or less and the like.

The drying may be performed, for example, at 20 to 100 °C, but it is not limited thereto. Alternatively, the drying may be performed in a vacuum state.

A composition for delivering a bioactive material in a blood vessel according to the present invention may further include any substance well known in the art, for the purpose of achieving efficiency for delivery of the bioactive material loaded on the porous silica particles or for the purpose of using the above composition. Such a substance well known in the art and further added to the composition may include fluorescent labeling materials, blood coagulation inhibitors, erythrocyte hemolytic agents, contrast agents and the like, but it is not limited thereto.

The blood coagulation inhibitors may be at least one selected from the group consisting of 1,2-distaroyl-sn-glycero-3-(phospho-lac-(1-glycerol), 1,2-dstearoyl-sn-glycero-3-phosphocholine, cetomacrozol 1000, cetostearyl alcohol, cetyl alcohol, cetylpyridinium chloride, cholesterol, dipalmitoyl phosphatidylglycerol, distearoyl phosphatidylcholine, alkyl polyglycoside, EGG phospholipids, fatty acid esters, glyceryl laurate, glyceryl oleate, hydroxyethylpiperazine ethane sulfonic acid, lactose monohydrate, lanolin, lauryl lactate,

Lecithin, magnesium stearate, monothioglycerol, oleic acid, oleyl alcohol, palmitic acid, PEG/PPG-18/18 dimethicone, polyethylene glycol (PEG), PEG-20 sorbitan isostearate, PEG-40 castor oil, PEG-60 hydrogenated castor oil, pentasom pentetate, phospholipid, poloxamer, poloxamer 188, poloxamer 407, polyoxyethylene fatty acid esters, polyoxyl 30 castor oil, polyoxyl 31 castor oil, polyoxyl 32 castor oil, polyoxyl 33 castor oil, polyoxyl 34 castor oil, polyoxyl 35 castor oil, polyoxyl 36 castor oil, polyoxyl 36 castor oil, polyoxyl 37 castor oil, polyoxyl 38 castor oil, polyoxyl 39 castor oil, polyoxyl 40 castor oil, polypropyleneglycol, polysorbate, polysorbate 20, polysorbate 40, polysorbate 80, povidone K12, povidon K17, povidone K30, povidone, propyleneglycol, polypyleneglycol monolaurate, protamine sulfate, sodium cholesteryl sulfate, sodium oleate, sorbitan, sorbitan monostearate, sorbitan tristearate, sorbitan monolaurate, sorbitan monooleate, sorbitan monopalmitate, stearyl alcohol, stearic acid, sulfactin, zinc stearate, cocamide DEA, cocamide MEA, decyl glucoside, decyl polyglucose, glycerol monostearate, IGEPAL CA-630, isoceteth-20, lauryl glucosidem maltoside, monolaurin, mycosubtilin, ethoxylate, nodidet P-40, nonoxynol, octaethyleneglycol monododecylether, N-octylbeta-D-thioglucopyranoside, octyl glucoside, oleyl alcohol, PEG-10 sunflower glycerides, pentaethyleneglycol monododecylether, polyethoxylated tallow amine, polyglycerol polyricinoleate, TRITON X-100, dextran, polyvinylpyrrolidone, 1,2-dioleoyl-SN-glycero-3-phosphocholine, exosome, micelles, liposome, polyvinyl alcohol, silicone, copolymers, nucleic acid, peptide and cell membrane, but it is not limited thereto.

The contrast agent may be at least one selected from the group consisting of metrizamide, iopamidol, iodixanol, iohexol, iopromide, iobitridol, iomeprol, iopentol, iopamiron, ioxylan, iotrolan, gadodiamide, gadoteridol, iotrol, ioversol, lipiodol, iodides oil, oil contrast agents, oil phase contrast agents, barium contrast agents or combinations thereof, but it is not limited thereto.

The composition for delivering a bioactive material in blood vessels according to the present invention specifically relates to "intravascular administration" of the composition according to the present invention. The term "in a blood vessel" will be understood to mean a delivery into a patient's vasculature, which refers to "into blood vessel(s)" or "in blood vessel(s)". In certain embodiments, the administration is (intravenous) administration into a vascular vessel that is considered to be a vein, while the administration in another embodiment may be administration into a vascular vessel that is considered to be an artery. Veins may include internal jugular veins, peripheral veins, coronary veins, hepatic veins, portal veins, great saphenous veins, pulmonary veins, superior vena cava, inferior vena cava, gastric veins, spleen veins, inferior mesenteric veins, superior mesenteric veins, head veins and/or femoral veins, but it is not limited thereto. Arteries may include coronary arteries, pulmonary arteries, brachial artery, internal carotid artery, aortic arch, femoral artery, peripheral artery and/or ciliary artery, but it is not limited thereto. It is contemplated that it may be delivered through a small artery or capillaries, or to a small artery or capillaries.

Intravascular administration of the composition for delivering a bioactive material in blood vessels according to the present invention may be performed by inserting a catheter into a blood vessel near the target tissue or cell in order to effectively achieve delivery of the bioactive material loaded on the porous silica particles, which is the purpose of the composition. In this case, the bioactive material loaded on the surfaces of the porous silica particles may be less washed away by the flow of blood stream, or release of the bioactive material loaded on the surface of the porous silica particle or inside of the pore by the diffusion in the blood stream may be reduced. Further, there is an advantage of improving targetability in delivery of the loaded bioactive material.

The present invention provides a pharmaceutical composition for treatment of specific diseases, which includes the composition for delivering a bioactive material in blood vessels.

As used herein, the term "treatment" means an approach to obtain beneficial or desirable clinical results. For the purposes of the present invention, the beneficial or desirable clinical results may include, without limitation, alleviation of symptoms, reduction in an extent of disease, stabilization (i.e., not worsening) of disease state, delay or slowing of disease progression, improvement, temporary mitigation and alleviation of disease state (partially or wholly), whether or not it is detectable. Further, the term "treatment" may also refer to increasing survival compared to that expected survival when untreated. The treatment refers to both therapeutic treatment and prophylactic or preventive measures. Such treatments may include treatments required for disorders that have already occurred as well as disorders to be prevented.

As used herein, the term "prevention" means any action to inhibit or delay development of a related disease. It will be apparent to those skilled in the art that the composition mentioned herein may prevent initial symptoms, or related diseases in a case of administering before symptoms appear.

Such specific diseases may include at least one selected from the group consisting of: hepatocellular carcinoma, metastatic liver cancer, colon cancer, metastatic colon cancer, lung cancer, metastatic lung cancer, gastric cancer, pancreatic cancer, metastatic pancreatic cancer, skin cancer, melanoma, metastatic melanoma, osteosarcoma, fibrosarcoma, lipoma, gallbladder cancer, intrahepatic bile duct cancer, bladder cancer, uterine cancer, cervical cancer, ovarian cancer, breast cancer, head and neck cancer, thyroid cancer and kidney cancer, brain cancer, glioblastoma, mediastinal tumor, mesenteric lymph node metastasis, hematologic cancer, blood cancer, leukemia, non-Hodgkin's lymphoma, Hodgkin's lymphoma, multiple myeloma, lymphoma, malignant lymphoma, myelodysplastic syndrome, acute lymphoblastic leukemia, acute myeloid leukemia, chronic lymphocytic leukemia, chronic myelogenous leukemia, isolated myeloma, aplastic anemia, spinal muscular atrophy, hereditary disease, hereditary skeletal disease, hereditary malformation syndrome, autosomal recessive genetic disease, rare disease, infectious disease, ischemic disease, nasal polyps, sinusitis, hypertrophic scar, keloid, immune disease, autoimmune disease, infectious immune disease, viral infection, bacterial infection, rheumatoid arthritis, diabetes, diabetic complication, foot ulcer, neuropathy, metabolic syndrome, intestinal disease, atopy, allergy, lupus, dementia, Parkinson's disease, wound disease, laceration disease, skin diseases, bedsores, vascular diseases, arterial diseases, venous diseases, lymphatic diseases, cardiovascular diseases, ischemic heart diseases, cerebrovascular diseases, hypertension, dyslipidemia, arteriosclerosis, peripheral vascular diseases, and lower limb artery occlusion, but it is not limited thereto.

A pharmaceutical composition for preventing or treating the above diseases, which includes the porous silica particles loaded with the bioactive material according to the present invention, may further include a pharmaceutically acceptable carrier and may be formulated with the carrier. As used herein, the term "pharmaceutically acceptable carrier" refers to a carrier or diluent that does not irritate an organism and does not inhibit biological activities and properties of the administered compound. The pharmaceutically acceptable carrier in a composition formulated in a liquid solution is sterile and physiologically compatible, and may include saline, sterile water, Ringer's solution, buffered saline, albumin injectable solution, dextrose solution, maltodextrin solution, glycerol, ethanol, and a combination of one or more of these components. Further, if necessary, other conventional additives such as antioxidants, buffers and bacteriostatic agents may also be added. In addition, diluents, dispersants, surfactants, binders and lubricants may also be added so as to formulate the composition into injectable formulations such as an aqueous solution, suspension, emulsion, etc., pills, capsules, granules or tablets and the like.

The composition of the present invention is applicable in any type of formulation that contains porous silica particles loaded with the bioactive material according to the present invention as an active ingredient, and may be prepared in oral or parenteral formulations. Such pharmaceutical formulations of the invention may include any one suitable for oral, rectal, nasal, topical (including the cheek and sublingual), subcutaneous, vaginal or parenteral (intramuscular, subcutaneous) administration, or otherwise, may be suitable for administration through inhalation or insufflation.

The composition of the present invention may be administered in a pharmaceutically effective amount. An effective dose level may be determined in consideration of the type of disease, severity, activity of the drug, sensitivity to the drug, administration time, administration route and rate of release, duration of treatment, factors including concurrent drug use, and other factors well known in the medical field. The composition of the present invention may be administered as a separate therapeutic agent or in combination with other therapeutic agents, may be administered sequentially or simultaneously with conventional therapeutic agents, and may be administered in single or multiple doses. Taking all of the above factors into consideration, it is important to administer a minimum amount that can obtain maximum effects without side effects, which can be easily determined by those skilled in the art.

Dosage of the composition of the present invention may vary greatly depending on a weight, age, gender and/or health condition of a patient, diet, administration time, method of administration, excretion rate and severity of the disease. Specifically, an appropriate dosage may depend on the amount of drug accumulated in the body and/or specific efficacy of the porous silica particles loaded with the bioactive material to be used. In general, the dosage may be estimated based on EC50 determined to be effective in *in vivo* animal models as well as *in vitro.* For example, the dosage may range from 0.01 μg to 1 g per kg of body weight, and the composition may be administered once or several times per unit period, in daily, weekly, monthly or yearly unit periods. Otherwise, the composition may be continuously administered for a long period of time via an infusion pump. The number of repeated doses is determined in consideration of a retention time of drug remaining in the body, a concentration of drug in the body and the like. Even after the treatment in the course of the disease treatment, the composition may be administered for preventing relapse.

The composition of the present invention may further include at least one active ingredient having the same or similar function in relation to treatment of the above disease or a compound which maintains/increases solubility and/or absorbency of the active ingredient. Further, chemotherapeutic agents, anti-inflammatory agents, antiviral agents and/or immune-modulators, etc. may be optionally included.

In addition, the composition of the present invention may be formulated by any conventional method known in the art to provide rapid, sustained or delayed release of the active ingredient after the administration thereof to a mammal. The formulation may be in a form of powders, granules, tablets, emulsions, syrups, aerosols, soft or hard gelatin capsules, sterile injectable solutions, sterile powders.

The present invention provides a composition for an embolic procedure, which includes the composition for delivering a bioactive material in blood vessels described above.

Physical properties of the porous silica particles used in the embolic composition are not significantly different from those of the aforementioned particles, but may be used by adjusting the particle diameter to an appropriate size according to the purpose of embolization.

More specifically, nanometer-sized particles are used to enter microvascular vessels within tumor tissues and to be accumulated in blood vessels directed to tumor tissues and blood vessels within the tumor tissues, thereby blocking the tumor tissues and preventing oxygen and nutrient supply to the same. Further, using micrometer-sized particles may block arteries connected to tumor tissues thus to embolize a wider range of tumor tissues.

When using the nanometer-sized particles, an average diameter of the particles may be, for example, 100 to 1000 nm, specifically, 100 to 800 nm, 100 to 500 nm, 100 to 400 nm, 100 to 300 nm, 100 to 200 nm, etc. within the above range, but it is not limited thereto.

When using the micrometer-sized particles, an average diameter of the particles may be, for example, 0.1 to 500 $\mu$m, 0.1 to 300 $\mu$m, 100 to 300 $\mu$m, 300 to 500 $\mu$m, 0.1 or more to 100 $\mu$m, 0.1 to 1 $\mu$m, 0.2 to 0.8 $\mu$m, etc., but it is not limited thereto.

The porous silica particles, as described above, are biodegradable particles, and may be degraded by body fluids or microorganisms in a living body, thereby releasing an anticancer drug in a sustained release manner over several hours to several hundred hours after the injection. The particles do not permanently block the blood vessels and may be re-administered in the same route (blood vessel) during the second procedure if the tumor is not completely necrotic/killed after the chemo-embolization.

Although the composition may further include at least one embolic material selected from the group consisting of, polyvinyl alcohol, contrast agents, iodide oil, oil contrast agents, oil phase contrast agents, barium contrast agents, lipiodol, N-butylcyanoacrylate, coil, gel foam, gelatin, ethanol, dextran, silica, fumed silica, polymers, copolymers, polysodium acrylate vinylalcohol copolymers, radioactive materials, glass, poly-L-guluronic alginate, polyglycolic-polyactic acid, polydioxanone, polyglycolic acid-co-caprolactone, polypropylene and porous silica particles having a diameter of 100 $\mu$m or more, any composition for embolization well known in the art may also be appropriately selected without limitation as long as those can be properly mixed with the composition of the present invention. More preferably, considering the common knowledge in the art of performing embolization with an emulsion type injection, the contrast agent or lipiodol capable of forming a stable emulsion when mixed with the porous silica particles of the composition according to the present invention is selected.

Administration of the composition may be performed via a catheter having the advantages described above and, when the composition is administered into a vessel directly connected to the tumor via the catheter, damage to normal tissue may be prevented while targeting only target tumor tissues thus to enhance targeting effects.

Diseases able to be embolized using the composition may include at least one selected from the group consisting of, hepatocellular carcinoma, metastatic liver cancer, colon cancer, metastatic colon cancer, lung cancer, metastatic lung cancer, gastric cancer, pancreatic cancer, metastatic pancreatic cancer, skin cancer, melanoma, metastatic melanoma, osteosarcoma, fibrosarcoma, lipoma, gallbladder cancer, intrahepatic bile duct cancer, bladder cancer, uterine cancer, cervical cancer, ovarian cancer, breast cancer, head and neck cancer, thyroid cancer and kidney cancer, brain cancer, glioblastoma, mediastinal tumor, mesenteric lymph node metastasis, blood cancer, leukemia, non-Hodgkin's lymphoma, Hodgkin's lymphoma, multiple myeloma, lymphoma, malignant lymphoma, myelodysplastic syndrome, acute lymphoblastic leukemia, acute myeloid leukemia, chronic lymphocytic leukemia, chronic myelogenous leukemia, isolated myeloma, aplastic anemia, spinal muscular atrophy, hereditary disease, hereditary skeletal disease, hereditary malformation syndrome, autosomal recessive genetic disease, rare disease, infectious disease, ischemic disease, nasal polyps, sinusitis, hypertrophic scars, keloid, immune disease, autoimmune disease, infectious immune disease, viral infection, bacterial infection, rheumatoid arthritis, diabetes, diabetic complication, foot ulcer, neuropathy, metabolic syndrome, intestinal disease, atopy, allergy, lupus, dementia, Parkinson's disease, wound disease, laceration disease, skin diseases, bedsores, vascular diseases, arterial diseases, venous diseases, lymphatic diseases, cardiovascular diseases, ischemic heart diseases, cerebrovascular diseases, hypertension, dyslipidemia, arteriosclerosis, peripheral vascular diseases, and lower limb artery occlusion, but it is not limited thereto.

Hereinafter, the present invention will be described in detail with reference to the following examples.

In the following examples, the porous silica particles of the present invention may be referred to as DegradaBALL (Korean Trademark Registeration No. 40-1292208).

## Example 1 - Preparation of porous silica particles

(1) Preparation of particle 1

1) Preparation of small pore particles

960 ml of distilled water (DW) and 810 ml of MeOH were placed in a 2L round bottom flask. 7.88 g of CTAB was added to the flask, followed by rapid addition of 4.52 ml of 1 M NaOH while agitating. After introducing a uniform mixture while agitating for 10 minutes, 2.6 ml of TMOS was added thereto. After agitating for 6 hours to uniformly mix, the mixture was aged for 24 hours.

Then, the reaction solution was centrifuged at 8000 rpm and 25 °C for 10 minutes to remove the supernatant. During centrifugation at 8000 rpm and 25 °C for 10 minutes, the product was washed five times with ethanol and distilled water by turns.

Thereafter, the resultant was dried in an oven at 70 °C to obtain 1.5 g of powdery small pore porous silica particles (pore average diameter: 2 nm, particle diameter: 200 nm).

2) Pore expansion

1.5 g of small pore porous silica particle powders were added to 10 ml of ethanol for ultrasonic dispersion, and 10 ml of water and 10 ml of trimethyl benzene (TMB) were added for ultrasonic dispersion.

Thereafter, the dispersion was placed in an autoclave and reacted at 160 °C for 48 hours.

The reaction was carried out starting at 25 °C, followed by warming up at a rate of 10 °C/min then slowly cooling at a rate of 1 to 10 °C/min in the autoclave.

The cooled reaction solution was centrifuged at 8000 rpm and 25 °C for 10 minutes to remove the supernatant. During centrifugation at 8000 rpm and 25 °C for 10 minutes, the product was washed five times with ethanol and distilled water by turns.

Thereafter, the resultant was dried in an oven at 70 °C to obtain powdery small pore porous silica particles (pore average diameter of 10 to 15 nm and particle diameter of 200 nm).

3) Calcinations

The porous silica particles prepared in the above section 2) were put in a glass vial, heated at 550 °C for 5 hours, and cooled slowly to room temperature after the completion of the reaction, thereby preparing particles.

(2) Preparation of particle 2

Porous silica particles were prepared in the same manner as in Example 1-(1) except that the reaction conditions upon pore expansion were changed to 140 °C and 72 hours.

(3) Preparation of particles 3 (10 L scale)

Porous silica particles were prepared in the same manner as in Example 1-(1) except that a 5-fold large container was used and each material was used in 5-fold volume.

(4) Preparation of particle 4 (particle diameter: 300 nm)

Porous silica particles were prepared in the same manner as in Example 1-(1) except that 920 ml of distilled water and 850 ml of methanol were used to prepare small pore particles.

(5) Preparation of particle 5 (particle diameter: 500 nm)

Porous silica particles were prepared in the same manner as in Example 1-(1) except that 800 ml of distilled water, 1010 ml of methanol and 10.6 g of CTAB were used to prepare small pore particles.

(6) Preparation of particle 6 (particle diameter: 1000 nm)

Porous silica particles were prepared in the same manner as in Example 1-(1) except that 620 ml of distilled water, 1380 ml of methanol and 7.88 g of CTAB were used to prepare small pore particles.

(7) Preparation of particle 7 (pore diameter: 4 nm)

Porous silica particles were prepared in the same manner as in Example 1-(1) except that 2.5 ml of TMB was used upon pore expansion.

(8) Preparation of particles 8 (pore diameter: 7 nm)

Porous silica particles were prepared in the same manner as in Example 1-(1) except that 4.5 ml of TMB was used upon pore expansion.

(9) Preparation of particle 9 (pore diameter: 17 nm)

Porous silica particles were prepared in the same manner as in Example 1-(1) except that 11 ml of TMB was used upon pore expansion.

(10) Preparation of particles 10 (pore diameter: 23 nm)

Porous silica particles were prepared in the same manner as in Example 1-(1) except that 12.5 ml of TMB was used upon pore expansion.

(11) Preparation of particles 11 (dual modification)

1) Preparation of small pore particles

Small pore particles were prepared in the same manner as in Example 1-(1)-1).

2) Pore expansion

The small pore particles were reacted with TMB in the same manner as in Example 1-(1)-2), then cooled and centrifuged to remove the supernatant. After centrifugation under the same conditions as in Example 1-(1)-2), the product was washed three times with ethanol and distilled water by turns, and then, dried under the same conditions as in Example 1-(1)-2), thereby preparing porous silica particle powder (pore diameter: 10 to 15 nm, particle diameter: 200 nm).

3) Surface modification

After dispersing 0.8 to 1 g of porous silica particles having expanded pores in 50 ml of toluene, 5 ml of (3-aminopropyl) triethoxysilane was added thereto, followed by heating under reflux at 120 °C for 12 hours. After washing and drying as described above, 1 ml of triethylene glycol (PEG3, 2-[2-(2-methoxyethoxy)ethoxy]acetic acid) and 100 mg of EDC (1-ethyl-3-(3-dimethylaminopropyl)carbodiimide) and 200 mg of N-hydroxysuccinimide (NHS) were dispersed in 30 ml of PBS, and then subjected to reaction for 12 hours while agitating at room temperature. Thereafter, the product was washed and dried as described above.
Since the reaction solution of the previous step remained inside the pore, the inside of the pore was not modified.

4) Washing inside of the pore

800 mg of surface modified particle powders were dissolved in 40 ml of 2M HCl/ethanol and refluxed under vigorous agitation for 12 hours.
Thereafter, the cooled reaction solution was centrifuged at 8000 rpm for 10 minutes to remove the supernatant.

During centrifugation at 8000 rpm and 25 °C for 10 minutes, the product was washed five times with ethanol and distilled water by turns.

After drying in an oven 70 °C, powdery porous silica particles were obtained.

5) Modification of inside of the pore

(i) A propyl group was introduced into the pores in the same manner as in Example 2-(2)-1 described below.
(ii) An octyl group was introduced into the pores in the same manner as in Example 2-(2)-2 described below.

**Example 2 - Surface modification of porous silica particles**

(1) Positive charging

1) Amino group - Particles with 300 nm particle diameter

The porous silica particles in Example 1-(4) were reacted with (3-Aminopropyl)triethoxysilane (APTES) so as to be positively charged.

Specifically, 100 mg of porous silica particles were dispersed in 10 ml of toluene in a 100 ml round bottom flask by means of a bath sonicator. Then, 1 ml of APTES was added and agitated at 400 rpm and 130 °C for 12 hours.

After the reaction, the product was slowly cooled to room temperature, followed by centrifugation at 8000 rpm for 10 minutes to remove the supernatant. During centrifugation at 8000 rpm and 25 °C for 10 minutes, the product was washed five times with ethanol and distilled water by turns.

Then, the washed product was dried in an oven at 70 °C to obtain powdery porous silica particles having an amino group on the surface of the particle and the inside of the pore.

2) Amino group - Particles with 200 nm particle diameter

(i) The porous silica particles in Example 1-(1) was modified in the same manner as in Example 2-(1)-1 except that the particles were reacted with (3-Aminopropyl) triethoxysilane (APTES) so as to be positively charged, and 0.4 ml of APTES was added and the reaction time was 3 hours.
(ii) The porous silica particles of Example 1-(9) were modified in the same manner as in Example 2-(1)-1 except that the particles were reacted with (3-Aminopropyl) triethoxysilane (APTES) so as to be positively charged.
(iii) The porous silica particles of Example 1-(10) were modified in the same manner as in Example 2-(1)-1 except that the particles were reacted with (3-Aminopropyl) triethoxysilane (APTES) so as to be positively charged.

3) Amino group - Difference in surface modification between particles

(i) The porous silica particles subjected to the procedures in Example 1-(1)-1 to Example 1-(1)-3 were modified in the same manner as in Example 2-(1)-1 except that the particles were reacted with (3-Aminopropyl) triethoxysilane (APTES) so as to be positively charged.
(ii) The porous silica particles in Example 1-(9) were modified in the same manner as in Example 2-(1)-1 except that the particles were reacted with (3-Aminopropyl) triethoxysilane (APTES) so as to be positively charged, and the reaction time was 24 hours.

4) Aldehyde group

The porous silica particles in Example 2-(1)-3)-(ii) were reacted with glutaraldehyde (GA) so as to be positively charged.

More specifically, 100 mg of porous silica particles were dispersed in 10 ml of distilled water in a 100 ml round bottom flask by means of a bath sonicator. Thereafter, 10 ml of GA was added and reacted while agitating at 400 rpm and room temperature for 24 hours.

After the reaction, the supernatant was removed by centrifugation at 8000 rpm for 10 minutes. During centrifugation at 8000 rpm and 25 °C for 10 minutes, the product was washed five times with distilled water.

(2) Introduction of hydrophobic group

1) Propyl group

The porous silica particles in Example 1-(1) were modified in the same manner as in Example 2-(1), except that the particles were reacted with trimethoxy(propyl)silane to introduce a propyl group on the surface of the particle and the inside of the pore, 0.35 ml of trimethoxy(propyl)silane was added instead of APTES, and the reaction was conducted for 12 hours.

2) Octyl group

The porous silica particles in Example 1-(1) were modified in the same manner as in Example 2-(1), except that the particles were reacted with trimethoxy-n-octylsilane to introduce an octyl group on the surface of the particle and the inside of the pore, 0.5 ml of trimethoxy-n-octylsilane was added instead of APTES, and the reaction was conducted for 12 hours.

(3) Negative charging

1) Carboxyl group

The porous silica particles in Example 1-(1) were modified in the same manner as in Example 2-(1)-1, except that the particles were reacted with succinic anhydride so as to be negatively charged, imethyl sulfoxide (DMSO) was used instead of toluene, 80 mg of succinic anhydride was added instead of APTES, followed by reaction while agitating at room temperature for 24 hours, and DMSO was used for washing instead of distilled water.

2) Thiol group

Modification was performed in the same manner as in Example 2-(1)-1 except that 1.1 ml of MPTES was used instead of APTES.

3) Sulfonic acid group

100 mg of porous silica particles in Example 2-(3)-2) were dispersed in 1 ml of 1 M sulfuric acid aqueous solution and 20 ml of 30% hydrogen peroxide solution, agitated at room temperature to induce oxidation reaction, thus to oxidize thiol groups into sulfonic acid groups. Thereafter, the product was washed and dried in the same manner as in Example 2-(1)-1).

4) Methylphosphonate group

(i) The porous silica particles subjected to the procedures in Example 1-(1)-1 to Example 1-(1)-3 were reacted with (3-trihydroxylsilyl)propyl methylphosphonate (THMP) so as to be charged.

More specifically, 100 mg of porous silica particles were dispersed in 10 ml of distilled water in a 100 ml round bottom flask by means of a bath sonicator. Then, 3 ml of THMP and 1.5 ml of 1 M HCl aqueous solution were added thereto, and the mixture was agitated at 400 rpm and 130 °C for 24 hours.

After the reaction, the product was slowly cooled to room temperature and the supernatant was removed by centrifugation at 8000 rpm for 10 minutes. During centrifugation at 8000 rpm and 25 °C for 10 minutes, the product was washed five times with distilled water.

(ii) The porous silica particles in Example 1-(9) were modified in the same manner as in the above section (i) except that the particles were reacted with 3-(Trihydroxysilyl)propyl methylphosphonate (THMP) so as to be negatively charged.

(iii) The porous silica particles in Example 1-(10) were modified in the same manner as in the above section (i) except that the particles were reacted with 3-(Trihydroxysilyl)propyl methylphosphonate (THMP) so as to be negatively charged.

(4) Introduction of hydrophilic group - PEG

100 mg of the porous silica particles in Example 1-(1) was dispersed in 20 ml of a N,N'-disuccinimidyl carbonate (DSC) solution at a concentration of 50 $\mu$g/ml, and agitated at room temperature to bind DSC to the surfaces of the porous silica particles. Then, the particles were washed three times with 10 ml of distilled water, followed by dispersing 10 mg of PEG (HO-PEG-NH$_2$) having 4 kDa molecular weight and amino groups at the end thereof in 10 ml of the above solution and agitating the same at room temperature, whereby PEG is linked on the surfaces of the porous silica particles. Thereafter, the product was washed and dried in the same manner as in Example 2-(1)-1).

## Example 3 - Bioactive material loading

(1) Doxorubicin

Doxorubicin was loaded onto the negatively charged porous silica particles in Example 2-(3-4).
Specifically, 5 mg of porous silica particle powders and 2 mg of doxorubicin were mixed under distilled water, then the mixture was settled at room temperature for 1 hour.

(2) Irinotecan

5 mg of the negatively charged porous silica particle powders in Example 2-(3)-4) were dispersed in 1 ml of 1 x PBS, 2 mg of irinotecan was added thereto, followed by dispersing the mixture for 15 minutes and then settling the same at room temperature for 1 hour.

(3) Sorafenib

Sorafenib was loaded onto the porous silica particles of Example 1-(11)-5)-(i).
Specifically, 5 mg of porous silica particle powders and 2 mg of sorafenib were mixed in 1 ml of deionized water/ethanol in a 5:5 mixing ratio (by volume), and then incubated at room temperature for 1 hour. Thereafter, the product was washed three times with 1 ml of deionized water.

(4) Retinoic acid

1 ml of retinoic acid solution (50 mM ethanol) was added to 100 pg of the porous silica particle powders in Example 2-(1)-2)-(i), followed by settling the same at room temperature for 4 hours and then washing three times with 1 ml of ethanol.

(5) p53 Peptide

As the porous silica particles, the particles in Example 1-(11)-5)-(ii) were used.
The p53 peptide used herein was imitated with a portion of the p53 protein sequence involved in apoptosis mechanism. The imitated sequence relates to the sequence of a hydrophobic secondary helix structure part in which the p53 protein binds to the hMDM2 protein. Therefore, the p53 peptide acts as an antagonist of the hMDM2 protein.
The amino acid sequence of the p53 peptide (Cal. m.w. 2596.78, found by MALDI-TOF 2597.92) is shown in Formula 1 (N termial → C termial) below.

[Formula 1]     Z-Gly-Gly-Qln-Ser-Qln-Qln-Thr-Phe-Y-Asn-Leu-Trp-Arg-Leu-Leu-X-Qln-Asn-NH2 (SEQ ID NO: 9)

(wherein X is a non-natural amino acid with introduced azide functional group which is 2-amino-5-azido-pentanoic acid; Y is a non-natural amino acid with introduced alkyne functional group wherein 4-pentynoic acid is introduced on a side chain of D-Lys;
X and Y are linked together to form a triazole functional group via azide-alkyne cycloaddition or click reaction;
Z is 5(6)-carboxyfluorescein (FAM)).

After dissolving 1.3 mg (500 nmole) of p53 peptide in 100 $\mu$l of DMSO, the solution was mixed with 5 ml of an aqueous solution including 5 mg of porous silica particle powders dissolved therein in a 15 ml conical tube, followed by incubation at room temperature for 12 hours.
The porous silica particles loaded with p53 peptide were purified by centrifuging (9289 rcf, 8500 rpm, 20 minutes, 15 ml conical tube) and repeatedly washing the same with water three times.

(6) siRNA

21 base pair duplex siRNAs targeting green fluorescence protein (GFP) synthesized by Bionic, Inc., on request were purchased. (SEQ ID NO: sense; 5'-GGCUACGUCCAGGAGCGCACC-3' (SEQ ID NO: 1), antisense; 5' UGCGCUCCUGGACGUAGCCUU-3' (SEQ ID NO: 2)).

10 µg of the porous silica particles in Example 2-(1)-2)-(ii) and 50 pmol of siRNA were mixed under 1 x PBS conditions, and then, loaded at room temperature for 30 minutes.

(7) Plasmid DNA

6.7k base pair plasmid DNA (SEQ ID NO: 5) prepared to express GFP as pcDNA3.3 backbone was produced from bacteria and used after the purification.

10 µg of porous silica particles in Example 2-(1)-2)-(ii) and 0.25 µg of plasmid DNA were mixed under 1 x PBS conditions and loaded at room temperature for 30 minutes.

(8) Linear DNA

Forward primer-CMV promotor-eGFP cDNA-Reverse primer were prepared in sequential order, followed by PCR amplification, thereby obtaining 1.9k base pair linear DNA (SEQ ID NO: 6) to be used.

12.5 µg of the porous silica particles in Example 2-(1)-2)-(iii) and 0.25 µg of linear DNA were mixed under 1 x PBS conditions and loaded at room temperature for 30 minutes.

(9) Protein

1) BSA

100 µg of the porous silica particle powders in Example 2-(1)-2)-(ii) and 10 µg of BSA (Sigma-Aldrich, A6003) were mixed in 200 µl of 1 x PBS, and then incubated at room temperature for 1 hour.

2) IgG

100 µg of the porous silica particle powders in Example 2-(1)-2)-(ii) and 10 µg of anti-twist IgG (Santacruz, sc-81417) were mixed in 200 µl of 1 x PBS, and then incubated at room temperature for 1 hour.

3) RNase A

100 µg of the porous silica particle powders in Example 1-(9) and 10 µg of RNase A (Sigma-Aldrich, R6513) were mixed in 200 µl of 1 x PBS, and then incubated at room temperature for 1 hour.

4) Cas9

40 µg of the porous silica particle powders in Example 2-(1)-2)-(i), 4 µg of Cas9 protein (SEQ ID NO: 3), and 2.25 µg of guide RNA (SEQ ID NO: 4) were mixed in 10 µl of 1 x PBS, and then incubated at room temperature for 1 hour.

(5) Anti-PD-1 antibody

100 µg of the porous silica particle powders in Example 2-(3)-4)-(ii) and 50 µg of anti-PD-1 (BioXCell, BP0146) were mixed in 100 µl of distilled water, and then incubated at room temperature for 5 minutes.

(6) Anti-PD-L1 antibody

100 µg of the porous silica particle powders in Example 2-(3)-4)-(ii) and 50 µg of anti-PD-L1 (BioXCell, BP0101) were mixed in 100 µl of distilled water, and then incubated at room temperature for 5 minutes.

## <u>Experimental Example 1</u> - <u>Identification of porous silica particle formation and pore expansion</u>

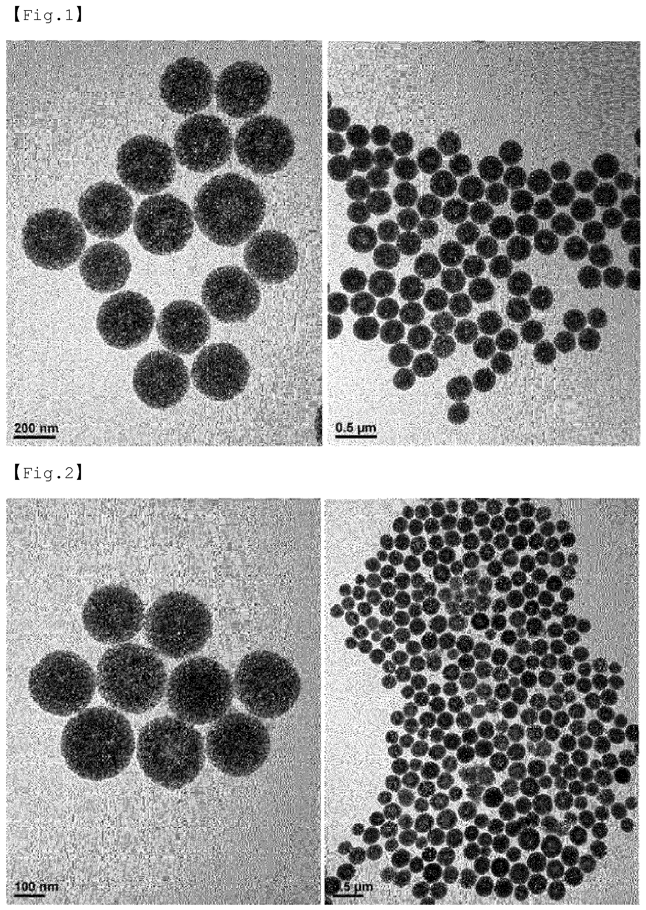

The small pore particles and the prepared porous silica particles in Examples 1-(1) to (3) were observed under a microscope to determine whether the small pore particles were uniformly formed and/or the pores were sufficiently expanded to uniformly form the porous silica particles (FIGS. 1 to 4).

FIG. 1 is microphotographs of the porous silica particles in Example 1-(1), and FIG. 2 is microphotographs of the porous silica particles in Example 1-(2), demonstrating that spherical porous silica particles with sufficiently expanded pores were evenly formed.

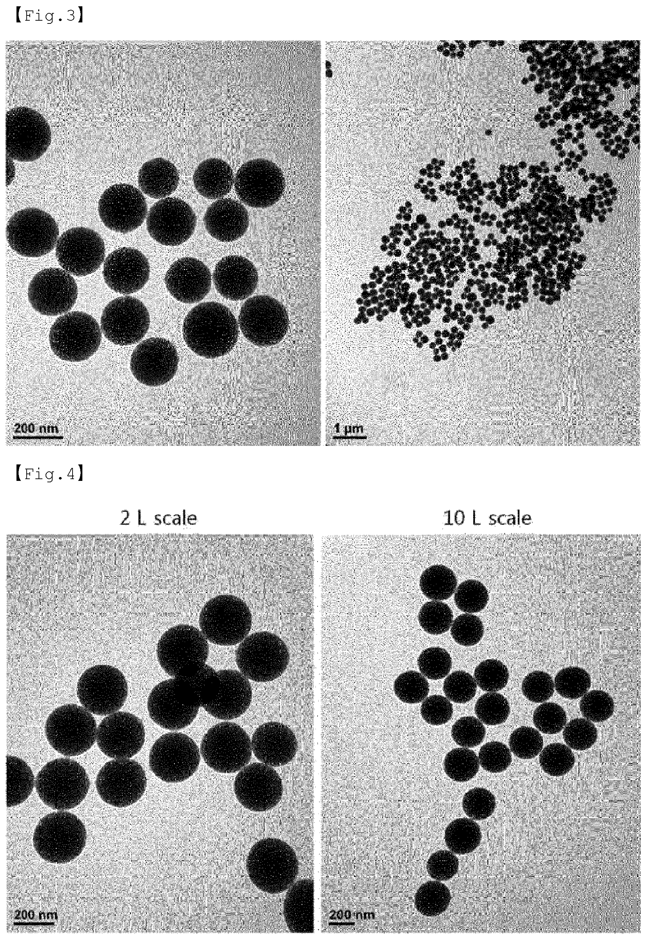

FIG. 3 is microphotographs of the small pore particles in Example 1-(1), and FIG. 4 is comparison microphotographs of the small pore particles in Example 1-(1) and Example 1-(3), demonstrating that spherical small pore particles were evenly formed.

**Experimental Example 2 - Calculation of BET surface area and pore volume**

Surface areas and pore volumes of the small pore particles in Examples 1-(1) and the porous silica particles in Examples 1-(1), (7), (8) and (10), respectively, were calculated. The surface area was calculated by Brunauer-Emmett-Teller (BET) method, while the pore size distribution was calculated by Barrett-Joyner-Halenda (BJH) method.

The microphotographs of the particles are shown in FIG. 5, and the calculation results are shown in Table 1 below.

[TABLE 1]

| Item | Pore diameter (nm) | BET surface area ($m^2$/g) | Pore volume (mL/g) |
|---|---|---|---|
| Small pore particles in Example 1-(1) | 2.1 | 1337 | 0.69 |
| Example 1-(7) | 4.3 | 630 | 0.72 |
| Example 1-(8) | 6.9 | 521 | 0.79 |
| Example 1-(1) | 10.4 | 486 | 0.82 |
| Example 1-(10) | 23 | 395 | 0.97 |

**Experimental Example 3 - Verification of biodegradability of porous silica particles**

In order to confirm the biodegradability of the porous silica particles in Example 1-(1), a degree of biodegradation at 37 °C and SBF (pH 7.4) were observed under a microscope at 0 hour, 120 hours and 360 hours, which are shown in FIG. 6.

Referring to the results, it can be seen that porous silica particles are biodegraded and almost completely degraded after 360 hours.

**Experimental Example 4 - Measurement of absorbance ratio of porous silica particles**

The absorbance ratio over time according to Equation 1 was measured.

$$A_t \; / A_0$$

(wherein $A_0$ is absorbance of the porous silica particles measured by placing 5 ml of a suspension including 1 mg/ml of the porous silica particles into a cylindrical dialysis membrane having pores with a diameter of 50 kDa,

15 ml of the same solvent as the suspension is placed outside the dialysis membrane while being in contact with the dialysis membrane, followed by horizontal agitation at 60 rpm and 37 °C inside and outside the dialysis membrane, and

At is absorbance of the porous silica particles measured after t hours elapses from the measurement of $A_0$).

Specifically, 5 mg of porous silica particle powders were dissolved in 5 ml of SBF (pH 7.4). Thereafter, 5 ml of the porous silica particle solution was placed in a dialysis membrane having pores with a diameter of 50 kDa shown in FIG. 7. Then, 15 ml of SBF was added to an outer membrane and the SBF of the outer membrane was changed every 12 hours. Degradation of the porous silica particles were performed while horizontally agitating at 60 rpm and 37 °C. Then, the absorbance was measured by UV-vis spectroscopy and analyzed at $\lambda$ = 640 nm.

(1) Measurement of absorbance ratio

The absorbance ratio of the porous silica particles in Example 1-(1) was measured according to the above method, and the results are shown in FIG. 8.

Referring to the results, it can be seen that t when the absorbance ratio becomes 1/2 was about 58 hours and the degradation proceeded very slowly.

(2) Measurement by particle diameter

Each absorbance of the porous silica particles in Examples 1-(1), (5) and (6), respectively, was measured according to Equation 1 above, and the results are shown in FIG. 9 (SBF was used as the suspension and the solvent).
Referring to the results, it can be seen that t decreases with increasing particle diameter.

(3) Measurement by pore average diameter

Each absorbance of the porous silica particles in Examples 1-(1) and (9), respectively, and the absorbance of the small pore silica particles in Example 1-(1) as a control, were measured according to Equation 1 above, and the results are shown in FIG. 10 (SBF was used as a suspension and a solvent).
Referring to the results, it can be seen that the porous silica particles in the examples have significantly larger t than the control.

(4) Measurement by pH

The absorbance of the porous silica particles in Example 1-(4) at each pH was measured. The absorbance was measured in SBF and in Tris at pH 2, 5, and 7.4, respectively, and the results are shown in FIG. 11.
Referring to the results, there is a difference in t to pH but, in all cases, t when the absorbance ratio becomes 1/2 was 20 or more.

(5) Measurement when charged

The absorbance of the porous silica particles of Example 2-(1)-1) was measured, and the results are shown in FIG. 12 (Tris (pH 7.4) was used as a suspension and a solvent) .
Referring to the results, in the case of positively charged particles, t when the absorbance ratio becomes 1/2 was 20 or more.

**Experimental Example 5 - Release of bioactive materials**

(1) doxorubicin

1) Dynamic condition

This simulates the environment in which a flow rate of blood stream is very high or the environment frequent high external shocks.
5 mg of porous silica particles loaded with doxorubicin (1 Mg) was dispersed in SBF (pH 7.4) (total volume: 1 ml), and then the solution was placed in a 1.5 ml tube and maintained in a dynamic condition of performing horizontal agitation at 20 rpm and 37 °C. At each time point, the porous silica solution loaded with doxorubicin was settled using a centrifuge, and the absorbance ($\lambda_{ab}$ = 480 nm) of the supernatant was measured to determine an amount of released doxorubicin. The results are shown in FIG. 13(A).
Referring to the results, it can be seen that doxorubicin is loaded on the surfaces of the particles with a relatively weak binding force and is relatively quickly released due to the high solubility of doxorubicin in SBF, about 1.5 hours elapses to reach a release rate of 50%, and the bioactive material was continuously released up to 12 hours or more.

2) Static condition

This simulates the environment in which a flow rate of blood stream is slow such as a tumor tissue, muscle tissue or tumor surrounding.
10 mg of porous silica particles loaded with doxorubicin (2 mg) were dispersed in SBF (pH 7.4) inside a dialysis membrane (total volume: 0.5 ml), and then the dialysis membrane was placed in a 1.5 ml SBF tube (pH 7.4) and maintained in static conditions at 37 °C. At each time point, the porous silica solution loaded with doxorubicin was settled using a centrifuge, and the absorbance ($\lambda_{ab}$ = 480 nm) of the supernatant was measured to determine an amount of released doxorubicin. The results are shown in FIG. 13(B).

Referring to the results, it can be seen that, although doxorubicin is loaded on the surfaces of the particles with a relatively weak binding force and is relatively quickly released due to the high solubility of doxorubicin in SBF, about 6 days have taken to reach a release rate of 50% and the bioactive material was continuously released up to 20 days or more.

(2) Irinotecan

1 Mg of porous silica particles loaded with irinotecan (0.2 mg) was dispersed in 1 ml of human plasma. The solution was maintained in a dynamic condition of performing horizontal agitation at 200 rpm and 37 °C. At each time point, the porous silica solution loaded with irinotecan was settled using a centrifuge, and the absorbance ($\lambda_{ab}$ = 255 or 278 nm) of the supernatant was measured to determine an amount of released irinotecan. The results are shown in FIG. 14.
Referring to the results, it can be seen that about 50% of irinotecan was released after 5.5 hours, and the bioactive material was continuously released up to 120 hours or more.

(3) Sorafenib

1 Mg of porous silica particles loaded with sorafenib (0.1 Mg) were dispersed in 10 ml of 1 x PBS. The solution was maintained under a dynamic condition of performing horizontal agitation at 200 rpm and 37 °C. At each time point, the porous silica solution loaded with sorafenib was settled using a centrifuge, and the absorbance ($\lambda_{ab}$ = 270 nm) of the supernatant was measured to determine an amount of released sorafenib. The results are shown in FIG. 15.
Referring to the results, it can be seen that sorafenib, a poorly soluble bioactive material, was released very slowly by interaction with porous silica particles having a hydrophobic substituent.

(4) Retinoic acid

0.1 mg of particles loaded with retinoic acid were placed in a PBS (pH 7.4) solution containing 5% ethanol and maintained at 37 °C while performing horizontal agitation. Every 24 hours, the solution containing the particles were centrifuged to measure the absorbance of the supernatant at a wavelength of 350 nm, thus to determine an amount of release retinoic acid. The results are shown in FIG. 16.
Referring to the results, it can be seen that the negatively charged retinoic acid was released very slowly due to interaction with the positively charged porous silica particles, and almost 100% was released for about 10 days.

(5) p53 peptide

5 mg of particles loaded with p53 peptide were placed in 5 ml of 1 x PBS containing 10% FBS or 5 ml of 1 x PBS, and maintained under a dynamic environment while rotating at 20 rpm and 37 °C. At each time point, centrifugation was conducted at 8500 rpm and a fluorescence intensity of 5(6)-carboxyfluorescein (FAM), which is a fluorescent label bound to p53 peptide from the supernatant (Absorbance: 480 nm, Emission: 520 nm). The results are shown in FIG. 17.
Referring to the results, it can be seen that the porous silica particles were loaded with p53 peptide by the binding force through hydrophobic property (hydrophobic effect) inside, therefore, the p53 peptide was not released within the PBS solution. However, when a protein such as FBS (fetal bovine serum) is present in the solution, the p53 peptide is bound to a hydrophobic segment of FBS protein and could be dissolved in the solution, and therefore, it can be seen that the p53 peptide was released outside the porous silica particles. Otherwise, while the p53 peptide loaded inside the particles is released outside the particles, FBS protein may be introduced into the particles.

(6) siRNA

1) Condition 1

10 $\mu$l of porous silica particles loaded with Cy5-siRNA was resuspended in SBF (pH 7.4, 37 °C) (total volume: 0.5 ml) and placed in a 1.5 ml tube. Release of siRNA was performed while horizontally agitating at 60 rpm and 37 °C. At each time point, the siRNA-loaded porous silica solution was settled using a centrifuge, and a fluorescence intensity of the supernatant was measured.
The fluorescence intensity of Cy5-siRNA was measured at 670 nm wavelength ($\lambda_{ex}$ = 647 nm) to determine a degree of release of siRNA, and the results are shown in FIG. 19(A).
Referring to the results, it can be seen that 50% of siRNA was released for about 6 hours.

2) Condition 2

20 μg of porous silica particles loaded with siRNA (1 μg) of the above 1) was dispersed in SBF (pH 7.4) inside a dialysis membrane (total volume: 0.5 ml), and the dialysis membrane was placed in a 1.5 ml SBF (pH 7.4) tube, and static conditions at 37 °C were maintained. At each time point, the siRNA-loaded porous silica solution was settled using a centrifuge, and the absorbance ($\lambda_{ab}$ = 480 nm) of the supernatant was measured to determine an amount of released siRNA. The results are shown in FIG. 19(B).

Referring to the results, it can be seen that about 48 hours elapses until siRNA reaches a release rate of 50%, and the bioactive material was continuously released up to 100 hours or more.

(7) Plasmid DNA

20 μg of porous silica particles loaded with pDNA (1 μg) were dispersed in SBF (pH 7.4) inside a dialysis membrane (total volume 0.5 ml), and the dialysis membrane was placed in a 1.5 ml SBF (pH 7.4) tube while shaking at 60 rpm and 37 °C. At each time point, the porous silica solution loaded with pDNA was settled using a centrifuge and the absorbance ($\lambda_{ab}$ = 480 nm) of the supernatant was measured to determine an amount of released pDNA. The results are shown in FIGS. 20 and 21.

Referring to the results, it can be seen that about 24 hours elapses to reach a release amount of pDNA reaches 50%, and the bioactive material was continuously released up to 100 hours or more.

(8) Linear DNA

Porous silica particles loaded with linear DNA (3 μg of linear DNA, 100 μg of porous silica particles) were resuspended in PBS (pH 7.4, 37 °C), and a dialysis membrane having a pore diameter of 20 kDa (the same tube as the tube in FIG. 18). After placing the suspension in the membrane, a dialysis tube was soaked in 1.5 ml of PBS. Release of Plasmid DNA was performed while horizontally agitating at 60 rpm and 37 °C.

The release solvent was recovered at 0.5h, 1h, 2h, 3h, 4h, 6h, 12h, and 24h points before 24 hours, and thereafter, 0.5 ml of the release solvent was collected for the Hoechst-binding assay at 24 hours, followed by addition of an equal amount of PBS.

A fluorescence intensity of Hoechst 33342 was measured at 460 nm wavelength ($\lambda_{ex}$ = 360 nm) to determine a degree of release of plasmid DNA, and the results are shown in FIG. 22.

Referring to the results, it can be seen that the release time of 50% linear DNA was about 24 hours.

(9) Protein

1) BSA

100 μg of porous silica particles loaded with Fluorescein fluorescence labeled BSA was resuspended in 200 μl of SBF (pH 7.4) or PBS (pH 7.4). Release of BSA was performed while horizontally agitating at 60 rpm and 37 °C.

At 6h, 12h, 24h, 48h, 96h, 144h and 240h points, 200 μl of release solvent was recovered for fluorescence measurement and an equivalent amount of SBF or PBS was added thereto.

A fluorescence intensity of Fluorescein fluorescence-labeled BSA was measured at 517 nm wavelength ($\lambda_{ex}$ = 492 nm) to determine a degree of release of BSA, and the results are shown in FIG. 23.

Referring to the results, BSA was released in a sustained manner in both the SBF and PBS, and it can be seen in every time period that a release amount was slightly higher in PBS than SBF, and almost 100% was released over 250 hours or more.

2) IgG

100 μg of porous silica particles loaded with Fluorescein fluorescence labeled IgG were resuspended in 200 μl of SBF (pH 7.4) or PBS (pH 7.4). Release of IgG was performed while horizontally agitating at 60 rpm and 37 °C.

At 6h, 12h, 24h, 48h, 96h, 144h and 240h points, 200 μl of release solvent was recovered for fluorescence measurement and an equivalent amount of SBF or PBS was added thereto.

A fluorescence intensity of Fluorescein fluorescence-labeled IgG was measured at 517 nm wavelength ($\lambda_{ex}$ = 492 nm) to determine a degree of release of BSA, and the results are shown in FIG. 24(A).

Referring to the results, it can be seen that IgG was released slowly in both SBF and PBS, and almost 100% was released over 250 hours or more.

3) Other antibodies

After dispersing 20 μg of porous silica particles loaded with antibody 1 (anti-PD-1) or antibody 2 (anti-PD-L1) (10 μg) in SBF (pH 7.4) (total volume: 1 ml), the solution was placed in a 1.5 ml tube, and maintained in a dynamic condition of performing horizontal agitation at 20 rpm and 37 °C. At each time point, the porous silica solution loaded with antibodies was settled using a centrifuge, and the absorbance of the supernatant ($\lambda_{ab}$ = 480 nm) was measured to determine an amount of released antibody. The results are shown in FIGS. 24(B) and (C).

Referring to the results, about 45 hours for antibody 1 and about 20 hours for antibody 2 elapses until the release rate reached about 50%. It can be seen that the release rate was higher in PBS than SBF at the initial time, however, was increased in SBF over time (100 hours as a turning point), and the antibody was continuously released up to 250 hours or more.

4) RNase A

100 μg of porous silica particles loaded with Fluorescein fluorescence labeled RNase A was resuspended in 200 μl of SBF (pH 7.4) or PBS (pH 7.4). Release of RNase A was performed while horizontally agitating at 60 rpm and 37 °C.

At 6h, 12h, 24h, 48h, 96h, 144h and 240h points, 200 μl of release solvent was recovered for fluorescence measurement, and an equivalent amount of SBF or PBS was added thereto.

A fluorescence intensity of Fluorescein fluorescence-labeled RNase A was measured at 517 nm wavelength ($\lambda_{ex}$ = 492 nm) to determine a degree of release of BSA, and the results are shown in FIG. 25.

Referring to the results, it can be seen that, although RNase A is released slowly from both SBF and PBS, the release amount was slightly higher in PBS than SBF in every time period, and almost 100% was released over 250 hours or more.

5) Cas9

40 μg of porous silica particles loaded with Cas9 protein/guide RNA complex were suspended in PBS (pH 7.4), and then the porous silica particles were treated in serum-free media on a slide glass of 50,000 NIH 3T3 cells known as mouse fibroblasts, followed by incubation at 5% $CO_2$ and 37 °C.

At 1h, 3h, 6h, and 24h points, the medium was removed, and the product was washed with 1 x PBS solution, and incubated with 4% paraformaldehyde for 15 minutes to fix cells.

After washing with PBS, the cells were incubated for 1 hour in a blocking buffer (1 x PBS, 5% normal goat serum, 0.3% triton X-100).

After washing with PBS, His tag antibody (Santa Cruz, sc-8036) was incubated for 16 hours.

After washing with PBS again, Alexa Fluor 488-linked anti-mouse secondary antibody (Abcam, ab150113) was incubated for 2 hours.

After washing with PBS, the slide glass was treated with DAPI to stain nuclei of the cells. The distribution of protein in the cells was identified using a fluorescence microscope, and the results are shown in FIG. 26.

In FIG. 26, DAPI is a reagent for staining nucleus, which appears blue in a fluorescence microscope image, and indicates a location of the cell nucleus. Further, Alexa Fluor 488 is a fluorescent dye labeled with Cas9 protein, which appears green in the fluorescence microscope image and indicates a location of the intracellular Cas9 protein. When the silica particles loaded with Cas 9 protein labeled Alexa Fluor488 were applied to the cells and subjected to DAPI staining, the presence or absence of the Cas 9 protein in the cells by the silica particles and the position of the cell nucleus may be demonstrated on the fluorescence microscope image.

Referring to the results, Cas9 protein introduced into the cell is mainly observed in the cytoplasmic part 3 hours after the introduction, while being observed in the nucleus after 24 hours. Since the used silica particles substantially hardly enter into the cell nucleus, it is understood that the Cas9 protein is released from the silica particles after 24 hours in the cell and enters the nucleus known as an intracellular organelle where the Cas9 protein accumulates.

**Experimental Example 6 - Delivery of bioactive material and treatment of disease**

(1) Direct delivery in cancer

In order to verify a possible role of the carrier in siRNA delivery studies at an animal level, tumor inhibition by the release of bioactive materials in mice was investigated.

Balb/c nude male mice (5 weeks old) were purchased from Orient Bio, Inc., and 3 million HeLa cells (cervical cancer cells) were dispersed in sterilized 1 x PBS to proliferate Xenograft tumors subcutaneously injected into the mice. When 70 mm$^3$ size of solidified tumors were observed, PBS, FITC-porous silica particles (porous silica particles in Example 2-(1)-2)-(ii)), and FITC-porous silica particles loaded with Cy5-siRNA (porous silica particles in Example 2(1)-2)-(ii)) were injected into tumors in the mice, respectively. Then, fluorescence intensities and distribution thereof were measured immediately before, immediately after, and 48 hours after the administration, by means of FOBI Fluorescence *in vivo* imaging system (Neo science, Korea).

FITC labeling was performed by: dispersing 50 mg of silica particles in 1 ml of dimethyl sulfoxide (DMSO); adding 25 μg (10 μl) of FITC-NHS (N-hydroxysuccinimide) solution (2.5 mg/mL) thereto; reacting the mixture at room temperature for 18 hours while shielding light with aluminum foil; purifying the reaction product through centrifugation (8500 rpm, 10 minutes); discarding the supernatant while collecting settled particles; and evenly dispersing the particles in ethanol, wherein the above processes were repeated three and four times with ethanol and distilled water to purify until FITC color is invisible in the supernatant. The results are shown in FIG. 27(A).

In FIG. 27(A), the control refers to administration of PBS alone, cy5-siRNA refers to administration of cy5-siRNA alone, FITC-DDV refers to administration of FITC-labeled porous silica particles alone, and the complex refers to administration of porous silica particles loaded with cy5-siRNA and labeled with FITC. Referring to this figure, it can be seen that siRNA loaded on the particles and delivered into the body has a longer duration of activity and stays longer at the injected site, thereby exhibiting strong fluorescence even after 48 hours.

(2) Delivery through intravenous injection

1) Experiment method

(i) Doxorubicin

Xeno was prepared in Balb/C nude mouse using HepG2 cells as a human liver cancer cell line. When a size of Xeno became suitable for experiment (50 to 100 mm³), the particles in Example 2-(3)-4)-(i) were loaded with doxorubicin, dispersed in 100 μl of PBS aqueous solution and injected through tail vein of the mouse. An injecting dose of doxorubicin was 4 mg/kg (mouse weight), and 80 μg of doxorubicin and 160 μg of particles were used based on an average body weight of 20 g of 5 to 8-week old Balb/C nude mouse.

(ii) VEGF inhibitory siRNA

Xeno was prepared in Balb/C nude mouse using MDA-MB-231 cells as a human breast cancer cell line. When a size of Xeno became suitable for experiment (50 to 100 mm³), the particles in Example 2-(1)-2)-(ii) were loaded with VEGF inhibitory siRNA (SEQ ID NO: 7 sense; 5'-GGAGUACCCUGAUGAGAUCdTdT-3', SEQ ID NO: 8 antisense; 5'-GAUCUCAUCAGGGUACUCCdTdT-3'), dispersed in 100 μl of PBS aqueous solution, and injected through tail vein of the mouse. An injecting dose of VEGF inhibitory siRNA was 1 mg/kg (mouse weight), and 20 μg of siRNA and 400 μg of particles were used based on the average body weight of 20 g of 5 to 8-week old Balb/C nude mouse.

(iii) Rnase A

Xeno was prepared in Balb/C nude mouse using HeLa cells as a human cervical cancer cell line. When a size of Xeno became suitable for experiment (50 to 100 mm³), the particles in Example 1-(9) were loaded with Rnase A, dispersed in 100 μl of PBS aqueous solution and injected through tail vein of the mouse. An injecting dose of Rnase A dose was 2 mg/kg (mouse weight), and 40 μg of Rnase A and 400 μg of particles were used based on an average body weight of 20 g of 5 to 8-week old Balb/C nude mouse.

(iv) p53

Xeno was prepared in Balb/C nude mouse using HeLa cells as a human cervical cancer cell line. When a size of Xeno became suitable for experiment (50 to 100 mm³), the particles in Example 1-(11)-5)-(ii) were loaded with p53 peptide, dispersed in 100 μl of PBS aqueous solution and injected through tail vein of the mouse. An injecting dose of p53 peptide was 2.5 mg/kg (mouse weight), and 50 μg of the p53 peptide and 200 μg of the particles were used based on an average body weight of 20 g of 5 to 8-week old Balb/C nude mouse.

2) Experiment result

Referring to FIG. 27(B), in all cases in which the doxorubicin, VEGF inhibitory siRNA, Rnase A or p53 was loaded on the porous silica particles of the present invention, followed by injecting the particles, tumor growth inhibition and inhibition of VEGF expression were excellent as compared to injection of each of doxorubicin, VEGF inhibitory siRNA, Rnase A or p53 alone. These results demonstrated functional effects based on intrinsic properties such as excellent intravascular delivery and biodegradability of the particles according to the present invention.

(3) Delivery to blood vessels around tumor via catheter

For effective delivery of the bioactive material to tumor, after inserting a catheter into the artery approaching the same to tumor-associated vessels, the porous silica particles of the present invention loaded with an anticancer agent were delivered through the vessels (FIG. 27(C)). Referring to FIG. 27(C), it can be seen that a composition including the porous silica particles of the present invention mixed with a contrast agent was accurately target-delivered without blocking the catheter and blood vessels and/or precipitation or aggregation, as shown by black drops in FIG. 27(C).

**Experimental Example 7** - **Measurement of zeta potential in porous silica particles**

(1) Experimental method

100 $\mu$g of porous silica particles were dispersed in 1 ml of PBS (pH 7.4), transferred to a disposable folded capillary cell (DTS1070), and then mounted on a zeta potential measurement device to measure the zeta potential.

(2) Experiment result

Referring to FIG. 28, P = O vibration peak, P-CH$_3$ rocking peak and P-CH$_3$ wagging peak appeared in the FT-IR spectrum, indicating that anionic functional groups were introduced onto the surfaces of the particles and are negatively charged.

Referring to Table 2 below, it can be seen that the porous silica particles may have a variety of zeta potentials depending on which functional groups are modified, and it can also be seen that the types of loaded bioactive materials are diversified. Specifically, it can be seen that the porous silica particles of the present invention exhibited a zeta potential of +3 mV or more and -18 mV or less, and more efficiently loaded the bioactive material by dual modification (e.g., PEG) to the hydrophobic functional group.

[TABLE 2]

| Particle | Functiona l group | Zeta potential | Proper bioactive material |
|---|---|---|---|
| Example 1-(1) | Si-OH (unmodifi ed) | Negative charge (about -18 mV) | |
| Example 2-(3)-4) | OPMeO$_2$H | Negative charge (about -30 mV) | Positively charged low molecular weight compound |
| Example 2-(1)-3)-(i) | NH$_2$ | Positive charge (about +15 mV) | Negatively charged low molecular weight compound |
| Example 2-(1)-3)-(ii) | NH$_2$ | Positive charge (about +20 mV) | RNA, DNA |
| Example 2-(3)-1) | COOH | Negative charge (about -26 mV) | Positively charged low molecular weight compound |
| Example 2-(3)-3 | SO$_3$H | Negative charge (about -27 mV) | Positively charged low molecular weight compound |
| Example 2-(3)-5 | CHO | Positive charge (about +8 mV) | Molecules that can form schiff base (imine) linkage |
| Example 1-(11)-5)-(i) | C$_3$, PEG | Positive charge (about +3.5 mV) | Hydrophobic molecule |
| Example 1-(11)-5)-(ii) | C$_8$, PEG | Positive charge (about +3.6 mV) | Hydrophobic nolecule |

**Experimental Example 8** - **Analysis of stability of porous silica particles in blood**

(1) Experimental method

10 mg of the particles in Example 1-(1) and 10 mg of the particles in Example 2-(3)-4)-(i) were dispersed in a PBS aqueous solution and 1 ml of 25% plasma solution, respectively, and then placed at room temperature for 1 hour, followed by removing the supernatant. The above solutions were subjected to comparion in terms of an amount of particles not dispersed but settled in the solution. Further, amounts of particles stably dispersed in the aqueous PBS solution and 25% plasma solutions, respectively, were compared by comparing the absorbance of the removed supernatants.

After dispersing 4 ml of human blood in 15 ml of PBS solution, the solution was centrifuged at 10,000 rpm for 5 minutes using a centrifuge, and 15 ml of the supernatant was discarded. This process was repeated five times to remove proteins except red blood cells that remained in the blood. The separated red blood cells were dispersed in 40 ml of PBS solution. The particles in both the Example 1-(1) and Example 2-(3)-4)-(i) were prepared at sequentially decreased concentrations from the highest value of 20 mg, and then dispersed in 0.8 ml of PBS solution, respectively. 0.2 ml of the red blood cell solution separately prepared above and then dispersed in a PBS solution was added to the above solution, followed by shielding the light at room temperature and placing the mixture in a rotary agitator at 80 rpm for 4 hours. After 4 hours, the particles were completely settled using a centrifuge at 10,000 rpm and 4 °C for 3 minutes, and the supernatant was subjected to absorbance measurement at 577 nm to compare degrees of hemolysis of erythrocytes. 100% hemolysis is defined to indicate that 0.8 ml of distilled water was used instead of the solution in which the particles were dispersed, while 0% hemolysis is defined to indicate that 0.8 ml of PBS solution was used instead of the solution in which the particles were dispersed.

(2) Experiment result

Referring to FIG. 29, it can be seen that the degree of precipitation or aggregation of the porous silica particles according to the present invention was significantly lower in both the aqueous PBS solution and 25% plasma solution conditions. More specifically, the control group exhibited precipitation rates of 80% and 70% in the PBS aqueous solution and 25% plasma solution, respectively. On the other hand, the particles of the invention exhibited precipitation rates of only 4% and 5%, respectively. The reason is that surface charge (zeta potential (mV)) was generated through surface treatment of the porous silica particles of the present invention, and a repulsive force between the particles was induced thus to maintain a stable solution.

Referring to FIG. 30, it can be seen that, even when erythrocytes were treated with a high concentration of particles of the present invention, hemolysis did not occur. On the other hand, referring to FIG. 31, in the case of porous silica particles (Silanol-MSN) without surface modification with other functional groups, it can be seen that the hemolysis of erythrocytes was increased depending on the concentration of particles. The reason is that the silica particles of the present invention were mostly modified with other functional groups including sulfonate, aldehyde, polyethyleneglycol, methyl phosphonate and amine functional groups instead of silanol group. Therefore, it is considered that: interaction with a quaternary ammonium group on the surface of the erythrocyte is not strong; a surface area in contact with erythrocytes is small due to a porous structure including numerous pores thus to reduce the interaction; and the particles have a diameter of 100 nm or more, thereby considering that erythrocyte hemolysis is significantly lower than that of the conventional silica particles.

**Experimental Example 9** - **Bioactive material loading capacity of porous silica particles**

(1) Experimental method

1) Doxorubicin

After loading the negatively charged porous silica particles of Example 2-(3)-4)-(i) with doxorubicin, the absorbance of the supernatant was measured to determine an amount of loaded doxorubicin, and a loading rate ("loading capacity") of the particles was calculated.

Specifically, 5 mg of porous silica particle powders and 2 mg of doxorubicin were mixed in 1 ml of distilled water, and then settled at room temperature for 1 hour. Subsequently, the solution was centrifuged at 8000 rpm for 10 minutes to settle the particles, and absorbance ($\lambda_{ab}$ = 480 nm) of the supernatant was measured to determine an amount of the low molecular weight compound remaining in the supernatant without loading. Further, an amount of the loaded low molecular weight compound was calculated (an amount of the loaded low molecular weight compound = an amount of initially added low molecular weight compound - an amount of the low molecular weight compound remaining in the supernatant) so as to determine the loading capacity (loading capacity = bioactive material/porous silica particles, w/w%)

2) Irinotecan

After loading the negatively charged porous silica particles in Example 2-(3)-4)-(i) with irinotecan, absorbance of the supernatant was measured and an amount of loaded irinotecan was calculated, and the loading capacity thereof was determined. The procedure for calculating the loading capacity was performed in the same manner as in Experimental Example 9-(1)-1) except that the absorbance was measured at $\lambda_{ab}$ = 255 nm.

3) Sorafenib

5 mg of porous silica particles in Example 1-(11)-5)-(i) and 2 mg of sorafenib were mixed in 1 ml of deionized water/ethanol in a 5:5 mixing ratio (by volume), followed by loading at room temperature for 1 hour. The procedure for calculating a loading capacity was performed in the same manner as in Experimental Example 9-(1)-1) except that the absorbance was measured at $\lambda_{ab}$ = 270 nm.

4) Retinoic acid

1 ml of retinoic acid solution (50 mM ethanol) was added to 100 µg of the porous silica particles in Example 2-(1)-2)-(i), followed by loading at room temperature for 4 hours. The procedure for calculating a loading capacity was performed in the same manner as in Experimental Example 9-(1)-1) except that the absorbance was measured at $\lambda_{ab}$ = 350 nm.

5) p53 peptide

5 mg of the porous silica particles in Example 1-(11)-5)-(ii) was dispersed in 100 µl of fluorescence (FAM)-labeled p53 peptide solution (13 mg/ml, DMSO), placed in a 15 ml conical tube, and then incubated at room temperature for 12 hours. Thereafter, the porous silica particles containing p53 peptide were centrifuged (9289 rcf, 8500 rpm, 20 minutes, 15 ml conical tube), and then fluorescence of the supernatant was measured to calculate an amount of peptide loaded on the particles.

6) siRNA

After loading the positively charged particles in Example 2-(1)-3)-(ii) with siRNA, siRNA remaining in the supernatant was measured to calculate an amount of loaded siRNA, thereby determining a loading capacity thereof. Specifically, 20 µg of porous silica particles were dispersed in 10 µl of PBS aqueous solution, then 1 µg of siRNA was added thereto, and the mixture was settled at room temperature for 30 minutes. Then, the solution was centrifuged at 8000 rpm for 10 minutes and the amount of siRNA remaining in the supernatant was measured using polyacrylamide gel electrophoresis (PAGE), and the amount of siRNA loaded on the particles was calculated.

7) mRNA

After loading the positively charged particles in Example 2-(1)-3)-(ii) with mRNA having a sequence of the following Formula (2), an amount of mRNA remaining in the supernatant was measured to calculate an amount of loaded mRNA, thereby determining a loading capacity thereof. Specifically, 20 µg of porous silica particles were dispersed in 10 µl of PBS aqueous solution, 1 µg of mRNA was added thereto, and the mixture was settled at room temperature for 30 minutes. Thereafter, the solution was centrifuged at 8000 rpm for 10 minutes, then the amount of mRNA remaining in the supernatant was measured using agarose gel, and the amount of mRNA loaded on the particles was calculated.

```
[Formula 2]

TTTGTTCATAAACGCGGGGTTCGGTCCCAGGGCTGGCACTCTGTCGATACC

CCACCGAGACCCCATTGGGGCCAATACGCCCGCGTTTCTTCCTTTTCCCCACCCCACCCC

CCAAGTTCGGGTGAAGGCCCAGGGCTCGCAGCCAACGTCGGGGCGGCAGGCCCTGCCATA

GCAGATCTGCGCAGCTGGG(A) ≥ 100 (SEQ ID NO: 10)
```

8) pDNA

After loading the positively charged particles in Example 2-(1)-3)-(iii) with pDNA, an amount of pDNA remaining in the supernatant was measured to calculate an amount of loaded pDNA, thereby determining a loading capacity thereof. Specifically, 20 $\mu$g of porous silica particles were dispersed in 10 $\mu$l of PBS aqueous solution, 1 $\mu$g of pDNA was added thereto, and the mixture was settled at room temperature for 30 minutes. Thereafter, the solution was centrifuged at 8000 rpm for 10 minutes, then the amount of pDNA remaining in the supernatant was measured using agarose gel, and the amount of pDNA loaded on the particles was calculated.

9) Linear DNA

After loading the positively charged particles in Example 2-(1)-3)-(iii) with linear DNA, an amount of pDNA remaining in the supernatant was measured to calculate an amount of loaded linear DNA, thereby determining a loading capacity thereof. Specifically, 20 $\mu$g of porous silica particles were dispersed in 10 $\mu$l of PBS aqueous solution, 1 $\mu$g of linear DNA was added thereto, and the mixture was settled at room temperature for 30 minutes. Thereafter, the solution was centrifuged at 8000 rpm for 10 minutes, then the amount of linear DNA remaining in the supernatant was measured using agarose gel, and the amount of linear DNA loaded on the particles was calculated.

10) Protein

(i) BSA

After loading the positively charged porous silica particles with BSA, an amount of BSA remaining in the supernatant was measured to calculate an amount of loaded BSA, thereby determining a loading capacity thereof.

Specifically, 100 $\mu$g of the porous silica particle powders in Example 2-(1)-2)-(ii) and 10 $\mu$g of BSA (Sigma-Aldrich, A6003) were mixed in 200 $\mu$l of 1 x PBS, followed by incubation at room temperature for 1 hour. Thereafter, the solution was centrifuged at 8000 rpm for 10 minutes to settle the particles, then 10 $\mu$l of the supernatant was collected and mixed well with 200 $\mu$l of 5-fold diluted Bradford reagent, and the absorbance thereof was measured at $\lambda_{ab} = 595$ nm thus to determine an amount of BSA remaining in the supernatant without being loaded. At this time, a BSA solution was mixed with Bradford reagent while diluting the solution to reduce a concentration thereof, and then the absorbance of the solution was measured and compared with the standard curve of BSA, thereby accurately calculating a loading capacity of BSA.

(ii) IgG

The same procedure as in Experimental Example 9-(1)-10)-(i) was conducted, except that 100 $\mu$g of the porous silica particle powders in Example 2-(1)-2)-(ii) and 10 $\mu$g of anti-twist IgG (Santacruz, sc-81417) were mixed in 200 $\mu$l of 1 x PBS, followed by incubation at room temperature for 1 hour and loading the same.

(iii) RNase A

The same procedure as in Experimental Example 9-(1)-10)-(i) was conducted, except that 100 $\mu$g of the porous silica particle powders in Example 1-(9) and 10 $\mu$g of RNase A (Sigma-Aldrich, R6513) were mixed in 200 $\mu$l of 1 x PBS, followed by incubation at room temperature for 1 hour and loading the same.

(iv) Cas9

The same procedure as in Experimental Example 9-(1)-10)-(i) was conducted, except that 40 $\mu$g of the porous silica particle powders in Example 2-(1)-2)-(i), 4 $\mu$g of Cas9 protein (SEQ ID NO: 3) and 2.25 $\mu$g of guide RNA (SEQ ID NO: 4) were mixed in 10 $\mu$l of 1 x PBS, followed by incubation at room temperature for 1 hour and loading the same.

(v) Anti-PD-1 antibody

The same procedure as in Experimental Example 9-(1)-10)-(i) was conducted, except that 100 $\mu$g of porous silica particle powder in Example 2-(3)-4)-(ii) and 50 $\mu$g of anti-PD-1 (BioXCell, BP0146) were mixed in 100 $\mu$l of distilled water, followed by incubation at room temperature for 5 minutes and loading the same.

(vi) Anti-PD-L1 antibody

The same procedure as in Experimental Example 9-(1)-10)-(i) was conducted, except that 100 μg of porous silica particle powder in Example 2-(3)-4)-(ii) and 50 μg of anti-PD-L1 (BioXCell, BP0101) were mixed in 100 μl of distilled water, followed by incubation at room temperature for 5 minutes and loading the same.

(2) Experiment result

Referring to FIG. 32, as a result of precipitating the porous silica particles loaded with doxorubicin through centrifugation, it can be seen that the doxorubicin was mostly loaded on the particles and thus the solution has remarkably transparent color compared to the control group.

Referring to Table 3 below, loading capacities of different bioactive materials to the porous silica particles (bioactive material/porous silica particles) (w/w%) according to the above experimental procedure could be demonstrated.

[TABLE 3]

| Bioactive materials | Loading capacity (w/w %) |
|---|---|
| **Small molecules** : irinotecan, sorafenib, regorafenib, tamoxifen, gefitinib, erlotinib, afatinib, bleomycin, dactinomycin, daunorubicin, idarubicin, plicamycin, mitoxantrone, epirubicin, carboplatin, oxaliplatin, 5-fluorouracil, gemcitabine, temozolomide, alkylating agents (cisplatin, chlorambucil, procarbazine, carmustine, etc.), antimetabolites (methotreaxate, cytarabine, gemcitabine, etc.), anti-microtublue agents (vinblastine, paclitaxel, etc.), topoisomerase inhibitors (etoposide, doxorubicin, etc.), cytotoxic agents (bleomycin, mitomycin, etc.), metformine, etc. | 10-30 |
| **Antibodies** : specific target antibodies for PD-1, PD-L1, CTLA4, LAG3, OX40, KIR, CD137, CD276, GITR, CD27, 4-1BB, VISTA, TIM-3, CDs (CD3, CD20, CD28, CD130, etc), Immune Checkpoint Inhibitors, VEGFRs, VEGFs, PDGFRs, EGFRs, HER2/neu, estrogen receptors, etc. | 10-60 |
| **Cytokine, Chemokine, Growth Factor, etc** : anti-tumor cytokines, chemokines, growth factors (VEGF, EGF, LTF, HGF, etc.), interleukins (IL-2, IL-7, IL-12, IL-23, IL-1$\alpha$, IL-1Receptor alpha, IL-5, IL-6, IL-7, IL-10, IL-12 p70, IL-18, etc), FGFs, G-CSF, interferons (IFN-alpha 2 beta, IFN-gammar, etc.), PDGF-BB, TNF-alpha, OX40L, 4-1BB, etc. | 5-50 |
| **Peptides, Aptamers:** p53, LTF, EGF, VEGF, HGF, growth factors, cytokines, chemokines, vaccines, antibodies, etc. | 5-50 |
| **Proteins:** enzymes (caspases, ribonuclease (Rnase, Ribonuclease A, etc.), proteasomes, kinase, phosphatase, alkaline phosphatase, phospholipase, etc), antibodies, toxins (botulinum toxin, etc.), TGF-beta superfamily, interleukin superfamily, M-CSF, hemoglobin, beta-galactosidase, KRAS, OX40L, relaxin, blood factors (Factor VII, Factor VIII, and Factor IX, albumin, etc.), cytokine, growth factors, hormone, interferons (IFN-alpha, IFN-beta, IFN-gamma, etc.), lectin, glycosylated proteins, glycoproteins, SUMOylated proteins, phosphorylated proteins, transcription factors, reprogramming factors, erythropoietin, TNFs, cas9, CRISPR, etc. | 5-50 |
| **siRNA** : siRNA specific for mammalian expressing genes (VEGF, CTGF, TSLP, beta-catenin, HIFs, STATs, Notch, etc), etc. | 10-30 |
| **mRNA:** interleukins (IL-2, IL-7, IL-12, IL-23, IL-1$\alpha$, IL-1Receptor alpha, IL-5, IL-6, IL-7, IL-10, IL-12 p70, IL-18, etc), FGFs, G-CSF, interferons (IFN-alpha 2 beta, IFN-gammar, etc.), PDGF-BB, TNF-alpha, VEGF, EGF, LTF, OX40L, 4-1BB, etc. | 5-30 |
| **DNA** (circular plasmid DNA and/or loop-shape contained DNA, etc.): interleukins (IL-2, IL-7, IL-12, IL-23, IL-1$\alpha$, IL-1Receptor alpha, IL-5, IL-6, IL-7, IL-10, IL-12 p70, IL-18, etc.), FGFs, G-CSF, interferons (IFN-alpha 2 beta, IFN-gammar, etc.), PDGF-BB, TNF-alpha, VEGF, EGF, LTF, OX40L, 4-1BB, etc. | 5-30 |
| **Linear DNA** (single strand DNA, double strand DNA, etc.): interleukins (IL-2, IL-7, IL-12, IL-23, IL-1$\alpha$, IL-1Receptor alpha, IL-5, IL-6, IL-7, IL-10, IL-12 p70, IL-18, etc), FGFs, G-CSF, interferons (IFN-alpha 2 beta, IFN-gammar, etc) PDGF-BB, TNF-alpha, VEGF, EGF, LTF, OX40L, 4-1BB, DNAzyme, etc. | 5-30 |
| **Vaccines:** anti-virus vaccines, anti-tumor vaccines, anti-bacteria vaccines, etc. | 5-30 |

(continued)

| Bioactive materials | Loading capacity (w/w %) |
|---|---|
| **Gene editing elements:** CRISPRs, Cas9, zinc finger nucleases, TALEN, Hybrid Meganulcease, etc. | 5-40 |
| **Polymer:** natural polymer, synthetic polymer, organic polymer, inorganic polymer, chitosan, alginate, dextran, pectin, hybrid polymer, collagen, hyaluronic acid, PLLA, PLGA, PMMA, hydrogel, etc. | 5-50 |

## Experimental Example 10 - Cytotoxicity test of porous silica particles

10,000 HepG2 cells per well were spread in a 96-well plate and, after 24 hours, the particles in Example 2-(3)-4) were dispersed sequentially at a lower concentration to the highest concentration of 1 Mg in each well, and then left for 24 hours. A survival rate of HepG2 cells was determined using a cell counting kit (CCK), and the results are shown in FIG. 33.

From FIG. 33, it can be seen that the composition including the porous silica particles of the present invention did not affect the survival rate of the HepG2 cell line regardless of the concentration, and no cytotoxicity was observed.

## Experimental Example 11 - Stability and targetability of composition for embolization, including porous silica particles

(1) Verification of stability when mixing with embolic material

An emulsion was prepared by mixing 1.6 ml of lipiodol widely used as an embolic material and 0.4 ml of porous silica particles loaded with doxorubicin, which was dropped onto a transparent plastic plate in a form of droplets and was photographed by means of a fluorescence microscope. The photographed fluorescent images are shown in FIG. 34.

Referring to FIG. 34, it can be seen that the emulsion form (B) mixed with the porous silica particles maintained the emulsion in uniform size for a longer time than the emulsion of lipiodol alone (A). therefore, it is understood that the porous silica particles of the present invention are suitable to be mixed and used with an embolic material such as lipiodol, may reduce aggregation and/or precipitation, thereby achieving excellent embolic effects and treatment effects.

(2) Verification of targetability based on damage of normal liver tissue

1) Experiment method

After a rabbit experiment is completed, the liver is collected and photographed to visually inspect infarcts of liver tissues, thus to determine whether there is damage to normal liver tissue.

2) Experiment result

Referring to Table 4 below and FIG. 35, when doxorubicin-loaded porous silica particles were mixed with lipiodol and subjected to liver cancer embolization in a form of emulsion, surrounding normal liver tissues other than liver cancer cells exhibited no hepatotoxicity such as inflammation. As a result, it is possible to demonstrate embolization effects based on high targetability of the composition for embolization, which includes the porous silica particles of the present invention as well as lipiodol, and therapeutic effects of the loaded bioactive material corresponding to specific disease.

[TABLE 4]

| | | cTACE | DEB-TACE | DegradaBALL-TACE |
|---|---|---|---|---|
| Composition | | Lipiodol | PVA microbead | Lipiodol+DegradaBALL |
| Doxorubicin loading conc. | | 1 mg | 1 mg | 1 mg |
| Liver/Biliary injury | Liver infarct | None | Observed | None |
| | Multiple portal vein narrowing | None | Observed | None |
| | Multiple portal vein thromboses | None | Observed | None |

(3) Verification of targetability based on release amount of bioactive material in liver cancer tissue or cells

1) Experimental method

(i) TACE

A microcatheter was inserted through an artery in the rabbit's ear where VX2 tumor was planted in the liver and, when the microcather reached the hepatic artery, 0.4 ml of the particles in Example 2-(3)-4)-(i) loaded with doxorubicin were mixed with 1.6 ml of lipiodol to prepare an emulsion and 0.2 ml of the emulsion was injected through the microcatheter inserted into the liver artery.

(ii) Fluorescence signal analysis equipment

The tumor, liver, spleen and kidney of a rabbit collected after TACE with the porous silica particles having fluorescence on the surfaces of the particles were finely ground and mixed with 2 ml of 1.5% hydrochloric acid-ethanol solution per 1 g of each tissue, followed by well mixing tissues and the solution using a homogenizer. Then, the mixture was left at 4 °C for 24 hours while shielding the light to allow the particles in the tissues to be eluted. The solution was centrifuged by a centrifuge at 5000 rpm and 4 °C for 10 minutes, and then fluorescence of the supernatant was measured to determine an amount of particles remaining in each tissue.

(iii) Flow cytometry

The tumor, liver, spleen and kidney of a rabbit collected after TACE with the porous silica particles having fluorescence on the surface of the particles were finely ground and mixed with 10 ml of 0.25% trypsin per 200 mg of each tissue. The mixture was left at room temperature for 30 minutes to allow cells to peel off from the tissues. After 30 minutes, the supernatant was collected, followed by comparing amounts of particles contained in the cells extracted by flow cytometry.

(iv) Pharmacokinetics

At 0, 1, 5, 10, 30, 60 minutes after TACE procedure, the rabbit's blood was collected and the plasma was separated from the blood. Then, an amount of doxorubicin present in the plasma was analyzed by HPLC.

(v) Amount of doxorubicin remaining in tissue

Collected tumor and normal liver tissues were finely ground and mixed with 2 ml of 1.5% hydrochloric acid-ethanol solution per 1 g of each tissue, followed by well mixing tissues and the solution using a homogenizer. Then, the mixture was left at 4 °C for 24 hours while shielding the light to allow the particles in the tissues to be eluted. The solution was centrifuged by a centrifuge at 5000 rpm and 4 °C for 10 minutes, and then fluorescence of doxorubicin at 480 nm in the supernatant was measured to determine an amount of doxorubicin remaining in both of the tumor and the normal liver tissue.

2) Experiment result

Referring to FIG. 36(A) and (B), when embolization (DegradaBALL-TACE) was performed using the embolic composition including the porous silica particles of the present invention as well as lipiodol, most of the injected particles were observed in the liver cancer tissues (A) or the liver cancer cells (B). Therefore, excellent target delivery effects of the embolic composition according to the present invention could be identified.

Referring FIG. 36(C), when the embolization using the embolic composition including porous silica particles loaded with doxorubicin according to the present invention (DegradaBALL-TACE without lipiodol) was executed, an amount of doxorubicin released from cells surrounding the tumor was considerably lower than the embolization using lipiodol alone (cTACE). Further, in the case of the embolization using the embolic composition of the present invention along with lipidol (DegradaBALL-TACE), it can be seen that the amount of released doxorubicin is very insignificant. On the other hand, referring to FIG. 36(D), when the embolization using the embolic composition including porous silica particles loaded with doxorubicin according to the present invention (DegradaBALL-TACE without lipiodol) was executed, an amount of doxorubicin remaining in liver cancer tissues to normal liver tissues was significantly higher than the embolization using lipiodol alone (cTACE). Further, in the case of the embolization using the embolic composition of the present invention along with lipidol (DegradaBALL-TACE), it can be seen that the amount of released doxorubicin was even higher than the above cases. This result demonstrates high targetability of the embolic composition including porous silica particles according to the present invention, and suggests that the composition in conjunction with lipidol may exhibit greater synergistic effects and achieve excellent embolization results.

**Experimental Example 11** - **Verification of liver cancer treatment effect of embolic composition including porous silica particles**

(1) Experimental method

1) Viability

According to histopathology with respect to the collected tumors, tumor flakes were prepared and TUNEL assay was executed to distinguish dead and living cells, so that the living and dead cancer cells in the tumor could be visually distinguished. After visually identifying and determining the living cancer cells and dead cancer cells, a size of the living tumor was compared to a total tumor size thus to determine viability.

2) AST and ALT

On 0, 1, 4 and 7 days after TACE procedure, 1 ml of rabbit blood was collected, respectively, and then the plasma was separated from the blood to analyze concentrations of AST and ALT, which are proteins to identify liver specific toxicity in plasma, through colorimetric analysis.

(2) Experiment result

Referring to FIG. 37(A) and (B), the liver cancer tissue portion of the rabbit was stained with brown (A). Further, when the embolization using the embolic composition including the porous silica particles loaded with doxorubicin according to the present invention along with lipiodol was executed, it can be seen that the survival rate of liver cancer cells in the stained liver cancer tissue is significantly decreased depending on the concentration of loaded doxorubicin (B). This demonstrates excellent drug delivery and therapeutic effects by embolization using the composition of the present invention.

Referring to FIG. 37(C) and (D), as a result of measuring the concentrations of AST (aspartate aminotransferase; (C)) and ALT (alanine aminotransferase; (D)) during embolization using the composition of the present invention, these concentrations were maintained in a low level, indicating that no liver toxicity appears during embolization.

**Claims**

1. A composition for delivering a bioactive material in blood vessels, comprising a porous silica particle,

   wherein the bioactive material is loaded on a surface of the particle or insides of pores thereof, and the porous silica particle has a zeta potential of +3 mV or more or -18 mV or less, and
   the particle is chemically modified on the surfaces of the particle or the insides of the pores,
   wherein the particle is prepared by a small pore particle preparation, pore expansion process and calcination process,
   wherein the calcination is performed at 400 to 700 °C for 3 to 8 hours,
   wherein t when a ratio of absorbance in the following Equation 1 becomes 1/2 is 20 hours or more:

$$[\text{Equation 1}]$$

$$A_t/A_0$$

   (wherein $A_0$ is absorbance of the porous silica particle measured by placing 5 ml of a suspension including 1 mg/ml of the porous silica particle into a cylindrical dialysis membrane having pores with a diameter of 50 kDa, 15 ml of the same solvent as the suspension is placed outside the dialysis membrane while being in contact with the dialysis membrane, followed by horizontal agitation at 60 rpm and 37 °C inside and outside the dialysis membrane,
   pH of the suspension is 7.4, and
   At is absorbance of the porous silica particles measured after t hours elapses from the measurement of $A_0$, wherein the suspension is a buffer solution).

**2.** The composition according to claim 1, wherein at least a part of a silanol group on the surface of the particle or the inside of the pore in the particle is substituted with at least one functional group selected from the group consisting of aldehyde, keto, carbamate, sulfate, sulfonate, amino, amine, aminoalkyl, silyl, carboxyl, sulfonic acid, thiol, ammonium, sulfhydryl, phosphate, ester, imide, thioimide, ether, indene, sulfonyl, methylphosphonate, polyethylene glycol, substituted or unsubstituted $C_1$ to $C_{30}$ alkyl, substituted or unsubstituted $C_3$ to $C_{30}$ cycloalkyl, substituted or unsubstituted $C_6$ to $C_{30}$ aryl and $C_1$ to $C_{30}$ ester groups.

**3.** The composition according to claim 1, wherein at least a part of a silanol group on the surface of the particle or the inside of the pore in the particle is substituted with at least one functional group selected from the group consisting of amino, amine, PEG, propyl, octyl, carboxyl, thiol, sulfonic acid, methylphosphonate and aldehyde groups.

**4.** The composition according to claim 1, wherein the particle has a diameter of 100 to 1000 nm.

**5.** The composition according to claim 1, wherein the particle has a zeta potential of +3 mV to +100 mV or -100 mV to -18 mV.

**6.** The composition according to claim 1, wherein the particles have a volume of 0.7 to 2.2 ml per gram (g).

**7.** The composition according to claim 1, wherein a maximum release amount of the bioactive material loaded on the particles is 99% by weight or more.

**8.** The composition according to claim 1, wherein the bioactive material is at least one selected from the group consisting of nucleic acids, nucleotides, proteins, peptides, amino acids, sugars, lipids, compounds, antibodies, antigens, cytokines, growth factors and elements constituting the same.

**9.** The composition according to claim 1, wherein the bioactive material is at least one selected from the group consisting of doxorubicin, irinotecan, sorafenib, adriamycin, daunomycin, mitomycin, cisplatin, epirubicin, methotrexate, 5-fluorouracil, aclacinomycin, nitrogen mustard, cyclophosphamide, bleomycin, daunorubicin, vincristine, vinblastine, vindesine, tamoxifen, valrubisin, pirarubicin, mitoxantrone, gemcitabine, idarubicin, temozolomide, paclitaxel, dexamethasone, aldesleukin, lipavelumab, bevacizumab, carboplatin, regorafenib, docetaxel, doxil, gefitinib, imatinib mesylate, herceptin, imatinib, aldesleukin, keytruda, opdivo, mitomycin C, nivolumab, olaparib, pembrolizumab, rituximab, sunitinib, atezolizumab, lapatinib and ipilimumab.

**10.** The composition according to claim 1, wherein the composition is for releasing through a catheter into target tissues.

**11.** An embolic composition comprising the composition according to any one of claims 1 to 10.

**12.** The composition according to claim 11, further comprising at least one among contrast agents and embolic agents, wherein the embolic agent is selected from a group consisting of lipiodol, dextran, polyvinyl alcohol, N-butyl cyanoacrylate, gel foam, gelatin, ethanol, dextran, silica, polysodium acrylate vinylalcohol copolymer, glass particles, poly-L-guluronic alginate, polyglycolic-polyactic acid, polydioxanone, polyglycolic acid-co-caprolactone, and polypropylene.

**13.** The composition according to claim 11, wherein the composition is for releasing into a blood vessel directly connected to a tumor via a catheter.

**Patentansprüche**

**1.** Zusammensetzung zum Bereitstellen eines bioaktiven Materials in Blutgefäße, die poröse Siliciumdioxidteilchen umfasst,

wobei das bioaktive Material auf eine Oberfläche des Teilchens oder in dem Inneren seiner Poren aufgetragen ist und das poröse Siliciumdioxidteilchen ein Zetapotenzial von +3 mV oder mehr oder -18 mV oder weniger aufweist und

das Teilchen auf der Oberfläche des Teilchens oder in dem Inneren der Poren chemisch modifiziert ist, wobei das Teilchen über eine kleinporige Teilchenherstellung, ein Porenexpansionsverfahren und Kalzinierungsverfahren hergestellt ist,

wobei die Kalzinierung bei 400 bis 700 °C für 3 bis 8 Stunden durchgeführt wird,

wobei t, wenn ein Extinktionsverhältnis in der folgenden Gleichung 1 zu 1/2 wird, 20 Stunden oder mehr beträgt:

[Gleichung 1]

$$A_t / A_0$$

(wobei $A_0$ die Extinktion des porösen Siliciumdioxidteilchens ist, gemessen durch die Einbringung von 5 ml an Suspension, die 1 mg/ml des porösen Siliciumdioxidteilchens umfasst, in eine zylindrische Dialysemembran mit Poren mit einem Durchmesser von 50 kDa,

15 ml desselben Lösungsmittels wie die Suspension auf der Außenseite der Dialysemembran platziert werden, während sie in Kontakt mit der Dialysemembran steht, gefolgt von horizontaler Bewegung mit 60 U/min bei 37 °C innerhalb und außerhalb der Dialysemembran,

der pH-Wert der Suspension 7,4 beträgt und

$A_t$ die Extinktion der porösen Siliciumdioxidteilchen ist, gemessen in t Stunden nach der Messung von $A_0$, wobei die Suspension eine Pufferlösung ist.)

2. Zusammensetzung nach Anspruch 1, wobei wenigstens ein Teil einer Silanolgruppe auf der Oberfläche des Teilchens oder in dem Inneren der Pore des Teilchens durch wenigstens eine funktionelle Gruppe substituiert ist, die aus Aldehyd-, Keto-, Carbamat-, Sulfat-, Sulfonat-, Amino-, Amin-, Aminoalkyl-, Silyl-, Carboxyl-, Sulfonsäure-, Thiol-, Ammonium-, Sulfhydryl-, Phosphat-, Ester-, Imid-, Thioimid-, Ether-, Inden-, Sulfonyl-, Methylphosphonat-, Polyethylenglykol-, substituierten oder unsubstituierten $C_1$- bis $C_{30}$-Alkyl-, substituierten oder unsubstituierten $C_3$- bis $C_{30}$-Cycloalkyl-, substituierten oder unsubstituierten $C_6$- bis $C_{30}$-Aryl- und $C_1$- bis $C_{30}$-Estergruppen ausgewählt ist.

3. Zusammensetzung nach Anspruch 1, wobei wenigstens ein Teil einer Silanolgruppe auf der Oberfläche des Teilchens oder in dem Inneren der Pore des Teilchens durch wenigstens eine funktionelle Gruppe substituiert ist, die aus Amino-, Amin-, PEG-, Propyl-, Octyl-, Carboxyl-, Thiol-, Sulfonsäure-, Methylphosphonat- und Aldehydgruppen ausgewählt ist.

4. Zusammensetzung nach Anspruch 1, wobei das Teilchen einen Durchmesser von 100 bis 1000 nm aufweist.

5. Zusammensetzung nach Anspruch 1, wobei das Teilchen ein Zetapotential von +3 mV bis +100 mV oder -100 mV bis -18 mV aufweist.

6. Zusammensetzung nach Anspruch 1, wobei die Teilchen ein Volumen von 0,7 bis 2,2 ml pro Gramm (g) aufweisen.

7. Zusammensetzung nach Anspruch 1, wobei eine maximale Freisetzungsmenge des auf das Teilchen geladenen bioaktiven Materials 99 Gewichtprozent oder mehr beträgt.

8. Zusammensetzung nach Anspruch 1, wobei das bioaktive Material wenigstens aus einer der folgenden Gruppen ausgewählt ist: Nukleinsäuren, Nukleotiden, Proteinen, Peptiden, Aminosäuren, Zuckern, Lipiden, Verbindungen, Antikörpern, Antigenen, Zytokinen, Wachstumsfaktoren und Elementen, die dieselben aufbauen.

9. Zusammensetzung nach Anspruch 1, wobei das bioaktive Material wenigstens aus einer der folgenden Gruppen ausgewählt ist: Doxorubicin, Irinotecan, Sorafenib, Adriamycin, Daunomycin, Mitomycin, Cisplatin, Epirubicin, Methotrexat, 5-Fluorouracil, Aclacinomycin, Stickstofflost, Cyclophosphamid, Bleomycin, Daunorubicin, Vincristin, Vinblastin, Vindesin, Tamoxifen, Valrubisin, Pirarubicin, Mitoxantron, Gemcitabin, Idarubicin, Temozolomid, Paclitaxel, Dexamethason, Aldesleukin, Avelumab, Bevacizumab, Carboplatin, Regorafenib, Docetaxel, Doxil, Gefitinib, Imatinibmesilat, Herceptin, Imatinib, Aldesleukin, Keytruda, Opdivo, Mitomycin C, Nivolumab, Olaparib, Pembrolizumab, Rituximab, Sunitinib, Atezolizumab, Lapatinib und Ipilimumab.

10. Zusammensetzung nach Anspruch 1, wobei die Zusammensetzung zur Freisetzung durch einen Katheter in das Zielgewebe bestimmt ist.

11. Emboliezusammensetzung, umfassend die Zusammensetzung gemäß einem der Ansprüche 1 bis 10.

12. Zusammensetzung nach Anspruch 11, weiterhin umfassend Kontrastmittel und/oder Emboliemittel,

wobei das Emboliemittel aus einer Gruppe ausgewählt ist, die aus Lipiodol, Dextran, Polyvinylalkohol, N-Butylcyanoacrylat, Gelschaum, Gelatine, Ethanol, Dextran, Silica, Polynatriumacrylat-Vinylalkohol-Copolymer, Glasteilchen, Poly-L-Guluronalginat, Polyglykolsäure-Polymilchsäure, Polydioxanon, Polyglykolsäure-co-Caprolacton und Polypropylen besteht.

13. Zusammensetzung nach Anspruch 11, wobei die Zusammensetzung zur Freisetzung in ein Blutgefäß, das direkt mit einem Tumor in Kontakt ist, über einen Katheter bestimmt ist.

**Revendications**

1. Composition pour délivrer un matériau bioactif dans des vaisseaux sanguins, comprenant une particule de silice poreuse,

   dans laquelle le matériau bioactif est chargé sur une surface de la particule ou à l'intérieur des pores de celle-ci, et
   la particule de silice poreuse a un potentiel zêta de +3 mV ou plus ou de -18 mV ou moins, et
   la particule est modifiée chimiquement sur les surfaces de la particule ou à l'intérieur des pores,
   dans laquelle la particule est préparée par une préparation de particules à petits pores, un processus d'expansion des pores et un processus de calcination,
   dans laquelle la calcination est effectuée à 400 à 700 °C pendant 3 à 8 heures,
   dans laquelle t, lorsqu'un rapport d'absorbance dans l'équation 1 suivante devient 1/2, est de 20 heures ou plus :

   $$[\text{équation } 1]$$

   $$A_t/A_0$$

   (où $A_0$ est l'absorbance de la particule de silice poreuse mesurée en plaçant 5 ml d'une suspension comprenant 1 mg/ml de la particule de silice poreuse dans une membrane de dialyse cylindrique ayant des pores d'un diamètre de 50 kDa,
   15 ml du même solvant que la suspension sont placés à l'extérieur de la membrane de dialyse, tout en étant en contact avec la membrane de dialyse, suivi d'une agitation horizontale à 60 tr/min et 37 °C à l'intérieur et à l'extérieur de la membrane de dialyse,
   le pH de la suspension est 7,4, et
   At est l'absorbance des particules de silice poreuses mesurée après t heures écoulées depuis la mesure de $A_0$,
   où la suspension est une solution de tampon).

2. Composition selon la revendication 1, dans laquelle au moins une partie d'un groupe silanol à la surface de la particule ou à l'intérieur du pore de la particule est substituée par au moins un groupe fonctionnel choisi dans le groupe consistant en aldéhyde, céto, carbamate, sulfate, sulfonate, amino, amine, aminoalkyle, silyle, carboxyle, acide sulfonique, thiol, ammonium, sulfhydryle, phosphate, ester, imide, thioimide, éther, indène, sulfonyle, méthylphosphonate, polyéthylène glycol, des groupes alkyle en $C_1$ à $C_{30}$ substitués ou non substitués, cycloalkyle en $C_3$ à $C_{30}$ substitués ou non substitués, aryle en $C_6$ à $C_{30}$ substitués ou non substitués, et ester en $C_1$ à C30.

3. Composition selon la revendication 1, dans laquelle au moins une partie d'un groupe silanol à la surface de la particule ou à l'intérieur du pore de la particule est substituée par au moins un groupe fonctionnel choisi dans le groupe consistant en des groupes amino, amine, PEG, propyle, octyle, carboxyle, thiol, acide sulfonique, méthylphosphonate et aldéhyde.

4. Composition selon la revendication 1, dans laquelle la particule présente un diamètre de 100 à 1000 nm.

5. Composition selon la revendication 1, dans laquelle la particule présente un potentiel zêta de +3 mV à +100 mV ou de -100 mV à -18 mV.

6. Composition selon la revendication 1, dans laquelle les particules ont un volume spécifique de 0,7 à 2,2 ml par gramme (g).

**7.** Composition selon la revendication 1, dans laquelle une quantité maximale de libération du matériau bioactif chargé sur les particules est de 99 % en poids ou plus.

**8.** Composition selon la revendication 1, dans laquelle ledit matériau bioactif est au moins l'un choisi dans le groupe consistant en acides nucléiques, nucléotides, protéines, peptides, acides aminés, sucres, lipides, composés, anticorps, antigènes, cytokines, facteurs de croissance et éléments les constituant.

**9.** Composition selon la revendication 1, dans laquelle ledit matériau bioactif est au moins l'un choisi dans le groupe consistant en doxorubicine, irinotécan, sorafénib, adriamycine, daunomycine, mitomycine, cisplatine, épirubicine, méthotrexate, 5-fluorouracile, aclacinomycine, moutarde azotée, cyclophosphamide, bléomycine, daunorubicine, vincristine, vinblastine, vindésine, tamoxifène, valrubisine, pirarubicine, mitoxantrone, gemcitabine, idarubicine, témozolomide, paclitaxel, dexaméthasone, aldesleukine, lipavelumab, bévacizumab, carboplatine, régorafénib, docétaxel, doxil, géfitinib, mésylate d'imatinib, herceptin, imatinib, aldesleukine, keytruda, opdivo, mitomycine C, nivolumab, olaparib, pembrolizumab, rituximab, sunitinib, atézolizumab, lapatinib et ipilimumab.

**10.** Composition selon la revendication 1, dans laquelle la composition est destinée à être libérée par l'intermédiaire d'un cathéter dans les tissus cibles.

**11.** Composition embolique, comprenant la composition selon l'une quelconque des revendications 1 à 10.

**12.** Composition selon la revendication 11, comprenant en outre au moins un parmi les agents de contraste et les agents emboliques,
dans laquelle ledit agent embolique est choisi dans le groupe consistant en lipiodol, dextrane, alcool polyvinylique, cyanoacrylate de N-butyle, mousse de gel, gélatine, éthanol, dextrane, silice, copolymère d'acrylate de polysodium et d'alcool vinylique, particules de verre, alginate poly-L-guluronique, acide polyglycolique-polyactique, polydioxanone, acide polyglycolique-co-caprolactone et polypropylène.

**13.** Composition selon la revendication 11, dans laquelle la composition est destinée à être libérée dans un vaisseau sanguin directement relié à une tumeur via un cathéter.

【Fig.1】

【Fig.2】

【Fig.3】

【Fig.4】

2 L scale                    10 L scale

【Fig.5】

【Fig.6】

【Fig.7】

【Fig.8】

【Fig.9】

| Sample | t$_{50\%}$ |
|---|---|
| DDV(200)$_{10}$ | 57.4 h |
| DDV(500)$_{10}$ | 37.5 h |
| DDV(1000)$_{10}$ | 30.0 h |

【Fig.10】

| Samples | $t_{50\%}$ |
|---|---|
| MSN(200)$_2$ | 17.9 h |
| DDV(200)$_{10}$ | 57.4 h |
| DDV(200)$_{17}$ | 53.6 h |

【Fig.11】

DDV(300)$_{17}$

【Fig.12】

【Fig.13】

【Fig.14】

Irinotecan release profile in human plasma

【Fig.15】

Sorafenib release profile in PBS

【Fig.16】

**Retinoic Acid**

$t_{50} = \sim 3.7$ days ($\sim 89$ hr) ($\pm$ 15%)

【Fig.17】

# p53 peptide

【Fig.18】

mini-dialysis tube

siRNA/**pSP** suspension

dialysis membrane

medium (PBS)

ep-tube

【Fig.19】

【Fig.20】

【Fig.21】

$t(RC_{50}) = ca.\ 24\ h \pm 15\%$

【Fig.22】

linear DNA release in PBS (pH=7.4)

$t_{50}$ = ca. 5 h ± 15%

【Fig.23】

【Fig.24】

【Fig.25】

【Fig.26】

【Fig.27】

【Fig.28】

【Fig.29】

【Fig.30】

【Fig.31】

【Fig.32】

【Fig.33】

【Fig.34】

## Doxorubicin emulsion

【Fig.35】

【Fig.36】

【Fig.37】

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20140010285

- KR 401292208